# EUROPEAN PATENT APPLICATION

(11) **EP 3 203 238 A1**
(43) Date of publication of application: **09.08.2017**
(21) Application number: 15847239.9
(22) Date of filing: 01.10.2015
(51) Int. Cl.: G01N 33/574, C07K 16/28, G01N 33/577, C12P 21/08

(54) **REAGENTS FOR DETECTING OR DIAGNOSING CANCER CELLS HAVING HIGH INVASIVE CAPACITY**

(30) Priority: 01.10.2014 JP 2014203162
(71) Applicant: Order-Made Medical Research Inc., Kashiwa-shi, Chiba 277-0882 (JP)
(72) Inventor: SATOFUKA, Hiroyuki, Kashiwa-shi Chiba 277-0882 (JP); OKABE, Yoko, Kashiwa-shi Chiba 277-0882 (JP)
(74) Representative: J A Kemp
(86) International application number: PCT/JP2015/078558
(87) International publication number: WO 2016/052756

(57) **Abstract**

The present invention provides a reagent for detection or diagnosis of cancer, which comprises a monoclonal antibody against MCT5 or a fragment thereof.

## Description

### TECHNICAL FIELD

The present invention relates to a method for detection of cancer cells having high invasive capacity, wherein MCT5 (SLC16A4) is used as an indicator, and a reagent for detection or diagnosis of these cancer cells.

### BACKGROUND ART

Cancer is a disease which ranks high in the causes of death all over the world. In particular, breast cancer is a woman's cancer most commonly seen in the United Kingdom, the United States and Japan. The number of women suffering from breast cancer in Japan has now exceeded 40,000 and still tends to increase. Recent advances in medical equipment for mammography or other diagnostic imaging techniques have allowed early diagnosis of breast cancer, and it is expected that the healing rate of breast cancer will gradually increase.

Patients who develop recurrence or metastasis are not few in number although they have undergone surgical excision and/or radiation therapy at an early stage of breast cancer as a result of early diagnosis. In fact, there is a report showing that about 40% of patients suffering from breast cancer will die although the five-year survival rate after primary breast cancer resection is 90% or more (Non-patent Document 1: Weigelt B, et al., Nature Reviews, 2005, 5, 591-602). Moreover, it is also reported that patients who have survived more than 20 years and are therefore diagnosed as having gone into remission account for only 2% to 3% of all patients. Namely, in addition to early diagnosis of breast cancer, there is a demand for the development of a new diagnostic method which allows evaluation of recurrence or metastasis of breast cancer.

Some cases of breast cancer do not develop recurrence or metastasis, but these cases are difficult to predict in advance; and hence many patients are now administered with therapeutic agents for the purpose of reducing the risk of recurrence or metastasis. For patients who do not develop recurrence or metastasis, this means that they receive unwanted treatment which may cause side effects.

On the other hand, in breast cancer surgery, conservative therapy is conducted for the purpose of improving the quality of life (QOL) after surgery. As of 2001, conservative therapy has been conducted in 40% or more of all patients. However, among patients who have undergone conservative therapy, some patients are at high risk of recurrence or metastasis.

The "recurrence" of cancer refers to an event where after surgical excision of the primary focus, invisible small cancer lesions are left behind without being removed and then will form tumors again, or an event where cancer lesions whose size has been reduced upon drug therapy (treatment with anticancer agents) and/or radiation therapy will become larger again. Such an event when occurring in close proximity to the first occurring cancer is referred to as "recurrence." On the other hand, "metastasis" refers to an event where cancer cells reach an organ different from the primary focus and cause secondary formation of cancer of the same type. For example, a case where breast cancer forms tumors again in mammae is "recurrence," while secondary cancer having metastasized to lungs is breast cancer lung "metastasis" formed by malignant breast cancer cells.

As to the mechanism by which "recurrence" or "metastasis" occurs, the major cause is probably that cancer cells having high invasive capacity invade another site in the same tissue as the primary focus or locally invade cancer adjacent tissues and are left behind without being removed by surgical operation, etc.

When cancer cells cause recurrence or metastasis even after surgical removable of cancer sites, the character of these cancer cells is critical. As indicators representing the poor character of cancer cells and the degree of progression, the grade of atypism and the depth of invasion are used. The grade of atypism indicates what degree of morphological difference exists between cancer cells and normal cells. On the other hand, the depth of invasion indicates the depth of cancer. When cancer cells have high invasive capacity, the depth of invasion is larger and these cancer cells are classified as more malignant cancer cells. For this reason, the depth of invasion is used for clinical stage classification of malignant tumors. Accordingly, cancer cells having high invasive capacity are highly malignant and considered to be cancer at high risk of recurrence or metastasis.

Namely, cancer cells having high invasive capacity cause an increase in the risk of recurrence or metastasis. Accordingly, if the invasive capacity of cancer cells can be evaluated, the grade of their malignancy can also be determined, which allows selection of a post-operative therapeutic strategy to ensure an optimal prognosis, so that it can be expected that cases of cancer recurrence or metastasis are reduced in number to thereby reduce the mortality.

In cancer diagnosis, when a subject is suspected to have cancer as a result of physical examination, urine analysis, biochemical analysis of blood, blood counting, chest X-ray examination, mammary X-ray examination (mammography) and/or fecal occult blood testing, such a subject will undergo histological examination and immunohistochemical examination using a biopsy sample which is a portion taken from a lump-containing tissue, followed by morphological diagnosis on the stained images. In most cases, the obtained tissue is prepared into thin sections, which are then fixed and subjected to hematoxylin-eosin staining (HE staining).

In histological observation, disappearance of tissue-constituting basal cells and other events serve as important factors for diagnosing a subject as having cancer. However, basal cells may also be altered in benign lesions, and diagnosis based solely on HE staining involves many difficulties, so that basal cells in atypical lesions are diagnosed by immunohistochemical staining in principal. In immunohistochemical staining, cancer is evaluated by detection of epidermal growth factor receptor 2 (Her2/erbB2) for breast cancer and epidermal growth factor receptor (EGFR) for colorectal cancer (Patent Document 1: JP 2006-519376 A).

However, the frequency of occurrence evaluated by immunohistochemical staining varies among reports from 25% to 77% in colorectal cancer upon detection of EGFR (Non-patent Document 2: Modem Media, Vol. 55, No. 7, 2009). Likewise, in breast cancer, the frequency of occurrence is 15% to 25% upon detection of Her2, which means that a limited percentage of patients can be diagnosed as being positive (Non-patent Document 3: Endocr Relat Cancer, 2002, 9, p. 75-85). Moreover, both EGFR and Her2 may be stained even in normal tissues, depending on the type of staining. Thus, a strictly normalized method is required to make a positive diagnosis.

Moreover, recent reports have pointed out that cancer cells constituting tumor tissue are not a homogeneous population and include several types of cancer cells, which is referred to as intratumoral heterogeneity (Non-patent Document 4: Cell, 2012, 149, p. 994-1007). The presence of these several types of cancer cells would be responsible for an event where tumors whose size has been reduced upon treatment with anticancer agents will become larger again to form tumors which are more resistant to the anticancer agents and more malignant (i.e., more invasive and more likely to repeat metastasis and recurrence) than before. Further, among these several types of cells, in tumor tissue, some cancer cells closely resemble normal tissue stem cells because of having self-replication capacity and pluripotency (i.e., cancer stem cells; also referred to as tumor initiating cells), and these cells would be a source of cancer cells repeating recurrence and metastasis.

For identification of these cells as mentioned above, experimental procedures using multiple marker molecules are now reported. In the case of breast cancer, cell populations positive to CD44, negative to CD24 and negative to differentiation lineage markers (CD2, CD3, CD10, CD16, CD18, CD31, D64, CD140b) are reported to be high in the frequency of tumor formation (Non-patent Document 5: Proc. Natl. Acad. Sci. USA, 2003, 100, p. 3983-8). In the case of colorectal cancer, cells positive to EpCAM and positive to CD44 or CD166 are reported to be high in the capacity of tumor formation (Non-patent Document 6: Proc. Natl. Acad. Sci. USA, 2007, 104, p. 10158-63). In this way, conventional techniques are designed to detect highly malignant cancer cells with the use of multiple markers and therefore have a problem in that highly malignant tumors cannot be identified in a simple manner.

Under these circumstances, there is a demand for the development of a new target molecule which allows detection of highly malignant cancer tissue in a cancer patient and allows prediction of the risk of recurrence or metastasis after surgery, and there is also a demand for the development of a monoclonal antibody for detection of such a new target molecule.

To specifically detect a particular type of cells, a molecule specifically expressed in these cells and an antibody or an aptamer are generally used for this purpose. Thus, the inventors of the present invention have narrowed down candidate genes whose expression is enhanced in cancer cells, and have thereby focused on a molecule called monocarboxylate transporter 5 (MCT5) or solute carrier family 16 member 4 (SLC16A4) as a marker molecule required to specifically detect cancer cells.

MCT5 is a membrane protein composed of 487 amino acids and registered under NCBI (the National Center for Biotechnology Information) Reference Sequences [RefSeq] ID: NM_004696.2 and NP_004687.1 (SEQ ID NO: 1: nucleotide sequence, SEQ ID NO: 2: amino acid sequence). However, the function of human MCT5 has been almost unknown and there is only a report showing that rabbit MCT5 is involved in lactic acid transport on the cornea (Non-patent Document 7: J. Pharm. Pharmacol., 2013, 65 p. 328-36).

MCT5 nucleotide sequence (SEQ ID NO: 1) 
ATGCTGAAGAGGGAGGGGAAGGTCCAACCTTACACTAAAACCCTGGATGGA
GGATGGGGATGGATGATTGTGATTCATTTTTCCTGGTGAATGTGTTTGTGATG
GGGATGACCAAGACTTTTGCAATTTCTTTGTGGTCTTTCAAGAAGAGTTTG
AAGGCACCTCAGAGCAAATTGGTTGGATTGGATCCATCATGTCATCTCTTCGT
TTTTGTGCAGGTCCCCTGGTTGCTATTATTTGTGACATACTTGGAGAGAAAAC
TACCTCCATTCTTGGGGCTTTCGTTGTTACTGGTGGATATCTGATCAGCAGCTG
GGCCACAAGTATTCCTTTTCTTTGTGTGACTATGGGACTTCTACCCGGTTTGG
GTTCTGCTTTCTTATACCAAGTGGCTGCTGTGGTAACTACCAAATACTTCAAA
AAACGATTGGCTCTTTCTACAGCTATTGCCCGTTCTGGGATGGGACTGACTTT
TCTTTTGGCACCCTTTACAAAATTCCTGATAGATCTGTATGACTGGACAGGAG
CCCTTATATTATTTGGAGCTATCGCATTGAATTTGGTGCCTTCTAGTATGCTCTT
AAGACCCATCCATATCAAAAGTGAGAACAATTCTGGTATTAAAGATAAAGGC
AGCAGTTTGTCTGCACATGGTCCAGAGGCACATGCAACAGAAACACACTGC
CATGAGACAGAAGAGTCTACCATCAAGGACAGTACTACGCAGAAGGCTGGA
 
CTACCTAGCAAAAATTTAACAGTCTCACAAAATCAAAGTGAAGAGTTCTACA
ATGGGCCTAACAGGAACAGACTGTTATTAAAGAGTGATGAAGAAAGTGATAA
GGTTATTTCGTGGAGCTGCAAACAACTGTTTGACATTTCTCTCTTTAGAAATC
CTTTCTTCTACATATTTACTTGGTCTTTTCTCCTCAGTCAGTTAGCATACTTCAT
CCCTACCTTTCACCTGGTAGCCAGAGCCAAAACACTGGGGATTGACATCATG
GATGCCTCTTACCTTGTTTCTGTAGCAGGTATCCTTGAGACGGTCAGTCAGAT
TATTTCTGGATGGGTTGCTGATCAAAACTGGATTAAGAAGTATCATTACCACA
AGTCTTACCTCATCCTCTGCGGCATCACTAACCTGCTTGCTCCTTTAGCCACC
ACATTTCCACTACTTATGACCTACACCATCTGCTTTGCCATCTTTGCTGGTGGT
TACCTGGCATTGATACTGCCTGTACTGGTTGATCTGTGTAGGAATTCTACAGTA
AACAGGTTTTTGGGACTTGCCAGTTTCTTTGCTGGGATGGCTGTCCTTTCTGG
ACCACCTATAGCAGGCTGGTTATATGATTATACCCAGACATACAATGGCTCTTT
CTACTTCTCTGGCATATGCTATCTCCTCTCTTCAGTTTCCTTTTTTTTGTACCA
TTGGCCGAAAGATGGAAAAACAGTCTGACCTGA
MCT5 amino acid sequence (SEQ ID NO: 2)

MLKREGKVQPYTKTLDGGWGWMIVIHFFLVNVFVMGMTKTFAIFFVVFQEEFE
GTSEQIGWIGSIMSSLRFCAGPLVAIICDILGEKTTSILGAFVVTGGYLISSWATSIP
FLCVTMGLLPGLGSAFLYQVAAVVTTKYFKKRLALSTAIARSGMGLTFLLAPFT
KFLIDLYDWTGALILFGAIALNLVPSSMLLRPIHIKSENNSGIKDKGSSLSAHGPE
AHATETHCHETEESTIKDSTTQKAGLPSKNLTVSQNQSEEFYNGPNRNRLLLKS
DEESDKVISWSCKQLFDISLFRNPFFYIFTWSFLLSQLAYFIPTFHLVARAKTLGID
IMDASYLVSVAGILETVSQIISGWVADQNWIKKYHYHKSYLILCGITNLLAPLAT
TFPLLMTYTICFAIFAGGYLALILPVLVDLCRNSTVNRFLGLASFFAGMAVLSGPP
IAGWLYDYTQTYNGSFYFSGICYLLSSVSFFFVPLAERWKNSLT*

As to the MCT family, the nomenclature was altered in the past, and there are cases where MCT4 is expressed as SLC16A4 and where MCT5 is expressed as MCT4. MCT5 and MCT4 are poorly homologous to each other because of sharing an identity of 15.5% and a similarity of 55.5%, and are therefore considered to be functionally distinct molecules (Figure 1). Hereinafter, unified expressions as found in Non-patent Document 8 (Biochem J 1999, 343 p. 281-299) are used.

Among molecules classified into the MCT family, MCT1 (SLC16A1) and MCT4 (SLC16A3) are reported as molecules involved in the invasion of cancer cells. MCT1 and MCT4 are molecules responsible for lactic acid transport. In particular, MCT4 is reported to interact with CD147 in breast cancer and to activate the synthesis of matrix metalloprotease which is an extracellular protease (Non-patent Document 9: Cancer Res. 2007 May 1; 67(9):4182-9). Moreover, Non-patent Document 10 (Izumi H, et al. Cancer Sci, 2011, 102, 1007-1013) reports that in human lung cancer, MCT1 and MCT4 are involved in invasion, but MCT5 is not involved in invasion.

MCT5 expression in cancer has been reported in pancreatic cancer (Non-patent Document 11: Nature 2012, 491 p. 399-405) or in head and neck squamous cell carcinoma (Non-patent Document 12: Oral Oncol. 2014 Mar; 50(3):200-7), although the physiological functional relationship between cancer and MCT5 has been unknown and there is no report showing that MCT5 is involved in invasion. Thus, it has also been unknown that an antibody detecting MCT5 can be used as a marker of invasion.

As antibodies against MCT5, HPA046986 (ATLAS ANTIBODIES) and LS-C25647 (LifeSpan Biosciences) are known, by way of example. These antibodies were prepared using, as an antigen, a sequence contained in a region within the fourth intracellular domain of MCT5 (ICD4: SEQ ID NO: 7), in light of the three-dimensional structure predicted from the primary structure of their amino acids. In addition, sc-14932 (Santa Cruz) is an antibody prepared using the N-terminal region of MCT5 as an antigen, while sc-14934 (Santa Cruz) is an antibody prepared using the C-terminal region of MCT5 as an antigen. These antibodies were each obtained by immunization of rabbits or goats with a purified polypeptide and the subsequent affinity purification, and there is no report of monoclonal antibodies.

To use an antibody as a tool for target protein detection or disease diagnosis, a monoclonal antibody is desired for this purpose because it is important to ensure continuous and uniform production and provision. In general, it is often difficult to prepare an antibody against a membrane protein, particularly a multi-transmembrane protein, such as MCT5. This is because a membrane protein is difficult to solubilize when purified as an antigen and because immunization with a partial protein often results in only antibodies incapable of recognizing the three-dimensional structure of the antigen. Moreover, even when polyclonal antibodies can be obtained from, e.g., the peripheral blood of immunized animals, the probability of successfully isolating antibody-producing cells and establishing monoclonal antibody-producing hybridoma cells is very low under the present circumstances.

The fourth intracellular domain of MCT5 (SEQ ID NO: 7)

RPIHIKSENNSGIKDKGSSLSAHGPEAHATETHCHETEESTIKDSTTQKAGLPSKN
LTVSQNQSEEFYNGPNRNRLLLKSDEESDKVISWSCKQLFDISLFRNP

### Prior Art Documents

### Patent Documents

Patent Document 1: JP 2006-519376 A

### Non-patent Documents

Non-patent Document 1: Weigelt B, et al., Nature Reviews, 2005, 5, 591-602
Non-patent Document 2: Modem Media, Vol. 55, No. 7, 2009 (in Japanese)
Non-patent Document 3: Endocr Relat Cancer, 2002, 9, p. 75-85
Non-patent Document 4: Cell, 2012, 149, p. 994-1007
Non-patent Document 5: Proc. Natl. Acad. Sci. USA, 2003, 100, p. 3983-8
Non-patent Document 6: Proc. Natl. Acad. Sci. USA, 2007, 104, p. 10158-63
Non-patent Document 7: J. Pharm. Pharmacol., 2013, 65 p. 328-36
Non-patent Document 8: Biochem J 1999, 343 p. 281-299
Non-patent Document 9: Cancer Res. 2007 May 1; 67(9):4182-9
Non-patent Document 10: Izumi H, et al. Cancer Sci, 2011, 102, 1007-1013
Non-patent Document 11: Nature 2012, 491 p. 399-405
Non-patent Document 12: Oral Oncol. 2014 Mar; 50(3):200-7

### SUMMARY OF THE INVENTION

### PROBLEM TO BE SOLVED BY THE INVENTION

The present invention has been made under these circumstances, and the problems to be solved by the present invention are to identify a target molecule whose expression is enhanced in highly malignant cancers, particularly highly invasive cancers, to obtain a monoclonal antibody which allows highly sensitive detection of such a target molecule, and to provide a reagent for detection or diagnosis of cancer, which comprises such an antibody.

As a result of extensive and intensive efforts made to solve the above problems, the inventors of the present invention have found that MCT5 is involved in increased invasion of cancer cells, and have succeeded in detecting a highly invasive cancer site by using an antibody which binds to the extracellular region of MCT5. This has led to the completion of the present invention.

### Namely, the present invention is as follows.

(1) A reagent for detection or diagnosis of a cancer having invasive capacity, which comprises a monoclonal antibody against monocarboxylate transporter 5 (MCT5) or a fragment of the antibody.
(2) The reagent according to (1) above, wherein the antibody or fragment thereof binds to the extracellular region of monocarboxylate transporter 5 (MCT5).
(3) The reagent according to (1) above, wherein the extracellular region comprises the amino acid sequence shown in SEQ ID NO: 13.
(4) The reagent according to (1) above, wherein the cancer is at least one selected from the group consisting of breast cancer, colorectal cancer, lung cancer, ovarian cancer, uterine cancer, uterine cervical cancer, esophageal cancer, thyroid cancer, skin cancer, gastric cancer, pancreatic cancer, brain tumor, tongue cancer, small intestinal cancer, duodenal cancer, bladder cancer, kidney cancer, liver cancer, prostate cancer, cervical cancer, gallbladder cancer, pharyngeal cancer, sarcoma, melanoma, leukemia, lymphoma and multiple myeloma.
(5) The reagent according to (1) above, wherein the cancer is at least one selected from the group consisting of breast cancer, colorectal cancer, lung cancer, ovarian cancer, uterine cancer, uterine cervical cancer, esophageal cancer, thyroid cancer and skin cancer.
(6) The reagent according to (1) above, wherein the cancer is at least one selected from the group consisting of breast cancer, colorectal cancer and lung cancer.
(7) The reagent according to (1) above, wherein the cancer is breast cancer or colorectal cancer.
(8) A method for detection of cancer, which comprises the step of contacting a monoclonal antibody against monocarboxylate transporter 5 (MCT5) or a fragment of the antibody with a biological sample taken from a subject to thereby detect MCT5 in the sample.
(9) A method for diagnosis of cancer, which comprises the step of contacting a monoclonal antibody against monocarboxylate transporter 5 (MCT5) or a fragment of the antibody with a biological sample taken from a subject to thereby detect MCT5 in the sample.
(10) The method according to (8) or (9) above, wherein the cancer is a cancer having invasive capacity.
(11) A monoclonal antibody against monocarboxylate transporter 5 (MCT5) or a fragment of the antibody.
(12) A kit for detection of cancer, which comprises a monoclonal antibody against monocarboxylate transporter 5 (MCT5) or a fragment of the antibody.
(13) The kit according to (12) above, wherein the cancer is a cancer having invasive capacity.

### EFFECTS OF THE INVENTION

When using the monoclonal antibody of the present invention, cancer cells which significantly highly express MCT5 can be detected with high sensitivity through various means. In cancer diagnosis, when MCT5 expression is used as an indicator for analysis of a biopsy sample or a surgically excised sample or cells circulating in blood, the invasive capacity of cancer cells can be evaluated. The high invasive capacity of cancer cells leads to an increased risk of cancer metastasis or recurrence. Thus, once the invasive capacity has been evaluated, the metastatic or recurrent capacity will be able to be evaluated.

Particularly in breast cancer surgery, the level of metastatic capacity serves as an important factor in the determination of the mammary area to be conserved. Thus, if the level of metastatic capacity can be evaluated, the QOL of patients can be improved remarkably. Moreover, once the level of metastatic capacity has been evaluated, it will greatly affect the determination of a post-operative therapeutic strategy. Information useful to predict metastasis and/or recurrence can be obtained, so that the mortality due to cancer can be reduced.

### BRIEF DESCRIPTION OF THE DRAWINGS

Figure 1 shows an alignment of the full-length amino acid sequences of human MCT4 and human MCT5.
Figure 2 shows the expression level of MCT5 per cell compared between MCF7 and MCF7-14.
Figure 3 shows the results obtained when MCF7 cells were transiently transfected with the MCT5 gene and analyzed for the mRNA level of MCT5. MCF7-Mock shows the results measured for the mRNA level of MCT5 at 24 hours after the cells were transfected with a MCT5-free vector, while MCF7-MCT5 shows the results measured for the mRNA level of MCT5 at 24, 48, 72 and 96 hours after the cells were transiently transfected with a MCT5-containing vector.
Figure 4 shows the results obtained when MCF7 was engineered to transiently express the MCT5 gene and Matrigel-permeating cells were analyzed.
Figure 5 shows the results obtained when MCF7 was engineered to stably express the MCT5 gene and analyzed for the mRNA level of MCT5.
Figure 6 shows the results obtained when cells stably expressing MCT5 were analyzed for their Matrigel permeability.
Figure 7 shows a schematic view of positions on the amino acid sequence which are matched between the full-length amino acid sequence of MCT5 and MCT5-ICD4 used as an antigen.
Figure 8 shows the activity of antibodies contained in the supernatants of two clones obtained during preparation of monoclonal antibodies using MCT5-ICD4 as an antigen, as analyzed by ELISA.
Figure 9 shows the results obtained when the antibody of clone No. IMI4C4a was reacted at varying concentrations with MCT5-ICD4 or a protein having a Tag sequence alone (Trx, S, His-tag).
Figure 10 shows the results of immunocytological staining with the IMI4C4a antibody performed on MCF7 and MCF7-MCT5-2.
Figure 11 shows the results of Western blotting with the IMI4C4a antibody performed on MCF7 and MCF7-MCT5-2.
Figure 12 shows the results of immunohistological staining with the IMI4C4a antibody performed on 3 cases of normal mammary gland tissue and 3 cases of breast cancer tissue.
Figure 13 shows some of the results of immunohistological staining with the IMI4C4a antibody performed on 10 cases of normal mammary gland tissue and 100 cases of breast cancer tissue at different advanced stages.
Figure 14 is a graph showing the results of statistical analysis on the degree of staining in immunohistological staining with the IMI4C4a antibody. The results determined to be positive are expressed as "3+," "2+," "1+" and "0" in the order from strong to weak signals.
Figure 15 shows some of the results of immunohistological staining with the IMI4C4a antibody performed on tissue samples which were found to be estrogen receptor-negative, progesterone receptor-negative and Her2-negative (i.e., triple negative), among 100 cases of breast cancer tissue.
Figure 16 shows the results of immunohistological staining with the IMI4C4a antibody performed on 2 cases of colorectal cancer and 3 cases of normal colorectal tissue.
Figure 17 shows the results of immunohistological staining with the IMI4C4a antibody performed on 3 cases of lung cancer and 3 cases of normal lung tissue.
Figure 18 shows the results of immunohistological staining with the IMI4C4a antibody performed on ovarian cancer, uterine cancer, uterine cervical cancer, esophageal cancer, thyroid cancer and skin cancer.
Figure 19 shows the results obtained when the IMI4C4a antibody was purified with Protein G and analyzed for its reactivity to HCT116 cells in a flow cytometer.
Figure 20 shows the results obtained when four types of antibodies, i.e., the IMI4C4a antibody, ab180699, HPA046986 and sc-14932 were used and analyzed for their reactivity to HCT116 cells in a flow cytometer.
Figure 21 shows the results obtained when three types of antibodies, i.e., the IMI4C4a antibody, ab180699 and HPA046986 were used and analyzed for their reactivity to HCT116 cells by immunocytological staining.
Figure 22 shows a schematic view of positions on the amino acid sequence which are matched between the full-length amino acid sequence of MCT5 (SEQ ID NO: 2) and MCT5-ICD4 (SEQ ID NO: 7), ICD4_196-258 (SEQ ID NO: 8) or ICD4_227-299 (SEQ ID NO: 9), each being used as an antigen.
Figure 23 shows the results obtained as to whether the binding reaction of the IMI4C4a antibody to ICD4 (SEQ ID NO: 7) is competitively inhibited by ICD4 or a partial protein of ICD4, i.e., ICD4_196-258 (SEQ ID NO: 8) or ICD4_227-299 (SEQ ID NO: 9), as analyzed by ELISA.
Figure 24 shows the results obtained as to whether the binding reaction of the IMI4C4a antibody to cancer cells is competitively inhibited by ICD4 or a partial protein of ICD4, i.e., ICD4_196-258 (SEQ ID NO: 8) or ICD4_227-299 (SEQ ID NO: 9), as analyzed in a flow cytometer.
Figure 25 shows a schematic view of positions on the amino acid sequence which are matched between the full-length amino acid sequence of MCT5 and MCT5-ICD4 (SEQ ID NO: 7), ICD4_196-258 (SEQ ID NO: 8), ICD4_227-299 (SEQ ID NO: 9), ICD4_227-242 (SEQ ID NO: 10), ICD4_235-250 (SEQ ID NO: 11) or ICD4_242-258 (SEQ ID NO: 12), each being used as an antigen.
Figure 26 shows the results obtained as to whether the binding reaction of the IMI4C4a antibody to ICD4 (SEQ ID NO: 7) is competitively inhibited by ICD4 or a partial peptide of ICD4, i.e., ICD4_227-242 (SEQ ID NO: 10), ICD4_235-250 (SEQ ID NO: 11) or ICD4_242-258 (SEQ ID NO: 12), as analyzed by ELISA.
Figure 27 shows positions on the amino acid sequence which are matched among ICD4-195-250 (SEQ ID NO: 15) used as an antigen for preparation of HPA046986 which is a commercially available antibody, ICD4 (SEQ ID NO: 7) used as an antigen for preparation of the IMI4C4a antibody, and ICD4_251-258 (SEQ ID NO: 13) which is an amino acid sequence comprising an epitope for the IMI4C4a antibody.

### DESCRIPTION OF EMBODIMENTS

The present invention will be described in more detail below. The following embodiments are illustrated to describe the present invention, and it is not intended to limit the present invention only to these embodiments. The present invention can be implemented in various modes, without departing from the spirit of the present invention.

### 1. Summary

The present invention relates to a reagent for detection or diagnosis of highly invasive cancer cells, which comprises a monoclonal antibody against monocarboxylate transporter 5 (MCT5) or solute carrier family 16 member 4 (SLC16A4) or a fragment of the antibody.

As a result of extensive and intensive efforts made with a focus on MCT5 which is a transmembrane protein, the inventors of the present invention have found that MCT5 is related to the invasive capacity of cancer cells, and have elucidated that upon detection of MCT5, the invasive capacity of cancer can be evaluated, i.e., the presence of cancer cells which are highly likely to cause metastasis and/or recurrence can be detected in cancer tissues. Moreover, the inventors of the present invention have found that MCT5 expression is enhanced in a part of cancer tissue particularly in breast cancer and colorectal cancer, lung cancer, ovarian cancer, uterine cancer, uterine cervical cancer, esophageal cancer, thyroid cancer and skin cancer.

Accordingly, the present invention provides a cancer marker comprising MCT5.

Moreover, the inventors of the present invention have succeeded in preparing a monoclonal antibody against MCT5, which allows cancer detection with extremely high sensitivity and high accuracy when compared to conventionally known antibodies. The antibody of the present invention allows detection of highly invasive cancers with high sensitivity even at an early stage of cancer progression and also allows detection of living cancer cells because of being capable of binding to the extracellular region of MCT5, which in turn allows detection of cancer cells present in blood samples, by way of example. Namely, when using the antibody of the present invention, highly invasive cancers which have been undetectable by conventional methods can be detected in a simple manner with high sensitivity and high accuracy, so that the antibody of the present invention is very useful for detection or diagnosis of highly malignant cancer cells. The present invention has been completed on the basis of such findings.

### 2. Antibody against MCT5 (anti-MCT5 antibody)

### (1) Antigen preparation

MCT5 intended in the present invention may be derived from any mammal, and examples of such a mammal include mice, rats, rabbits, goats, dogs, monkeys and humans, with mice, rats and humans being preferred, and with humans being more preferred.

In the present invention, the amino acid sequences of human, mouse and rat MCT5 are shown in SEQ ID NOs: 2, 4 and 6, respectively. Likewise, the nucleotide sequences of DNAs encoding human, mouse and rat MCT5 are shown in SEQ ID NOs: 1, 3 and 5, respectively. These amino acid sequences and nucleotide sequences have been registered in the GenBank database under the accession numbers (Accession Nos.) given to the respective sequences.
Amino acid sequence of human MCT5: NP_004687.1 (SEQ ID NO: 2) 
MLKREGKVQPYTKTLDGGWGWMIVIHFFLVNVFVMGMTKTFAIFFVVFQEEFE
GTSEQIGWIGSIMSSLRFCAGPLVAIICDILGEKTTSILGAFVVTGGYLISSWATSIP
FLCVTMGLLPGLGSAFLYQVAAVVTTKYFKKRLALSTAIARSGMGLTFLLAPFT
KFLIDLYDWTGALILFGAIALNLVPSSMLLRPIHIKSENNSGIKDKGSSLSAHGPE
AHATETHCHETEESTIKDSTTQKAGLPSKNLTVSQNQSEEFYNGPNRNRLLLKS
DEESDKVISWSCKQLFDISLFRNPFFYIFTWSFLLSQLAYFIPTFHLVARAKTLGID
IMDASYLVSVAGILETVSQIISGWVADQNWIKKYHYHKSYLILCGITNLLAPLAT
TFPLLMTYTICFAIFAGGYLALILPVLVDLCRNSTVNRFLGLASFFAGMAVLSGPP
IAGWLYDYTQTYNGSFYFSGICYLLSSVSFFFVPLAERWKNSLT*
Amino acid sequence of mouse MCT5: NP_666248.1 (SEQ ID NO: 4) 
MVKKEKTPSPYTKPLDGGWGWMVVLHFFLVNVFVMGMTKTFAIFFVVFQEEF
EGTSEQIGWIGSIMSSLRFSAGPLAAIICDVLGEKATSILGTFLVSGGYVISSWATG
IPFLCVTMGLLPGLGSAFLYQVAAVVITKYFKKRLGLSTAIARSGMGLTFLLAPFT
KFLIDLYDWTGALILFGAIILNLVPSSMLLRPIHSQSNNNSDIENKGSILSATEPEA
SYKTETSKCKETQEPFIKDSTMKKSEQPTTTLTVLGNQSEEFSNRPHRNRPLLMS
NGKSHKKKFVSWNCKQKLLDISLFRNPFFYIFTWSFLLSQLAYFIPTFHLVARAK
TLGIDVMDASYLVSVAGITETVSQLISGWIADQNWIKKYQYHKSYLILCGVTNL
LAPLATTFPLLMAYTILFAIFAGGYLALILPVLVDLSKNSRVHKFLGYASFFAGIAV
LSGPPIAGWIYDYTQTYTGSFYFSGTCYILSSVSLFFVPLAERWKRKQSDLLRTTI
 K*
Amino acid sequence of rat MCT5: NP_666248.1 (SEQ ID NO: 6) 
MKKREEKPSPYTKPLDGGWGWMVVLHFFLVNVFVMGMTKTFAIFFVVFQEEF
EGTSEQIGWIGSIMSSLRFSAGPLAAIICDIFGEKTTSILGTFLVSGGYLISSWATGI
PFLCVTMGLLPGLGSAFLYQVAAVVITKYFKKRLGLSTAIARSGMGLTFLLAPFT
KLLIDLYDWTGALILFGAIALNLVPSSMLLRPVHIQSDSNSDIKNKGSILSATGPE
LSHKTETPNRNKAQESLIKDSAVKKSGQPTTTLTVLENQIEEISTRPHRNRPLLMS
NDKTYKKKFVSWNCKEKLLDISLFRNPFFYIFTWSFLLSQLAYFIPTFHLVARAK
TLGIDIMDASYLVSVAGITETVSQLISGWIADQNWIKKYQYHKAYLILCGVTNLL
APLATTFPLLMAYTIFFAIFAGGYLALILPVLVDLSKNSRVHRFLGYASFFAGMAV
LSGPPIAGWIYDYTQTYVGSFYFSGTCYILSSVSLFFVPLAERWKLKQSDLLRTTI
K*
Nucleotide sequence of DNA encoding human MCT5: NM_004696.2 (SEQ ID NO: 1) 
ATGCTGAAGAGGGAGGGGAAGGTCCAACCTTACACTAAAACCCTGGATGGA
GGATGGGGATGGATGATTGTGATTCATTTTTCCTGGTGAATGTGTTTGTGATG
GGGATGACCAAGACTTTTGCAATTTCTTTGTGGTCTTTCAAGAAGAGTTTG
AAGGCACCTCAGAGCAAATTGGTTGGATTGGATCCATCATGTCATCTCTTCGT
TTTTGTGCAGGTCCCCTGGTTGCTATTATTTGTGACATACTTGGAGAGAAAAC
TACCTCCATTCTTGGGGCTTTCGTTGTTACTGGTGGATATCTGATCAGCAGCTG
GGCCACAAGTATTCCTTTTCTTTGTGTGACTATGGGACTTCTACCCGGTTTGG
GTTCTGCTTTCTTATACCAAGTGGCTGCTGTGGTAACTACCAAATACTTCAAA
AAACGATTGGCTCTTTCTACAGCTATTGCCCGTTCTGGGATGGGACTGACTTT
TCTTTTGGCACCCTTTACAAAATTCCTGATAGATCTGTATGACTGGACAGGAG
CCCTTATATTATTTGGAGCTATCGCATTGAATTTGGTGCCTTCTAGTATGCTCTT
AAGACCCATCCATATCAAAAGTGAGAACAATTCTGGTATTAAAGATAAAGGC
AGCAGTTTGTCTGCACATGGTCCAGAGGCACATGCAACAGAAACACACTGC
CATGAGACAGAAGAGTCTACCATCAAGGACAGTACTACGCAGAAGGCTGGA
CTACCTAGCAAAAATTTAACAGTCTCACAAAATCAAAGTGAAGAGTTCTACA
ATGGGCCTAACAGGAACAGACTGTTATTAAAGAGTGATGAAGAAAGTGATAA
GGTTATTTCGTGGAGCTGCAAACAACTGTTTGACATTTCTCTCTTTAGAAATC
CTTTCTTCTACATATTTACTTGGTCTTTTCTCCTCAGTCAGTTAGCATACTTCAT
CCCTACCTTTCACCTGGTAGCCAGAGCCAAAACACTGGGGATTGACATCATG
GATGCCTCTTACCTTGTTTCTGTAGCAGGTATCCTTGAGACGGTCAGTCAGAT
TATTTCTGGATGGGTTGCTGATCAAAACTGGATTAAGAAGTATCATTACCACA
AGTCTTACCTCATCCTCTGCGGCATCACTAACCTGCTTGCTCCTTTAGCCACC
ACATTTCCACTACTTATGACCTACACCATCTGCTTTGCCATCTTTGCTGGTGGT
TACCTGGCATTGATACTGCCTGTACTGGTTGATCTGTGTAGGAATTCTACAGTA
AACAGGTTTTTGGGACTTGCCAGTTTCTTTGCTGGGATGGCTGTCCTTTCTGG
ACCACCTATAGCAGGCTGGTTATATGATTATACCCAGACATACAATGGCTCTTT
 
CTACTTCTCTGGCATATGCTATCTCCTCTCTTCAGTTTCCTTTTTTTTTGTACCA
TTGGCCGAAAGATGGAAAAACAGTCTGACCTGA
Nucleotide sequence of DNA encoding mouse MCT5: NM_146136.1 (SEQ ID NO: 3) 
ATGGTGAAGAAGGAAAAACGCCCTCCCCTTACACCAAACCCCTTGATGGA
GGATGGGGGTGGATGGTCGTGCTTCATTTTTCCTGGTAAATGTGTTTGTGAT
GGGAATGACCAAGACATTTGCAATTTCTTTGTGGTTTTCAAGAAGAGTTTG
AAGGCACTTCAGAGCAAATTGGTTGGATTGGATCCATTATGTCATCACTCCGT
TTCTCTGCAGGTCCCCTGGCTGCTATCATCTGTGACGTACTTGGAGAGAAAG
CTACCTCCATTCTTGGGACGTTCCTTGTCTCTGGTGGCTATGTCATCAGCAGC
TGGGCCACAGGCATTCCCTTTCTTTGTGTGACCATGGGGCTGCTACCTGGTTT
GGGATCTGCTTTCTTGTACCAAGTAGCTGCTGTGGTAATTACCAAATATTTCA
AAAAACGATTGGGCCTTTCTACAGCAATTGCCCGTTCTGGAATGGGGCTGAC
ATTTTTGTTGGCACCCTTTACAAAATTCCTCATAGATCTTTATGACTGGACAGG
CGCTCTTATTTTATTTGGAGCAATTATATTGAACTTGGTGCCTTCCAGCATGCT
CTTAAGACCCATCCACAGCCAATCAAACAACAATTCTGACATTGAAAACAAA
GGTAGCATTTTGTCTGCAACTGAACCAGAGGCATCATACAAGACAGAGACAT
CGAAGTGCAAGGAGACACAGGAGCCTTTCATCAAAGACAGTACTATGAAGA
AATCTGAACAACCTACCACAACCTTAACTGTCTTAGGAAATCAAAGCGAAGA
GTTCAGCAATAGGCCTCACAGGAACAGGCCGTTACTTATGAGCAATGGCAAA
AGTCACAAGAAAAAGTTTGTTTCATGGAACTGCAAACAAAAACTTTTAGATA
TCTCGCTCTTTAGAAACCCTTTCTTCTACATTTTTACCTGGTCTTTTCTCCTGA
GCCAATTAGCATATTTCATTCCCACTTTCCACCTAGTAGCCAGAGCAAAAACA
CTTGGGATTGACGTAATGGATGCCTCCTACCTTGTGTCTGTAGCTGGTATCAC
TGAGACAGTCAGTCAGCTCATTTCTGGATGGATTGCTGATCAAAATTGGATCA
AGAAGTATCAATACCACAAGTCTTACCTCATTCTGTGTGGTGTGACTAACCTG
CTTGCTCCTTTAGCCACCACATTCCCACTCCTTATGGCCTATACCATCCTTTTT
GCCATTTTTGCTGGTGGCTACCTGGCACTAATACTGCCAGTATTGGTTGACCTA
TCTAAGAATTCTAGAGTACACAAGTTTTTGGGATATGCCAGTTTCTTTGCCGG
GATAGCTGTACTTTCTGGACCACCAATAGCAGGCTGGATATATGATTACACAC
AGACTTACACAGGCTCTTTCTATTTCTCTGGCACCTGCTATATCCTCTCTTCAG
TTTCCCTTTTTTTTGTACCACTGGCTGAAAGATGGAAACGCAAACAGTCTGA
CCTTCTGAGGACTACAATCAAGTAA
Nucleotide sequence of DNA encoding rat MCT5: NM_001013913.1 (SEQ ID NO: 5)

ATGAAGAAGAGGGAAGAAAAGCCCTCCCCTTACACCAAACCCCTGGATGGA
GGATGGGGATGGATGGTTGTGCTTCATTTTTCCTGGTGAATGTGTTTGTGAT
GGGAATGACCAAGACATTTGCAATTTTCTTTGTGGTTTTCAAGAAGAGTTTG
AAGGCACTTCAGAGCAAATTGGTTGGATTGGATCTATTATGTCATCACTCCGT
TTTTCTGCAGGTCCCCTGGCTGCTATTATTTGTGACATATTTGGAGAAAAAC
TACCTCCATTCTTGGGACTTTCCTTGTTTCTGGTGGCTATCTCATCAGCAGCTG

GGCCACAGGCATTCCCTTTCTTTGTGTGACCATGGGACTGCTACCTGGTTTGG
GGTCTGCTTTCTTGTACCAAGTAGCTGCTGTGGTAATTACCAAATATTTCAAA
AAACGATTGGGTCTTTCTACAGCAATTGCCCGTTCAGGGATGGGACTGACAT
TTTTGTTGGCACCCTTTACAAAACTCCTGATAGATCTTTATGACTGGACAGGT
GCTCTTATATTATTTGGAGCTATTGCATTGAATCTGGTGCCTTCCAGCATGCTC
CTAAGACCTGTCCACATCCAATCAGACAGCAATTCTGATATTAAAAACAAAG
GTAGCATTTTGTCTGCAACTGGACCAGAGTTATCACACAAGACAGAAACACC
AAACCGCAATAAGGCACAAGAGTCTCTCATCAAAGACAGTGCTGTGAAGAA
ATCTGGACAACCTACTACAACTTTAACTGTCTTAGAAAATCAAATTGAAGAGA
TTAGCACTAGGCCTCATAGGAACAGGCCGTTACTTATGAGCAATGACAAAAC
TTACAAGAAAAAGTTTGTTTCATGGAACTGCAAAGAAAAACTTCTAGATATT
TCTCTCTTTAGAAATCCTTTCTTCTACATATTTACCTGGTCTTTTCTCCTGAGTC
AACTAGCATATTTCATTCCTACCTTTCATCTGGTAGCCAGAGCAAAAACACTT
GGAATCGACATAATGGATGCCTCCTACCTTGTGTCTGTAGCTGGTATCACTGA
GACAGTCAGTCAGCTCATTTCTGGATGGATTGCAGATCAGAATTGGATCAAG
AAGTATCAATACCACAAGGCTTACCTCATCCTGTGTGGTGTTACTAACCTGCT
TGCTCCTTTAGCCACCACATTCCCACTCCTTATGGCCTATACCATCTTTTTTGC
CATTTTTGCTGGTGGCTACCTGGCACTAATACTGCCAGTATTGGTTGACCTATC
TAAGAATTCTAGAGTACACAGGTTTTTGGGATATGCCAGTTTCTTTGCTGGGA
TGGCTGTTCTTTCTGGACCACCAATAGCAGGCTGGATATACGATTATACACAG
ACATACGTGGGCTCTTTCTATTTCTCTGGCACCTGCTATATCCTCTCTTCAGTT
TCCCTTTTTTTTGTACCACTGGCTGAAAGATGGAAACTCAAGCAGTCTGACC
TTCTAAGGACTACAATCAAGTAA

As an antigen, it is possible to use a polypeptide or peptide (also collectively referred to as a peptide) comprising at least a part (whole or a part) of the amino acid sequence of MCT5, preferably a peptide comprising at least a part (whole or a part) of the amino acid sequence of the extracellular region of MCT5.

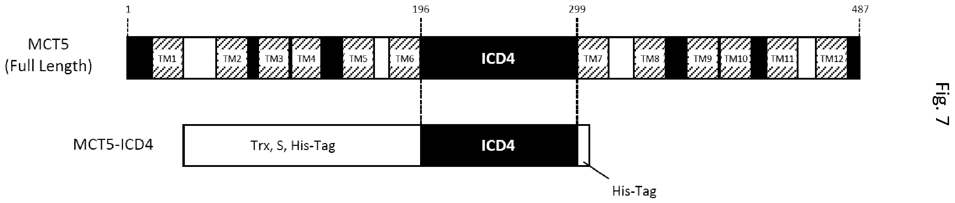

The predicted extracellular region of MCT5 has been registered as 015374 (MOT5_HUMAN) in the Uniprot database. On the database, for example in the case of human MCT5, a region consisting of amino acids at positions 196 to 299 (SEQ ID NO: 7) in the amino acid sequence shown in SEQ ID NO: 2 is predicted to be an intracellular region (Figure 7). In the case of MCT5 derived from other animals, a region corresponding to such a region is intended.

The peptide to be used as an antigen is not limited in any way as long as it is at least a part of MCT5. Preferred is at least a part of the extracellular region of MCT5, e.g., at least a part of a region consisting of amino acids at positions 196 to 299 (SEQ ID NO: 7) in the amino acid sequence of MCT5 shown in SEQ ID NO: 2. Above all, more preferred is at least a part of a region consisting of amino acids at positions 227 to 258 (SEQ ID NO: 14), and particularly preferred is at least a part of a region consisting of amino acids at positions 251 to 258 (SEQ ID NO: 13). In the present invention, a region consisting of amino acids at positions 196 to 299 in the amino acid sequence of MCT5 shown in SEQ ID NO: 2 is also referred to as "ICD4."

Moreover, MCT5 has mutants which are derived from the same mRNA but differ in their translation initiation site. For example, human MCT5 has mutants such as a peptide (SEQ ID NO: 44) lacking amino acids at positions 74 to 121, a peptide (SEQ ID NO: 45) lacking N-terminal 62 amino acids and having different amino acids at positions 63 to 73 from those of the wild type (SEQ ID NO: 2), a peptide (SEQ ID NO: 46) lacking N-terminal 110 amino acids and having different amino acids at positions 111 to 120 from those of the wild type (SEQ ID NO: 2), a peptide (SEQ ID NO: 47) lacking N-terminal 110 amino acids and having different amino acids at position 446 to the C-terminal end from those of the wild type (SEQ ID NO: 2), a peptide lacking amino acids at positions 179 to 346 and having leucine as an amino acid at position 177 in place of alanine in the wild type.

MCT5 intended in the present invention also includes these mutants.
SEQ ID NO: 44 
MLKREGKVQPYTKTLDGGWGWMIVIHFFLVNVFVMGMTKTFAIFFVVFQEEFE
GTSEQIGWIGSIMSSLRFCAGLGSAFLYQVAAVVTTKYFKKRLALSTAIARSGMG
LTFLLAPFTKFLIDLYDWTGALILFGAIALNLVPSSMLLRPIHIKSENNSGIKDKGS
SLSAHGPEAHATETHCHETEEESTIKDSTTQKAGLPSKNLTVSQNQSEEFYNGPNR
NRLLLKSDEESDKVISWSCKQLFDISLFRNPFFYIFTWSFLLSQLAYFIPTFHLVAR
AKTLGIDIMDASYLVSVAGILETVSQIISGWVADQNWIKKYHYHKSYLILCGITN
LLAPLATTFPLLMTYTICFAIFAGGYLALILPVLVDLCRNSTVNRFLGLASFFAGM
AVLSGPPIAGWLYDYTQTYNGSFYFSGICYLLSSVSFFFVPLAERWKNSLT*
SEQ ID NO: 45
   GSIMSSLRFCA
SEQ ID NO: 46
   MGMDDCDSFF
SEQ ID NO: 47
   EIIPSFQAGYMIIPRHTMALSTSLAYAISSLQFPFFLYHWPKDGKTV

MCT5 may be naturally occurring MCT5 purified from mouse, rat, human or other animal tissues or cells, or alternatively, may be produced through genetic engineering procedures. For example, a biological sample known to contain MCT5 may be fractionated into a soluble fraction and an insoluble fraction by using any type of surfactant such as Triton-X, Sarkosyl, etc. The insoluble fraction may further be dissolved in, e.g., urea or guanidine hydrochloride and then bound to any type of column, such as a heparin column, or any type of crosslinked resin to thereby obtain MCT5.

Alternatively, the peptide to be used as an antigen may be prepared by chemical synthesis or by synthesis through genetic engineering procedures using *E. coli* or the like. Techniques well known to those skilled in the art may be used for this purpose.

For chemical synthesis, the peptide may be synthesized by well-known peptide synthesis techniques. Moreover, the synthesis may be accomplished by applying either solid phase synthesis or liquid phase synthesis. A commercially available peptide synthesizer (e.g., PSSM-8, Shimadzu Corporation, Japan) may also be used for this purpose.

For peptide synthesis through genetic engineering procedures, DNA encoding the peptide is first designed and synthesized. The design and synthesis may be accomplished, for example, by PCR techniques using a vector or the like containing the full-length MCT5 gene as a template and using primers which have been designed to allow synthesis of a desired DNA region. Then, the above DNA may be ligated to an appropriate vector to obtain a recombinant vector for protein expression, and this recombinant vector may be introduced into a host, such that a desired gene can be expressed therein, thereby obtaining a transformant (Sambrook J. et al., Molecular Cloning, A Laboratory Manual (4th edition) (Cold Spring Harbor Laboratory Press (2012)).

As a vector, a phage or plasmid which is autonomously replicable in host microorganisms is used. Further, it is also possible to use an animal virus or insect virus vector. To prepare a recombinant vector, purified DNA may be cleaved with an appropriate restriction enzyme and ligated to a vector by being inserted into, e.g., an appropriate restriction enzyme site in the vector DNA. There is no particular limitation on the host for use in transformation as long as it is capable of expressing a desired gene. Examples include bacteria (e.g., *E. coli, Bacillus subtilis*), yeast, animal cells (e.g., COS cells, CHO cells), insect cells or insects. It is also possible to use a mammal (e.g., goat) as a host. Procedures for introduction of a recombinant vector into a host are known.

Moreover, the above transformant may be cultured, and a peptide for use as an antigen may be collected from the cultured product. The term "cultured product" is intended to mean either (a) a culture supernatant or (b) cultured cells or cultured microorganisms or a homogenate thereof.

After culture, when the desired peptide is produced within microorganisms or cells, the microorganisms or cells may be homogenized to thereby extract the peptide. Alternatively, when the desired peptide is produced outside microorganisms or cells, the cultured solution may be used directly or treated by centrifugation or other techniques to remove the microorganisms or cells. Then, the desired peptide may be isolated and purified by biochemical techniques commonly used for isolation and purification of peptides, as exemplified by ammonium sulfate precipitation, gel filtration, ion exchange chromatography, affinity chromatography and so on, which may be used either alone or in combination as appropriate.

In the present invention, the peptide to be used as an antigen may also be obtained by *in vitro* translation using a cell-free synthesis system. In this case, it is possible to use two methods, i.e., a method in which RNA is used as a template and a method in which DNA is used as a template (transcription/translation). As a cell-free synthesis system, a commercially available system may be used, as exemplified by an Expressway^{™} system (Invitrogen), etc.

The peptide obtained as described above may also be linked to an appropriate carrier protein such as bovine serum albumin (BSA), keyhole limpet hemocyanin (KLH), human thyroglobulin, avian gamma globulin, etc.

Moreover, the antigen intended in the present invention encompasses not only (a) a polypeptide comprising the amino acid sequence shown in SEQ ID NO: 2 or 7, but also (b) a polypeptide comprising an amino acid sequence with deletion, substitution or addition of one or several amino acids in the amino acid sequence shown in SEQ ID NO: 12 or 14, and (c) a polypeptide comprising an amino acid sequence sharing a homology of 70% or more with the amino acid sequence shown in SEQ ID NO: 13.

In the context of the present invention, the "polypeptide comprising the amino acid sequence shown in SEQ ID NO: 2 or 7" includes a polypeptide consisting of the amino acid sequence shown in SEQ ID NO: 12, 13 or 14.

Moreover, the "amino acid sequence with deletion, substitution or addition of one or several amino acids in the amino acid sequence shown in SEQ ID NO: 2 or 7" includes, for example:
(i) an amino acid sequence with deletion of 1 to 10 amino acids (e.g., 1 to 5 amino acids, preferably 1 to 3 amino acids, more preferably 1 or 2 amino acids, even more preferably a single amino acid) in the amino acid sequence shown in SEQ ID NO: 2 or 7;
(ii) an amino acid sequence with substitution of other amino acids for 1 to 5 amino acids (e.g., 1 to 4 amino acids, preferably 1 to 3 amino acids, more preferably 1 or 2 amino acids, even more preferably a single amino acid) in the amino acid sequence shown in SEQ ID NO: 12 or 14;
(iii) an amino acid sequence with addition of 1 or 2 amino acids (e.g., 1 or 2 amino acids, preferably a single amino acid) to the amino acid sequence shown in SEQ ID NO: 13; and
(iv) an amino acid sequence mutated by any combination of (i) to (iii) shown above.

In addition, MCT5 intended in the present invention encompasses not only a polypeptide comprising the amino acid sequence shown in SEQ ID NO: 2 or 7, but also a polypeptide comprising an amino acid sequence sharing a homology (identity) of 70% or more with the amino acid sequence shown in SEQ ID NO: 2 or 7. Such a polypeptide also includes those comprising amino acid sequences sharing a homology of about 70% or more, 75% or more, about 80% or more, 85% or more, 90% or more, 95% or more, 96% or more, 97% or more, 98% or more, or 99% or more with the amino acid sequence shown in SEQ ID NO: 2 or 7 (i.e., amino acid sequences substantially equivalent to the amino acid sequence shown in SEQ ID NO: 2 or 7). For homology determination, it is possible to use homology search tools such as FASTA, BLAST, PSI-BLAST and so on in a homology search site on the Internet (e.g., DNA Data Bank of Japan (DDBJ)). Alternatively, it is also possible to conduct a BLAST search in the National Center for Biotechnology Information (NCBI).

To prepare the above mutation-carrying proteins, mutations may be introduced into a gene (DNA) encoding the protein by using a kit for mutation introduction based on site-directed mutagenesis (e.g., Kunkel method or Gapped duplex method), as exemplified by a QuikChange^{™} Site-Directed Mutagenesis Kit (Stratagene), GeneTailor^{™} Site-Directed Mutagenesis Systems (Invitrogen), TaKaRa Site-Directed Mutagenesis Systems (e.g., Mutan-K, Mutan-Super Express Km; Takara Bio Inc., Japan). Alternatively, it is also possible to use techniques for site-directed mutagenesis as described in "Molecular Cloning, A Laboratory Manual (4th edition)" (Cold Spring Harbor Laboratory Press (2012)), etc.

In the present invention, the gene to be introduced into cells or the like may be a gene encoding MCT5 or a partial fragment thereof or a mutated peptide thereof. As such a gene, it is possible to use a gene comprising the nucleotide sequence shown in SEQ ID NO: 1, 3 or 5, by way of example.

Alternatively, the gene to be introduced into cells or the like may also be a gene comprising a nucleotide sequence or a partial sequence thereof, which is hybridizable under stringent conditions with a sequence complementary to the nucleotide sequence shown in SEQ ID NO: 1, 3 or 5.

In the context of the present invention, the term "stringent conditions" may be any of low stringent conditions, moderately stringent conditions and high stringent conditions. "Low stringent conditions" refer to, for example, conditions of 5 × SSC, 5 × Denhardt's solution, 0.5% SDS, 50% formamide and 32°C. Likewise, "moderately stringent conditions" refer to, for example, conditions of 5 × SSC, 5 × Denhardt's solution, 0.5% SDS, 50% formamide and 42°C. "High stringent conditions" refer to, for example, conditions of 5 × SSC, 5 × Denhardt's solution, 0.5% SDS, 50% formamide and 50°C. Under these conditions, it can be expected that DNA having a higher homology is more efficiently obtained at a higher temperature. However, the stringency of hybridization would be affected by a plurality of factors, including temperature, probe concentration, probe length, ionic strength, reaction time, salt concentration and so on. Those skilled in the art would be able to achieve the same stringency by selecting these factors as appropriate.

Moreover, the gene encoding MCT5 for use in the present invention also encompasses a gene comprising a nucleotide sequence sharing a homology of 60% or more with the nucleotide sequence shown in SEQ ID NO: 1, 3 or 5. Such a gene may be exemplified by a gene sharing a homology of 60% or more, 70% or more, 80% or more, 85% or more, 90% or more, 95% or more, 96% or more, 97% or more, 98% or more, or 99% or more with the nucleotide sequence shown in SEQ ID NO: 1, 3 or 5, as calculated by homology search software such as FASTA or BLAST using default parameters.

The above gene encoding MCT5 or mutants thereof can be obtained, for example, on the basis of the nucleotide sequences found in the NCBI GenBank database by using known genetic engineering procedures as described in "Molecular Cloning, A Laboratory Manual (4th edition)" (Cold Spring Harbor Laboratory Press (2012)).

### (2) Preparation of polyclonal antibodies

The thus prepared MCT5 or partial peptide thereof is administered directly or together with a carrier or diluent to immunize non-human mammals (e.g., rabbits, dogs, guinea pigs, mice, rats, goats). The amount of the antigen to be administered per animal is 1 µg to 10 mg in the case of using an adjuvant. Examples of an adjuvant include Freund's complete adjuvant (FCA), Freund's incomplete adjuvant (FIA), aluminum hydroxide adjuvant and so on. Immunization is accomplished primarily by injection via the intravenous, subcutaneous or intraperitoneal route, etc. Moreover, the interval between immunizations is not limited in any way, and immunization may be repeated twice to 20 times, preferably 5 to 15 times, at intervals of several days to several weeks, preferably at intervals of 1 or 2 weeks.

Those skilled in the art would be able to determine the interval between immunizations in consideration of the resulting antibody titers. Preferably, at the time of repeating subcutaneous immunization 3 to 4 times, blood is sampled and measured for antibody titers. Antibody titers in serum may be measured by ELISA (enzyme-linked immunosorbent assay), EIA (enzyme immunoassay), radioimmunoassay (RIA), etc. After antibody titers have been confirmed to rise sufficiently, blood may be collected completely and treated in a commonly used manner to separate and purify antibodies. For separation and purification, known techniques such as salting out with ammonium sulfate, ion exchange chromatography, gel filtration chromatography, affinity chromatography and so on may be selected as appropriate or used in combination. More specifically, serum containing desired antibodies may be passed through a column on which proteins other than MCT5 have been immobilized, and fractions passing through the column may be collected to thereby obtain polyclonal antibodies with improved specificity to MCT5.

### (3) Preparation of monoclonal antibodies

### (i) Collection of antibody-producing cells

As in the case of polyclonal antibody preparation, MCT5 or a partial peptide thereof (e.g., ICD4 (SEQ ID NO: 7) or ICD4_251-258 (SEQ ID NO: 13)) is administered directly or together with a carrier or diluent to immunize non-human mammals. The amount of the antigen to be administered per animal, the type of adjuvant to be used, the method of immunization and the interval between immunizations are the same as those used for preparation of polyclonal antibodies. After 1 to 30 days, preferably 2 to 5 days, from the final immunization date, animals showing antibody titers may be selected to collect antibody-producing cells. Antibody-producing cells may be exemplified by spleen cells, lymph node cells, peripheral blood cells and so on, with spleen cells or lymph node cells being preferred.

### (ii) Cell fusion

To obtain hybridomas, cell fusion is conducted between antibody-producing cells and myeloma cells. Operations for cell fusion may be accomplished in a known manner, for example, according to the method of Kohler et al. As myeloma cells to be fused with antibody-producing cells, it is possible to use generally available established cell lines of mouse or other animal origin. Cell lines preferred for use are those having drug selectivity and having the property of not surviving in HAT selective medium (i.e., a medium containing hypoxanthine, aminopterin and thymidine) in an unfused state, but surviving only when fused with antibody-producing cells. Examples of myeloma cells include mouse myeloma cell lines (e.g., P3X63-Ag8, P3X63-Ag8U.1, SP2/O-Ag14, PAI, P3U1, NSI/1-Ag4-1, NSO/1) and rat myeloma cell lines (e.g., YB2/0), etc.

Cell fusion between the above myeloma cells and antibody-producing cells may be accomplished as follows: in a serum-free medium for animal cell culture (e.g., DMEM or RPMI-1640 medium), 1 × 10⁸ to 5 × 10⁸ antibody-producing cells may be mixed with 2 × 10⁷ to 10 × 10⁷ myeloma cells (the ratio of antibody-producing cells to myeloma cells is 10:1 to 1:1) to cause fusion reaction in the presence of a cell fusion promoter. As a cell fusion promoter, it is possible to use polyethylene glycol having an average molecular weight of 1000 to 6000 daltons or Sendai virus, etc. Alternatively, a commercially available cell fusion apparatus using electrical stimulation (e.g., electroporation) may also be used to cause cell fusion between antibody-producing cells and myeloma cells.

### (iii) Screening and cloning of hybridomas

After cell fusion, the cells are screened to select desired hybridomas. For screening, a cell suspension may be diluted as appropriate with, e.g., RPMI-1640 medium containing 10% to 20% fetal bovine serum and then seeded by limiting dilution in microtiter plates at a density of about 0.3 cells/well by calculation, and a selective medium (e.g., HAT medium) may be added to each well, followed by culture while replacing the selective medium as appropriate. As a result, cells growing at around 10 days after the initiation of culture in the selective medium may be obtained as hybridomas.

Subsequently, the growing hybridomas are further screened. Screening of these hybridomas is not limited in any way and may be conducted in accordance with commonly used procedures. For example, aliquots of the culture supernatants contained in the wells where hybridomas have been cultured may be sampled and screened by enzyme immunoassay, radioimmunoassay, etc. More specifically, an antigen is adsorbed to 96-well plates, and the plates are then blocked with calf serum, skimmed milk, etc. The culture supernatants of hybridoma cells are reacted with the immobilized antigen at 37°C for 1 hour and then reacted with peroxidase labeled anti-mouse IgG at 37°C for 1 hour, followed by color development using orthophenylenediamine as a substrate. After the reaction is stopped with an acid, the plates are measured for absorbance at a wavelength of 490 nm for screening purposes.

Monoclonal antibody-producing hybridomas found to be positive when measured in the above manner are cloned by limiting dilution or other techniques to thereby finally establish hybridomas which are cells producing monoclonal antibodies specifically binding to MCT5.

Cell lines (hybridomas) producing the monoclonal antibodies of the present invention may be exemplified by "Mouse-Mouse hybridoma IMI4C4a" (hereinafter referred to as "IMI4C4a") and "Mouse-Mouse hybridoma IMI4C12a" (hereinafter referred to as "IMI4C12a"). Upon culture of these hybridomas, homogeneous monoclonal antibodies can be prepared.

### (iv) Collection of monoclonal antibodies

For collection of monoclonal antibodies from the establish hybridomas, commonly used procedures, e.g., cell culture-based procedures or ascites formation procedures may be used for this purpose. In cell culture-based procedures, hybridomas are cultured in an animal cell culture medium (e.g., 10% fetal bovine serum-containing RPMI-1640 medium, MEM medium or serum-free medium) under standard culture conditions (e.g., 37°C, 5% CO₂ concentration) for 7 to 14 days, and antibodies are obtained from their culture supernatants. In the case of ascites formation procedures, hybridomas are intraperitoneally administered at about 5 × 10⁶ to 2 × 10⁷ cells to animals of the same species as the mammal from which myeloma cells are derived, e.g., mice (BALB/c), whereby the hybridomas are allowed to grow in abundance. Then, their ascites are collected after 1 to 2 weeks. In cases where antibodies are required to be purified in the above antibody collection procedures, known techniques such as salting out with ammonium sulfate, ion exchange chromatography, gel filtration, affinity chromatography and so on may be selected as appropriate or used in combination for purification purposes.

A preferred example of the anti-MCT5 antibody of the present invention is an antibody in which the amino acid sequences of complementarity determining regions (CDRs) 1 to 3 in the heavy chain variable region (VH) comprise or consist of the amino acid sequences shown in SEQ ID NOs: 18, 19 and 20, respectively, and/or the amino acid sequences of complementarity determining regions (CDRs) 1 to 3 in the light chain variable region (VL) comprise or consist of the amino acid sequences shown in SEQ ID NOs: 26, 27 and 28, respectively. In another embodiment, a preferred example of the anti-MCT5 antibody of the present invention is an antibody in which the amino acid sequence of the heavy chain variable region (VH) comprises or consists of the amino acid sequence shown in SEQ ID NO: 17 (which is encoded by the nucleotide sequence shown in SEQ ID NO: 16), and/or the amino acid sequence of the light chain variable region (VL) comprises or consists of the amino acid sequence shown in SEQ ID NO: 25 (which is encoded by the nucleotide sequence shown in SEQ ID NO: 24).

### H chain V region sequence

DNA sequence (SEQ ID NO: 16) 
ATGAAATGCAGCTGGGTCATCTTCTTCCTGATGGCAGTGGTTACAGGGGTCAA
TTCAGAGGTTCAGCTGCAGCAGTCTGGGGCTGAGCTTGTGAGGCCAGGGGC
CTTAGTCAAGTTGTCCTGCAAAGCTTCTGGCTTCAACATTAAAGACTACTATA
TGTTCTGGGTGAAGCAGAGGCCTGAACAGGGCCTGGAGTGGATTGGATGGA
TTGATCCTGAGAATGGTAATACTATATTTGACCCGAAGTTCCAGGGCAAGGCC
AGTATAACAGCAGACACATCCTCCAACACAGCCTACCTGCAGCTCAGCAGCC
TGACATCTGAGGACACTGCCGTCTATTACTGTGCTAGAATGATTACGACCTATT
ACTATGCTATGGACTTCTGGGGTCAAGGAACCTCAGTCACCGTCTCCTCAGC
C
Amino acid sequence (SEQ ID NO: 17)

MKCSWVIFFLMAVVTGVNSEVQLQQSGAELVRPGALVKLSCKASGFNIKDYYM
FWVKQRPEQGLEWIGWIDPENGNTIFDPKFQGKASITADTSSNTAYLQLSSLTSE
DTAVYYCARMITTYYYAMDFWGQGTSVTVSSA

### H chain CDR regions

VH-CDR1: FNIKDYYMF (46-54) (SEQ ID NO: 18)
VH-CDR2: WIDPENGNTIFDPKFQGK (69-86) (SEQ ID NO: 19)
VH-CDR3: MITTYYYAMDFW (118-129) (SEQ ID NO: 20)

### H chain CDR regions

VH-CDR1: FNIKDYYMF (46-54) (SEQ ID NO: 18)
VH-CDR2: WIDPENGNTIFDPKFQGK (69-86) (SEQ ID NO: 19)
VH-CDR3: MITTYYYAMDFW (118-129) (SEQ ID NO: 20)

### L chain V region sequence

DNA sequence (SEQ ID NO: 24) 
ATGGCCTGGACTCCTCTCTTCTTCTTCTTTGTTCTTCATTGCTCAGGTTCTTTC
TCCCAACTTGTGCTCACTCAGTCATCTTCAGCCTCTTTCTCCCTGGGAGCCTC
AGCAAAACTCACGTGCACCTTGAGTAGTCAGCACAGTACGTACACCATTGAA
TGGTATCAGCAACAGCCACTCAAGCCTCCTAAGTATGTGATGGAGCTTAAGA
AAGATGGAAGCCACAGCACAGGTGATGGGATTCCTGATCGCTTCTCTGGATC
CAGCTCTGGTGCTGATCGCTACCTTAGCGTTTCCAGCATCCAGCCTGAAGATG
AAGCAATATACATCTGTGGTGTGGGTTATACAATTAAGGAACAATTTGTGTAT
GTTTTCGGCGGTGGAACCAAG
Amino acid sequence (SEQ ID NO: 25)

MAWTPLFFFFVLHCSGSFSQLVLTQSSSASFSLGASAKLTCTLSSQHSTYTIEWY
QQQPLKPPKYVMELKKDGSHSTGDGIPDRFSGSSSGADRYLSVSSIQPEDEAIYI
CGVGYTIKEQFVYVFGGGTK

### L chain CDR regions

VL-CDR1: SQHSTYT (45-51) (SEQ ID NO: 26)
VL-CDR2: ELKKDGSHST (68-77) (SEQ ID NO: 27)
VL-CDR3: GYTIKEQ (114-120) (SEQ ID NO: 28)

### (v) Epitope for anti-MCT5 antibody

The epitope (antigenic determinant) for the anti-MCT5 antibody of the present invention is not limited in any way as long as it is at least a part of the antigen MCT5, but it is preferably at least a part of the extracellular region of MCT5, for example, at least a part of a region consisting of amino acids at positions 196 to 299 (SEQ ID NO: 7) in the amino acid sequence of MCT5 shown in SEQ ID NO: 2. Above all, preferred is at least a part of a region consisting of amino acids at positions 227 to 258 (SEQ ID NO: 14), and particularly preferred is at least a part of a region consisting of amino acids at positions 251 to 258 (SEQ ID NO: 13). The anti-MCT5 antibody recognizing such a region (binding to such a region) is, for example, very useful for use in detection and diagnosis of cancer as described later because of ensuring high detection sensitivity for MCT5 in biological samples.

Moreover, as described above, MCT5 has four deletion mutants. More specifically, human MCT5 has deletion mutants such as a peptide (SEQ ID NO: 44) lacking amino acids at positions 74 to 121, a peptide (SEQ ID NO: 45) lacking N-terminal 62 amino acids and having different amino acids at positions 63 to 73 from those of the wild type (SEQ ID NO: 2), a peptide (SEQ ID NO: 46) lacking N-terminal 110 amino acids and having different amino acids at positions 111 to 120 from those of the wild type (SEQ ID NO: 2), a peptide (SEQ ID NO: 47) lacking N-terminal 110 amino acids and having different amino acids at position 446 to the C-terminal end from those of the wild type (SEQ ID NO: 2), a peptide lacking amino acids at positions 179 to 346 and having leucine as an amino acid at position 177 in place of alanine in the wild type, etc.

Among these peptides, except for the peptide lacking amino acids at positions 179 to 346 and having leucine as an amino acid at position 177 in place of alanine in the wild type, the remaining three peptides are identical with wild-type MCT5 in a region consisting of amino acids at positions 196 to 299 in the amino acid sequence of MCT5 shown in SEQ ID NO: 2. Thus, the antibody of the present invention allows detection of these deletion mutants.

Moreover, the epitope (antigenic determinant) for the anti-MCT5 antibody of the present invention also includes at least a part of the corresponding region in MCT5 of other animal origin.

The antibody against MCT5 of the present invention includes an antibody binding to a site (e.g., epitope) to which the antibody binds, as exemplified by an antibody binding to a site to which an antibody produced by the hybridoma of the present invention binds.

### (4) Preparation of genetically recombinant antibodies

A preferred embodiment of the antibody of the present invention may be a genetically recombinant antibody. Examples of a genetically recombinant antibody include, but are not limited to, a chimeric antibody, a humanized antibody and a reconstituted human antibody, etc.

A chimeric antibody (i.e., a humanized chimeric antibody) is an antibody in which antibody variable regions of mouse origin are linked (conjugated) to constant regions of human origin (see, e.g., Proc. Natl. Acad. Sci. U.S.A. 81, 6851-6855 (1984)). For preparation of a chimeric antibody, gene recombination technology may be used for its construction such that the thus linked antibody is obtained.

In this regard, the antibody variable regions of mouse origin are preferably composed of a heavy chain variable region which comprises or consists of, for example, the amino acid sequence shown in SEQ ID NO: 17 and a light chain variable region which comprises or consists of, for example, the amino acid sequence shown in SEQ ID NO: 25.

For preparation of a humanized antibody, it is possible to use a process referred to as so-called CDR grafting (CDR transplantation). CDR grafting is a technique to prepare reconstituted variable regions whose framework regions (FRs) are of human origin and whose CDRs are of mouse origin by transplanting complementarity determining regions (CDRs) from mouse antibody variable regions to human variable regions. Next, these humanized reconstituted human variable regions are linked to human constant regions. Procedures for preparation of such a humanized antibody are well known in the art (see, e.g., Nature, 321, 522-525 (1986); J. Mol. Biol., 196, 901-917 (1987); Queen C et al., Proc. Natl. Acad. Sci. USA, 86: 10029-10033 (1989); Japanese Patent No. 2828340). In this regard, the amino acid sequences of CDRs of mouse origin which can be used for the humanized anti-MCT5 antibody of the present invention are preferably, but not limited to, the amino acid sequences shown in SEQ ID NOs: 18, 19 and 20 for heavy chain variable region CDRs 1 to 3, respectively, and the amino acid sequences shown in SEQ ID NOs: 26, 27 and 28 for light chain variable region CDRs 1 to 3, respectively, by way of example.

A reconstituted human antibody (complete human antibody) is generally an antibody in which hyper variable regions serving as antigen-binding sites in its variable regions (V regions), the other regions in its V regions and its constant regions have the same structures as those of human antibody. Techniques for reconstituted human antibody preparation are also known, and in the case of gene sequences common to humans, an approach has been established for their preparation by genetic engineering procedures. Such a reconstituted human antibody may be obtained, for example, by using human antibody-producing mice which have human chromosome fragments comprising genes for human antibody heavy chain (H chain) and light chain (L chain) (see, e.g., Tomizuka, K. et al., Nature Genetics, (1977) 16, 133-143; Kuroiwa, Y. et al., Nuc. Acids Res., (1998) 26, 3447-3448; Yoshida, H. et al., Animal Cell Technology: Basic and Applied Aspects, (1999) 10, 69-73 (Kitagawa, Y., Matuda, T. and Iijima, S. eds.), Kluwer Academic Publishers; Tomizuka, K. et al., Proc. Natl. Acad. Sci. USA, (2000) 97, 722-727) or by obtaining a phage display-derived human antibody selected from human antibody libraries (see, e.g., Wormstone, I. M. et al, Investigative Ophthalmology & Visual Science., (2002) 43 (7), 2301-8; Carmen, S. et al., Briefings in Functional Genomics and Proteomics, (2002) 1 (2), 189-203; Siriwardena, D. et al., Opthalmology, (2002) 109 (3), 427-431).

Alternatively, in the present invention, a hybridoma or DNA or RNA extracted from this hybridoma may be used as a starting material to prepare a chimeric antibody, a humanized antibody or a reconstituted human antibody in accordance with the well-known approaches mentioned above.

Further, a protein fused with the antibody of the present invention may be prepared from the antibody variable regions and any other protein by known gene recombination techniques. Alternatively, such a fusion protein may be prepared by crosslinking the monoclonal antibody with any other protein using a crosslinker.

### (5) Preparation of antibody fragments

A fragment of the antibody against MCT5 for use in the present invention specifically binds to MCT5.

A fragment of the antibody is intended to mean a partial region of the antibody of the present invention, and examples include Fab, Fab', F(ab')₂, Fv, diabody (dibodies), dsFv, scFv (single chain Fv) and so on. The above antibody fragments may be obtained by cleaving the antibody of the present invention with various proteases depending on the intended purpose.

For example, Fab may be obtained by treating an antibody molecule with papain, while F(ab')₂ may be obtained by treating an antibody molecule with pepsin. Likewise, Fab' may be obtained by cleaving the disulfide bonds in the hinge region of the above F(ab')₂.

In the case of scFv, cDNAs encoding antibody heavy chain variable region (H chain V region) and light chain variable region (L chain V region) may be obtained to construct DNA encoding scFv. This DNA may be inserted into an expression vector, and the resulting expression vector may be introduced into a host organism to cause expression, whereby scFv may be prepared.

In the case of diabody, cDNAs encoding antibody H chain V region and L chain V region may be obtained to construct DNA encoding scFv such that the amino acid sequence of a peptide linker has a length of 8 residues or less. This DNA may be inserted into an expression vector, and the resulting expression vector may be introduced into a host organism to cause expression, whereby diabody may be prepared.

In the case of dsFv, cDNAs encoding antibody H chain V region and L chain V region may be obtained to construct DNA encoding dsFv. This DNA may be inserted into an expression vector, and the resulting expression vector may be introduced into a host organism to cause expression, whereby dsFv may be prepared.

In the present invention, the nucleotide sequence of DNA encoding the heavy chain variable region may be exemplified by those comprising or consisting of the nucleotide sequence shown in SEQ ID NO: 16, while the nucleotide sequence of DNA encoding the light chain variable region may be exemplified by those comprising or consisting of the nucleotide sequence shown in SEQ ID NO: 24.

Moreover, specific examples of the antibody fragment of the present invention include, but are not limited to, antibody fragments comprising the amino acid sequences shown in SEQ ID NOs: 18, 19 and 20 for the amino acid sequences of VH CDRs 1 to 3, respectively, and/or comprising the amino acid sequences shown in SEQ ID NOs: 26, 27 and 28 for the amino acid sequences of VL CDRs 1 to 3, respectively. Further examples include antibody fragments comprising the amino acid sequence shown in SEQ ID NO: 17 for VH, and/or comprising the amino acid sequence shown in SEQ ID NO: 25 for VL.

A CDR-containing antibody fragment (peptide) is configured to comprise at least one or more regions of VH or VL CDRs (CDRs 1 to 3). In the case of an antibody fragment containing a plurality of CDRs, these CDRs may be linked directly or via an appropriate peptide linker. For preparation of a CDR-containing antibody fragment, DNA encoding antibody VH and VL CDRs may be constructed, and this DNA may be inserted into an expression vector for prokaryotic organisms or an expression vector for eukaryotic organisms, and the resulting expression vector may be introduced into a prokaryotic organism or a eukaryotic organism to cause expression. Alternatively, a CDR-containing peptide may also be prepared by chemical synthesis such as Fmoc method (fluorenylmethyloxycarbonyl method) and tBoc method (t-butyloxycarbonyl method).

The nucleotide sequences encoding VH CDRs 1 to 3 are preferably the nucleotide sequences shown in SEQ ID NOs: 21, 22 and 23, respectively, by way of example, while the nucleotide sequences encoding VL CDRs 1 to 3 are preferably the nucleotide sequences shown in SEQ ID NOs: 29, 30 and 31, respectively, by way of example.

VH-CDR1 nucleotide sequence (SEQ ID NO: 21)
TTCAACATTAAAGACTACTATATGTTC VH-CDR2 nucleotide sequence (SEQ ID NO: 22) 
TGGATTGATCCTGAGAATGGTAATACTATATTTGACCCGAAGTTCCAGGGCA
AG
VH-CDR3 nucleotide sequence (SEQ ID NO: 23)
ATGATTACGACCTATTACTATGCTATGGACTTCTGG

VL-CDR1 nucleotide sequence (SEQ ID NO: 29)
AGTCAGCACAGTACGTACACC VL-CDR2 nucleotide sequence (SEQ ID NO: 30)
GAGCTTAAGAAAGATGGAAGCCACAGCACA VL-CDR3 nucleotide sequence (SEQ ID NO: 31)
GGTTATACAATTAAGGAACAA

### (6) Binding affinity

The binding affinity can be determined from the binding constant (KA) and the dissociation constant (KD). The affinity equilibrium constant (K) is expressed as the KA/KD ratio. The binding affinity may be detected in the following manner.

The antibody of the present invention has a dissociation constant (KD) of at least 1 × 10⁻¹⁰ M and has an affinity which is, for example, 2- to 5-fold, 5- to 10-fold, 10- to 100-fold, 100- to 1000-fold or 1000- to 10,000-fold higher than this dissociation constant. More specifically, the dissociation constant (KD) of the antibody of the present invention in relation to MCT5 binding affinity is 1 × 10⁻¹⁰ M, 5 × 10⁻¹¹ M, 1 × 10⁻¹¹ M, 5 × 10⁻¹² M, 1 × 10⁻¹² M, 5 × 10⁻¹³ M, 1 × 10⁻¹³ M, 5 × 10⁻¹⁴ M, 1 × 10⁻¹⁴ M, 5 × 10⁻¹⁵ M or 1 × 10⁻¹⁵ M, and is preferably 1 × 10⁻¹⁰ M to 1 × 10⁻¹³ M. Alternatively, the antibody of the present invention may have a value lower than these KD values and a higher affinity.

In this regard, if the dissociation constant (KD) of an antibody to be measured for its affinity is within about 1- to 100-fold of the KD of the antibody of the present invention, this antibody is regarded as being substantially the same as the antibody of the present invention and therefore falls within the present invention.

The binding constant (KA) and the dissociation constant (KD) may be measured by surface plasmon resonance (SPR), and any known instrument and method which allow real-time detection and monitoring of the binding rate may be used for this purpose (e.g., Biacore^{®} T200 (GE Healthcare), ProteON XPR36 (Bio-Rad)).

### 3. Reagent for detection or diagnosis of cancer (agent for detection or diagnosis of cancer)

The reagent for detection or diagnosis of cancer according to the present invention comprises the antibody or fragment thereof described above in the section "2. Antibody against MCT5 (anti-MCT5 antibody)." Since the antibody of the present invention is capable of specifically binding to MCT5 expressed in cancer, the reagent of the present invention comprising this antibody can be used for detection or diagnosis of cancer.

Examples of cancer to be detected or diagnosed in the present invention include breast cancer, colorectal cancer, lung cancer, ovarian cancer, uterine cancer, uterine cervical cancer, esophageal cancer, thyroid cancer, skin cancer, gastric cancer, pancreatic cancer, brain tumor, cervical cancer, tongue cancer, small intestinal cancer, duodenal cancer, bladder cancer, kidney cancer, liver cancer, prostate cancer, gallbladder cancer, pharyngeal cancer, sarcoma, melanoma, leukemia, lymphoma, multiple myeloma and so on, each having invasive capacity. Preferred are cancers in which MCT5 is significantly highly expressed when compared to normal cells or tissues. Examples of such cancers include breast cancer, colorectal cancer, lung cancer, ovarian cancer, uterine cancer, uterine cervical cancer, esophageal cancer, thyroid cancer, skin cancer and so on, with breast cancer, colorectal cancer and lung cancer being preferred. The expression "having invasive capacity" is intended to mean the property of cancer cells to actively grow in tumor tissues and also spread to surrounding tissues or organs. To spread to surrounding tissues or organs, cancer cells are required to migrate while degrading the extracellular matrix (basement membrane) present in the space between tissues or organs, and cancer cells having such a property can be regarded as cancer having invasive capacity. This property may be evaluated, for example, as the ability to pass through Matrigel which mimics the extracellular matrix, and may be readily analyzed by measurement using a Matrigel invasion chamber, etc.

The reagent of the present invention may further comprise a pharmaceutically acceptable carrier, in addition to the antibody of the present invention. The term "pharmaceutically acceptable carrier" refers to any type of carrier (e.g., liposomes, lipid vesicles, micelles), diluent, excipient, wetting agent, buffering agent, suspending agent, lubricant, adjuvant, emulsifier, disintegrant, absorbent, preservative, surfactant, coloring agent, flavoring agent or the like, which is suitable for the reagent of the present invention.

In another embodiment, the present invention provides the use of a monoclonal antibody against MCT5 or a fragment thereof for the manufacture of a reagent for detection or diagnosis of cancer. Moreover, the present invention also provides a monoclonal antibody against MCT5 or a fragment thereof for use in the detection or diagnosis of cancer. Further, the present invention provides a monoclonal antibody against MCT5 or a fragment thereof for use as a reagent for detection or diagnosis of cancer. Such a monoclonal antibody against MCT5 or a fragment thereof is as described above in the section "2. Antibody against MCT5 (anti-MCT5 antibody)."

### 4. Method for detection or diagnosis of cancer

The method for detection or diagnosis of cancer according to the present invention comprises the step of contacting the antibody or fragment thereof described above in the section "2. Antibody against MCT5 (anti-MCT5 antibody)" with a biological sample taken from a subject (hereinafter also simply referred to as a "biological sample") to thereby detect MCT5 in the sample. In this case, it is preferred that the detection results of MCT5 are connected with the possibility of cancer.

In the context of the present invention, the term "subject" is intended to include, for example, humans, rabbits, guinea pigs, rats, mice, hamsters, cats, dogs, goats, pigs, sheep, cows, horses, monkeys and other mammals, with humans being preferred.

Likewise, the biological sample is not limited in any way and refers to, for example, mammalian tissue or cells, a homogenate or extract thereof, body fluid or excrement per se or a sample containing any of them. Moreover, the biological sample also includes samples obtained from mammalian tissue or cells, a homogenate or extract thereof, body fluid and excrement through any type of treatment, e.g., dilution, separation, nucleic acid or protein extraction, protein denaturation, etc. A biological sample preferred for screening of colorectal cancer or gastric cancer is body fluid or excrement, particularly feces. For screening of bladder cancer or kidney cancer, it is possible to use tissue or urine. Likewise, for screening of breast cancer or uterine cancer, it is possible to use mother's milk, sanitary goods or the like. Any biological samples may be applied by being pretreated appropriately.

When feces is used as a sample, the sample is preferably prepared by the method described in JP 2005-46065 A. This method is designed to efficiently collect cancer cells from feces. More specifically, feces and a buffer are introduced into a stomacher bag to prepare a suspension of feces. This suspension is filtered through a multi-filter apparatus to capture cells on the final filter whose bore size is set to 10 µm or less, from which the cells are then collected using Ber-EP4 antibody-bound magnetic beads (Dynabeads Epithelial Enrich, Dynal Biotech). In addition to this, efficient cell collection techniques (e.g., Percoll centrifugation) commonly used by those skilled in the art may be used for this purpose.

In various cancers including colorectal cancer and breast cancer, any tumor marker has not yet been established which allows early detection using body fluid. Commonly used markers may show false negative results even in advanced stages of cancer. In contrast, when using the antibody of the present invention, a trace amount of cells, which express MCT5 serving as a marker, contained in body fluid or excrement can be detected and/or measured before cancer has progressed.

If the "subject" is a human, the subject includes cancer patients (e.g., early cancer patients, advanced cancer patients) and normal subjects. For example, in the case of colorectal cancer, gastric cancer or the like, early cancer refers to a state where cancer cells remain in the mucosa or submucosa, while advanced cancer refers to a state where cancer cells have reached the muscularis propria or deeper layers. Based on these criteria, patients may be categorized and provided for the detection of the present invention. Moreover, as a blood sample, preferred is peripheral blood and more preferred is peripheral blood-derived serum. Further, tissues include cancer tissues and normal tissues which may develop cancer.

In the context of the present invention, the term "contact" is intended to mean that the antibody of the present invention and a biological sample taken from a subject are allowed to exist in the same reaction system, as exemplified by mixing the antibody of the present invention and the biological sample in a reaction well, adding the antibody of the present invention to the biological sample, adding either the antibody of the present invention or the biological sample to a carrier on which the other is immobilized, etc.

In the present invention, for MCT5 detection or for measurement or evaluation of MCT5 expression levels, it is possible to use, for example, enzyme immunoassay (EIA), fluorescence immunoassay (FIA), radioimmunoassay (RIA), chemiluminescence immunoassay (CIA), turbidimetric immunoassay, immunonephelometry, latex agglutination, latex turbidimetry, hemagglutination reaction, particle agglutination reaction, Western blotting, immunostaining, immunoprecipitation, immunochromatography and so on.

Any device may be used for such detection and other purposes, and examples include a micro-well plate, an array, a chip, a flow cytometer, a surface plasmon resonance apparatus, an immunochromatographic strip and so on. In the case of using such a device, signals (e.g., color development, fluorescence intensity, luminescence intensity) can be detected, measured and/or evaluated, and the results of detection, measurement or evaluation can be connected with the possibility of cancer, etc.

In the present invention, based on the results of MCT5 detection or on the results of measurement or evaluation of MCT5 expression levels, a subject can be diagnosed for the presence or absence of the possibility of suffering from cancer.

In cases where the detection or diagnostic method of the present invention is accomplished by enzyme immunoassay or fluorescence immunoassay, an appropriately pretreated sample as described above (e.g., cells including cancer cells collected from feces or sections prepared from tissue taken from a patient) may be immobilized on a solid phase (e.g., micro-well plates or slide glasses) and provided for immunological reaction. Alternatively, a cell suspension may be reacted with the antibody in a tube. Moreover, it is also possible to use procedures in which the monoclonal antibody of the present invention is immobilized on beads or micro-well plates and then reacted with cells.

In this case, the monoclonal antibody of the present invention may be labeled with an enzyme or a fluorescent substance to thereby directly detect the reaction as fluorescence signals, or alternatively, a labeled secondary antibody which binds to the monoclonal antibody of the present invention may be used to indirectly detect signals. As a labelling substance, any substance commonly used by those skilled in the art may be used, as exemplified by peroxidase (POD), alkaline phosphatase, β-galactosidase, a biotin-avidin complex, etc. Likewise, the detection method used for this purpose may be any of competitive assay, sandwich assay or direct adsorption assay, etc. Moreover, the strength of the reaction or the ratio of cells bound to the antibody among all cells in the reacted sample may be measured and compared with the predetermined reference value or index to thereby determine or diagnose the possibility of suffering from cancer.

In the present invention, when MCT5 detection or measurement or evaluation of MCT5 expression levels is accomplished by using immunostaining, for example, the degree of staining in immunohistological staining (i.e., the detection results of MCT5) may be ranked as strongly positive (Strong), positive (Moderate), weakly positive (Weak), negative (Negative), etc., and the degree of staining is connected with the possibility of cancer to thereby detect cancer or diagnose a subject for the presence or absence of the possibility of cancer.

Moreover, under the assumption that the extracellular region of MCT5 is released into blood, the expression level of MCT5 in a blood sample may be measured to thereby detect cancer or diagnose a subject for the presence or absence of the possibility of cancer. In this case, for detection of cancer by connecting the results measured for the expression level of MCT5 with the possibility of cancer, it is desired to define a critical value (cut-off value) for the expression level of MCT5 in a biological sample.

The cut-off value for the expression level of MCT5 may be determined as follows, by way of example. First, biological samples derived from cancer patients are measured for their expression levels of MCT5. The number of patients intended here is two or more, for example, 5 or more, 10 or more, 50 or more, or 100 or more. Preferably, biological samples derived from two or more normal subjects have also been measured for their expression levels of MCT5, and the number of normal subjects intended here is two or more, for example, 5 or more, 10 or more, 50 or more, or 100 or more. Then, from all the cases including both the group of biological samples derived from cancer patients and the group of biological samples derived from normal subjects, the cut-off value for the expression level of MCT5 is determined by statistical processing. For statistical processing, 4-parameter logistic fitting may be used to prepare a standard curve. On the basis of the signal value in a MCT5-free sample, the double of this numerical value may be defined to be the cut-off value. Cases provided for statistical analysis may also be classified by the type of cancer (e.g., colorectal cancer, breast cancer, uterine cancer, gastric cancer, thyroid cancer, pancreatic cancer, leukemia), the stage of cancer, the presence or absence of recurrence, the presence or absence of metastasis, before or after surgery, etc. Furthermore, statistical processing may also be accomplished by combining, as appropriate, the measured values of MCT5 expression levels in normal subjects and the measured values of MCT5 expression levels in cancer patients when classified by the type of cancer, the stage of cancer, the presence or absence of recurrence, the presence or absence of metastasis, before or after surgery, etc.

In the present invention, the critical value (cut-off value) for the expression levels of MCT5 (extracellular region) in blood samples is, for example, about 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 21 ng/ml, 22 ng/ml, 23 ng/ml, 24 ng/ml, 25 ng/ml, 26 ng/ml, 27 ng/ml, 28 ng/ml, 29 ng/ml, 30 ng/ml, 35 ng/ml, 40 ng/ml, 45 ng/ml, 50 ng/ml, 55 ng/ml, 60 ng/ml or 65 ng/ml when expressed as a concentration in serum, with about 20 ng/ml being preferred.

When a sample is measured for its expression level of MCT5 under the above conditions, if the measured expression level of MCT5 is equal to or higher than the above cut-off value, it is possible to make a determination that cancer was detected or to make a diagnosis that the subject has the possibility of suffering from cancer. In the present invention, an upper limit may be provided for the value of serum concentration required for the above determination or diagnosis. For example, if the measured value is equal to or higher than the above cut-off value and is equal to or lower than the given upper limit value (e.g., 200 ng/ml or less, 190 ng/ml or less, 180 ng/ml or less, 170 ng/ml or less, 160 ng/ml or less, 150 ng/ml or less, 145 ng/ml or less, 140 ng/ml or less, 135 ng/ml or less, 130 ng/ml or less), it is possible to detect cancer or diagnose a subject for the presence or absence of the possibility of cancer.

In the present invention, the probability of the detection results obtained when cancer was detected is 60% or more, 70% or more, 80% or more, 90% or more, or 95% or more, and preferably 99% or more.

Further, in addition to the embodiment where the expression level of MCT5 measured in a sample from a single subject (patient) is compared with the above cut-off value to detect or diagnose the relation to cancer, biological samples derived from a plurality of patients may be used and measured for their expression levels of MCT5 in another embodiment of the present invention. Thus, in the same manner as used to determine the above cut-off value, in a population (primary population) including a given number of normal subjects and patients, their expression levels of MCT5 may be measured and processed by statistical analysis, whereby these subjects belonging to the population may be divided into a group where cancer was detected and a group where cancer was not detected. Further, the thus obtained measured values are used as master data, and a comparison may be made between these master data and the expression levels of MCT5 in samples derived from individual subjects to be detected or diagnosed in another population (secondary population).

Alternatively, the data of the respective patients may be incorporated into the values of the above population and subjected again to data processing of MCT5 expression levels to increase the number of cases of target patients (population). The increased number of cases can improve the accuracy of the critical value for the expression level of MCT5 to thereby improve the accuracy of detection or diagnosis in a single subject or a plurality of subjects.

In the present invention, a comparison may also be made between (i) the expression level of MCT5 in a biological sample from a subject and (ii) the expression level of MCT5 in a biological sample from a normal subject.

Moreover, if the expression level of MCT5 in (i) above is higher than the expression level of MCT5 in (ii) above, e.g., is about 10% or more, about 20% or more, about 30% or more, about 40% or more, about 50% or more, about 60% or more, about 70% or more, about 80% or more, about 90% or more, or about 100% or more higher than the expression level of MCT5 in a biological sample from a normal subject, it is possible to make a determination that cancer was detected or to make a diagnosis that the subject has the possibility of suffering from cancer.

The above detection results may be used, for example, as main data or supplemental data in screening of cancer or in definitive diagnosis of therapeutic effects.

Since MCT5 expression is observed in patients with various types of cancer, it cannot be identified in some cases what type of cancer a subject has even when the expression level of MCT5 is detected during medical examination, etc. For this reason, such a subject is evaluated as being "suspected to have cancer" in group health screening or the like, and this evaluation result may be used later as supplemental data to identify the type of cancer and/or determine the degree of cancer progression (for detailed examination). Moreover, when MCT5 was detected by using a sample derived from a patient who was suspected to have some certain type of cancer, such a result may be used as main data on cancer type (e.g., definitive diagnosis). It should be noted that identification of cancer type may be accomplished by using other tumor markers, diagnostic imaging, pathological diagnosis, etc.

Namely, the present invention also provides a method for aiding detection or diagnosis of cancer, which comprises the step of contacting a monoclonal antibody against MCT5 or a fragment thereof with a biological sample taken from a subject to thereby detect MCT5 in the sample.

### 5. Kit for detection or diagnosis of cancer

The anti-MCT5 antibody of the present invention may be provided in the form of a kit for detection or diagnosis of cancer. The kit of the present invention comprises the antibody and may further comprise a labelling substance, or alternatively, may comprise an immobilized reagent in which the antibody or a labeled product thereof is immobilized. The labeled product of the antibody is intended to mean the antibody labeled with an enzyme, a radioisotope, a fluorescent compound, a chemiluminescent compound or the like. In addition to the above constituent elements, the kit of the present invention may further comprise other reagents required to accomplish the detection of the present invention, as exemplified by an enzyme and a substrate (e.g., a chromogenic substrate), a substrate diluent, an enzyme reaction stop solution, or an analyte diluent and so on when the labeled product is an enzymatically labeled product. Moreover, the kit of the present invention may also comprise various buffers, sterilized water, various cell culture vessels, various reaction vessels (e.g., Eppendorf tubes), a blocking agent (e.g., bovine serum albumin (BSA), skimmed milk, goat serum or other serum components), a detergent, a surfactant, various plates, an antiseptic (e.g., sodium azide), an instruction manual for experimental operations (manufacturer's instructions) and so on. These reagents are provided, e.g., in an immobilized state, in an aqueous solution state or in a lyophilized state, and may be reconstituted into an appropriate state before use.

The kit of the present invention can be used effectively to accomplish the above detection method of the present invention and is extremely useful. The use of the kit of the present invention allows highly accurate screening of cancer patients during medical examination or the like, early diagnosis of cancer, determination of the degree of cancer progression, monitoring of therapeutic effects during treatment, and acquisition of information useful to predict metastasis and/or recurrence, so that the mortality due to cancer can be reduced.

Details on the anti-MCT5 antibody, the type of cancer, the detection method and so on are the same as described above.

### EXAMPLES

### Example 1

### (1) Identification of MCT5

New membrane proteins specific for cancer cells were identified, and new membrane protein markers were identified in an attempt to prepare antibodies for diagnostic and therapeutic purposes. From among metastatic MCF7-14 cells (BMC Cancer 2010, 10, p. 414) prepared from non-metastatic MCF7 cells, two clones (CL15 and CL6) were selected and analyzed for their expression profile with a DNA microarray, whereby membrane proteins whose gene expression was enhanced two-fold or more in CL15 and CL6 cells as compared to MCF7 cells were narrowed down to 19 candidates. These candidates were further narrowed down with a focus on genes whose expression was enhanced 5-fold or more in both CL15 and CL6 cells and which had been found to be localized in the cell membrane, thereby obtaining three candidates. Among them, MCT5 was selected for analysis.

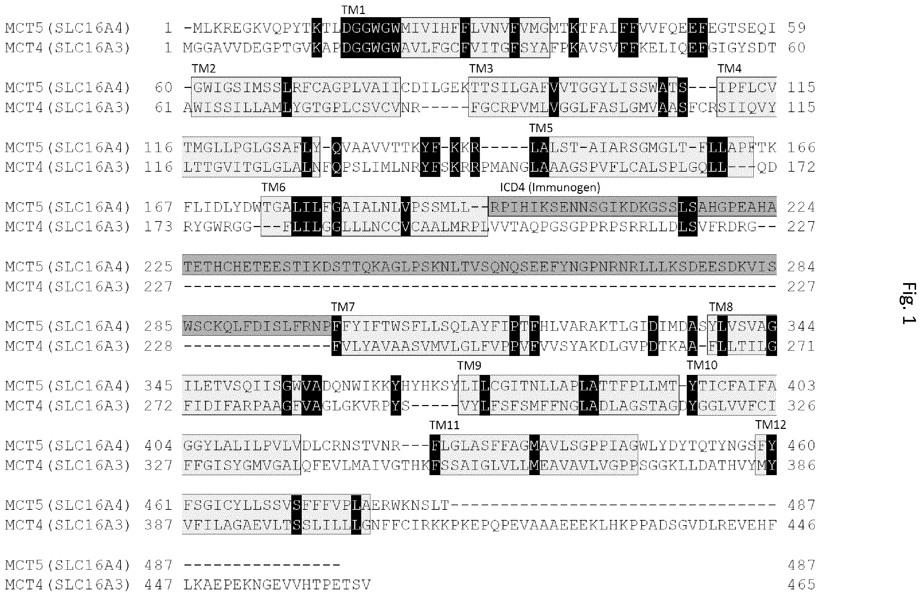

Figure 1 shows the alignment results analyzed for amino acid sequence homology between MCT5 and MCT4. MCT5 and MCT4 were found to be poorly homologous to each other because of sharing an identity of 15.5% and a similarity of 55.5%, and were therefore considered to be functionally distinct molecules.

### (2) Cells

MCF7-14 CL6 and CL15 were established from Accession No. FERM BP-10944. MCF7 and MCF7-14 were cultured and subcultured in RPMI 1640 medium (Sigma) containing 10% (v/v) serum (Hyclone) at 37°C under 5% CO₂ for 48 to 72 hours, such that the confluency did not exceed 80, while other cells were cultured and subcultured in DMEM medium (Sigma) at 37°C under 5% CO₂ for 48 to 72 hours, such that the confluency did not exceed 80%.

### (3) Cloning of MCT5 gene

MCF7-14 CL6 was cultured and the total RNA was extracted with a Qiagen RNeasy Mini kit. From the extracted total RNA (2 µg), cDNA was synthesized by reverse transcription (RT) reaction at 50°C for 1 hour with SuperScript III reverse transcriptase (Invitrogen), followed by heating at 85°C for 5 minutes to stop the reaction. The resulting cDNA was used as a template for PCR reaction with the following primers.

### Primer sequences

S16A4-F1: 5'-AAAGGTACCATGCTGAAGAGGGAGGGGAAGG-3' (SEQ ID NO: 32)
S16A4-R1: 5'-AAATCTAGAGGTCAGACTGTTTTTCCATCTTTCG-3' (SEQ ID NO: 33)

The PCR reaction was conducted by preincubation at 95°C for 10 minutes and subsequent 35 cycles of denaturation at 95°C for 15 seconds and annealing/elongation at 56°C for 1 minute to thereby amplify the desired gene fragment.

The resulting amplified fragment was integrated into pEF6-myc/HisA vector (Invitrogen) with restriction enzymes (*Kpn*I and *Xba*I) located on the primers. The amplified fragment was confirmed by DNA sequencing, and the same gene sequence as found in the database was confirmed to be integrated therein. Hereinafter, this prepared vector is referred to as pEF6-MCT5-myc/HisA.

### (4) Confirmation of MCT5 expression (real-time PCR)

For confirmation of MCT5 gene expression, the amount of mRNA transcription was determined by real-time PCR. In the same manner as shown in (2) above, total RNA was extracted and PCR reaction was conducted with the following primers and THUNDERBIRD SYBR qPCR Mix (TOYOBO) to detect the amount of amplified fragment in a real-time manner by the SYBR Green method. As an internal standard, the human *GAPDH* gene was also amplified and used for correction. The PCR reaction was conducted by preincubation at 95°C for 10 seconds and subsequent 40 cycles of denaturation at 95°C for 5 seconds and annealing/elongation at 60°C for 40 seconds.

hSLC16a4 Real-time F1 : 5'-CTGTAGCAGGTATCCTTGAGACG-3' (SEQ ID NO: 34)
hSLC16a4 Real-time R1: 5'-GGATGAGGTAAGACTTGTGGTAATG-3' (SEQ ID NO: 35)
hGapdh real-time F: 5'-TGCACCACCAACTGCTTAGC-3' (SEQ ID NO: 36)
hGapdh real-time R: 5'-GGCATGGACTGTGGTCATGA-3' (SEQ ID NO: 37)

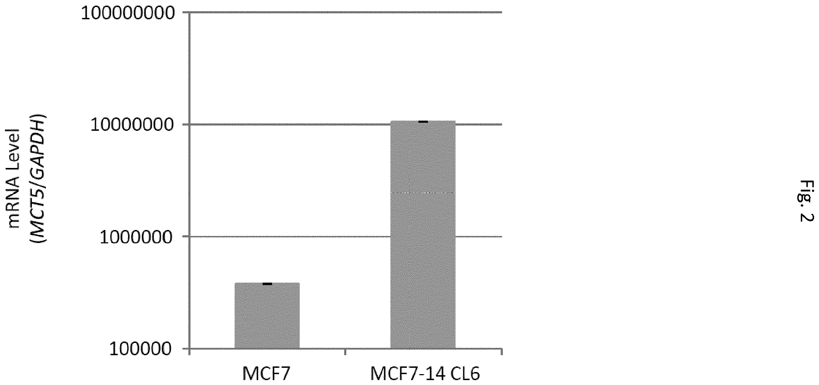

The expression level of the MCT5 gene was compared between MCF7 and MCF7-14 CL6, indicating that the expression was clearly enhanced (Figure 2).

### (5) Transient expression of MCT5

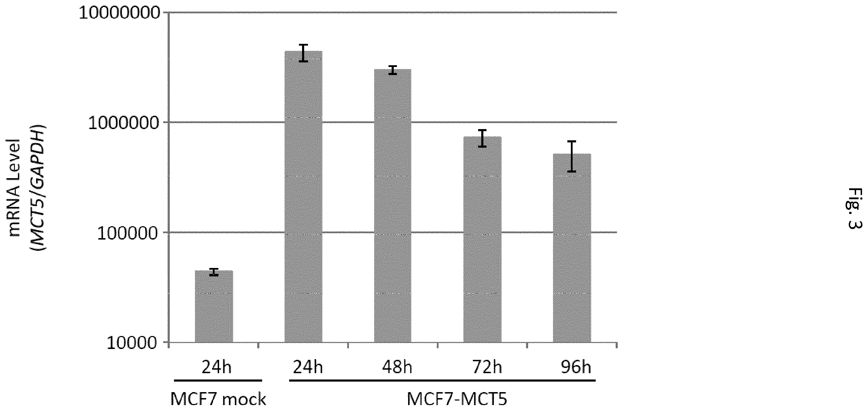

The plasmid prepared in (1) above was introduced into MCF7 cells using FUGENE6 (Roche Applied Science). Operations were conducted in accordance with the information attached to the kit. After gene transfer, aliquots were sampled at 24, 48, 72 and 96 hours and analyzed for expression levels according to the procedures shown in (3) above. The results obtained are shown in Figure 3. The results indicated that the mRNA of MCT5 was detected at 24 hours after gene transfer, and the expression was maintained even after 96 hours.

### (6) Matrigel invasive capacity test

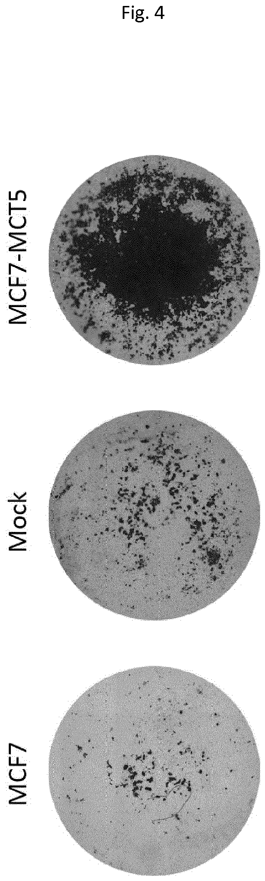

The cells prepared in (5) above, MCF7 cells, and pcDNA3.1-carrying MCF7 cells (Mock) were each suspended in a serum-free medium and then introduced (1 × 10⁵ cells each) into a transwell (the upper side of a filter: upper compartment) in a Matrigel invasion chamber (BD), followed by culture at 37°C for 3 days. Cells migrating through the membrane (pore size: 8 µm) in the transwell (the lower side of the filter: lower compartment) were stained with a 0.1% crystal violet solution and rinsed with MilliQ to remove the excess dye, and cell counts were then observed. The results obtained are shown in Figure 4. Upon introduction of MCT5, the number of cells permeating Matrigel was clearly increased. This indicated that MCT5 clearly increased the invasive capacity of MCF7 cells.

### Example 2

### (1) Preparation of cell lines stably expressing MCT5

The pEF6-MCT5-mycHisA vector prepared in Example 1 (1) was cleaved with a restriction enzyme (PmeI) to thereby cut off a region downstream of the stop codon for MCT5. On the other hand, pMXs-IG vector (purchased under license from Professor Toshio Kitamura, the University of Tokyo) was cleaved with restriction enzymes (XhoI/SalI) to excise a gene sequence encoding IRES-GFP. This gene fragment was blunt-ended with Klenow fragment (Takara) and ligated to the restriction enzyme-treated pEF6-MCT5-mycHisA vector. Hereinafter, this vector is referred to as pEF6-MCT5-IG.

The above two vectors (i.e., pEF6-MCT5-IG and pEF6-IG) were provided for gene transfer into MCF7 cells in the same manner as shown in Example 1 (5) and cultured in the same medium as shown in Example 1 (2) supplemented with blasticidin S at 25 µg/mL to establish several lines of cells showing drug resistance. From among them, cells in which EGFP fluorescence was observed under a fluorescence microscope were selected to thereby establish two cell lines MCF7-MCT5-2 and MCF7-MCT5-7 as cells carrying the pEF6-SLC16A4-myc-IG vector and three cell lines MCF7-Mock1, MCF7-Mock2 and MCF7-Mock3 as cells carrying the pEF6-IG vector.

### (2) Confirmation of MCT5 expression (real-time PCR)

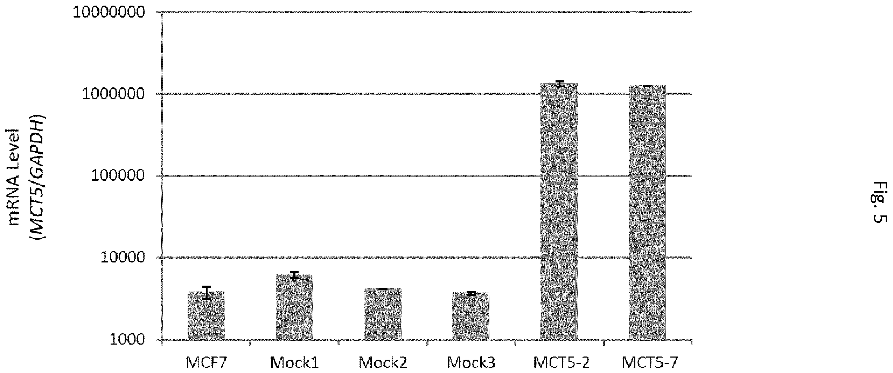

MCT5 expression was analyzed in the same manner as shown in Example 1 (4) above. The results obtained are shown in Figure 5. The results indicated that the mRNA of MCT5 was increased 100-fold or more in MCF7-MCTS-2 and MCF7-MCT5-7 cells, when compared to MCF7 and MCF7-Mock1 to MCF7-Mock3.

### (3) Matrigel invasive capacity test

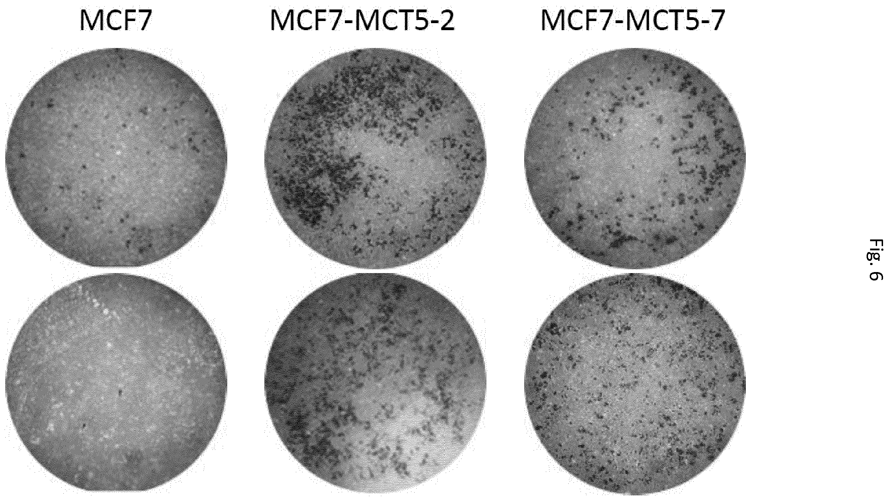

MCF7-MCT5-2 and MCF7-MCT5-7 were analyzed for changes in their invasive capacity in comparison with MCF7. The results obtained are shown in Figure 6. The results indicated that MCF7-MCT5-2 and MCF7-MCT5-7 showed clearly enhanced invasive capacity when compared to MCF7.

### Example 3

### (1) Preparation of anti-MCT5 antibodies

### (1) Cell culture

Mouse myeloma cell line P3X63-Ag8 (ATCC Accession No. CRL-1580), human colorectal cancer cell line HCT-116 (ATCC Accession No. CCL-247) and human fetal kidney 293T cells were each cultured and subcultured in RPMI 1640 medium (Sigma) containing 10% (v/v) serum (Hyclone) at 37°C under 5% CO₂ for 48 to 72 hours, such that the confluency did not exceed 80%.

### (2) Cloning of MCT5 intracellular domain 4 (ICD4) gene

The plasmid prepared in Example 1 (3) was used as a template for PCR reaction with the following primers and with KOD PLUS (TOYOBO) to thereby amplify DNA comprising a nucleotide sequence (SEQ ID NO: 40) encoding amino acids at positions 196 to 299 of MCT5. Figure 7 shows a schematic view of positions on the amino acid sequence which are matched between MCT5 and ICD4.

### Primer sequences

hSLC16A4 ICD4 F1: 5'-GCGCGGATCCGAGACCCATCCATATCAAAAGTG-3' (SEQ ID NO: 38)
hSLC16A4 ICD4 R1: 5'-GCGCGCGTCGACAGGATTTCTAAAGAGAGAAATGTC-3' (SEQ ID NO: 39)
ICD4 DNA sequence (SEQ ID NO: 40) 
AGACCCATCCATATCAAAAGTGAGAACAATTCTGGTATTAAAGATAAAGGCA
GCAGTTTGTCTGCACATGGTCCAGAGGCACATGCAACAGAAACACACTGCCA
TGAGACAGAAGAGTCTACCATCAAGGACAGTACTACGCAGAAGGCTGGACT
ACCTAGCAAAAATTTAACAGTCTCACAAAATCAAAGTGAAGAGTTCTACAAT
GGGCCTAACAGGAACAGACTGTTATTAAAGAGTGATGAAGAAAGTGATAAG
GTTATTTCGTGGAGCTGCAAACAACTGTTTGACATTTCTCTCTTTAGAAATCC
T
ICD4 amino acid sequence (SEQ ID NO: 7)

RPIHIKSENNSGIKDKGSSLSAHGPEAHATETHCHETEESTIKDSTTQKAGLPSKN
LTVSQNQSEEFYNGPNRNRLLLKSDEESDKVISWSCKQLFDISLFRNP

The PCR reaction was conducted by preincubation at 95°C for 10 minutes and subsequent 40 cycles of denaturation at 95°C for 15 seconds and annealing/elongation at 58°C for 1 minute to thereby amplify the desired gene fragment.

The resulting amplified fragment was cleaved with restriction enzymes (*Bam*HI and *Sal*I) at the restriction enzyme sites located on the primers and then integrated into pET32b vector (Invitrogen) to thereby obtain DNA comprising a nucleotide sequence encoding a partial protein of MCT5 having Trx-, S- and His-tags on the N-terminal side and a His-tag on the C-terminal side.

*E. coli* Rosseta gami (DE3) pLysS (Novagen) was transformed with this vector and cultured in LB medium (1% (w/v) tryptone (Sigma), 0.5% (w/v) yeast extract (Sigma), 0.5% (w/v) NaCl (Sigma)) supplemented with 1% (w/v) glucose. After the medium turbidity reached 0.6 at a wavelength of 600 nm, 1 mM IPTG (WAKO) was added and culture was continued for 16 hours. The microbial cells were collected by centrifugation and then homogenized by ultrasonication to obtain a fraction containing the extracellular region of SLC6A6(the intracellular region of MCT5?) as an insoluble protein.

About 10 mg of the sample was dissolved in Buffer A (1 M guanidine hydrochloride (Sigma), 10 mM DTT (Sigma), 10 mM EDTA (Sigma)) and reacted at 37°C for 1 hour. The reaction mixture was added gently to 1 L of Buffer B (50 mM Tris, 150 mM NaCl, 5% glycerol, 0.4 mM oxidized glutathione (Sigma), pH 8.5) and stirred at 4°C for 18 hours. The dissolved sample was applied to a Ni-sepharose column (GE) and eluted with Buffer C (50 mM potassium phosphate buffer, 150 mM NaCl, 200 mM imidazole, pH 8.0), followed by dialysis against imidazole-free Buffer C to obtain a partial protein of the intracellular region of MCT5 (ICD4) in purified form.

### (3) Immunization

ICD4 prepared above was mixed with an equal amount of Freund's complete adjuvant and intraperitoneally administered to BALB/c mice in a volume of 100 µl (about 40 µg/mouse). After about 3 weeks, ICD4 was also mixed with an equal amount of Freund's incomplete adjuvant and intraperitoneally administered to the mice. This operation was repeated 7 to 12 times, followed by confirmation of an increase in antibody titers. The mice showing increased titers were intravenously administered with ICD4 (about 40 µg) as the final immunization and, after 3 days, their spleens were excised.

### (4) Cell fusion

Mouse spleen lymphocytes were electrically fused with mouse myeloma cell line P3X63-Ag8. For cell fusion, 1 × 10⁸ spleen cells were mixed with 0.25 × 10⁸ cells of the myeloma cell line and suspended again in EP Buffer (0.3 M mannitol, 0.1 mM CaCl₂, 0.1 mM MgCl₂) at a cell density of 0.25 × 10⁸ cells/mL, followed by cell fusion with an electro cell fusion generator LF201 (Nepa Gene Co., Ltd., Japan). Cell fusion conditions were set in accordance with the manufacturer's recommended protocols.

The fused cells were suspended in HAT medium (Invitrogen) at 1.6 × 10⁵ cells/mL and dispensed into 96-well plates in a volume of 200 µL per well. During culture, 100 µL of HT medium was added to each well. After culture for 11 to 16 days, the plates were observed under a microscope, indicating that 2 to 12 colonies were formed per well.

### (5) ELISA

Culture supernatants were collected from wells in which hybridomas were growing, and provided for screening to select hybridomas producing monoclonal antibodies reactive to ICD4. ICD4 was diluted with PBS(-) and dispensed into 96-well ELISA plates (Nunc) in an amount of 50 ng per well, and immobilized on the plate surface by being allowed to stand overnight at 4°C. Then, after the plates were washed three times with 350 µL of PBS(-) containing 0.05% Tween 20 (hereinafter abbreviated as PBS-T), PBS-T containing 1% skimmed milk was dispensed in a volume of 300 µL per well, followed by blocking for 1 hour at room temperature. After washing with PBS-T, the culture supernatants of hybridomas were dispensed into the ICD4-immobilized ELISA plates and reacted for 1 hour at room temperature. After washing with PBS-T, a 10000-fold dilution of peroxidase (hereinafter abbreviated as POD)-labeled anti-mouse immunoglobulin antibody (BETHYL) was dispensed in a volume of 100 µL per well and reacted for 1 hour at room temperature. After washing in the same manner, a OPD(POD?) substrate prepared at 0.5 mg/mL POD was added to cause color development at room temperature for 20 minutes. After the reaction was stopped with 1.5 N sulfuric acid, the plates were measured for absorbance at 490 nm with a plate reader (Molecular Devices).

### (6) Obtaining of monoclonal antibodies

From among antibodies produced from the hybridoma cells formed in (5) above, antibodies reactive to ICD4 were selected in the same manner as shown in (5) above. Moreover, to remove antibodies reactive to the Trx-, S- and His-tags added during preparation of ICD4, pET32b vector alone was introduced into *E. coli* and the same procedure as shown in (2) above was repeated to express and purify a protein for use as a negative control. Hereinafter, this protein for use as a negative control is referred to as Tag.

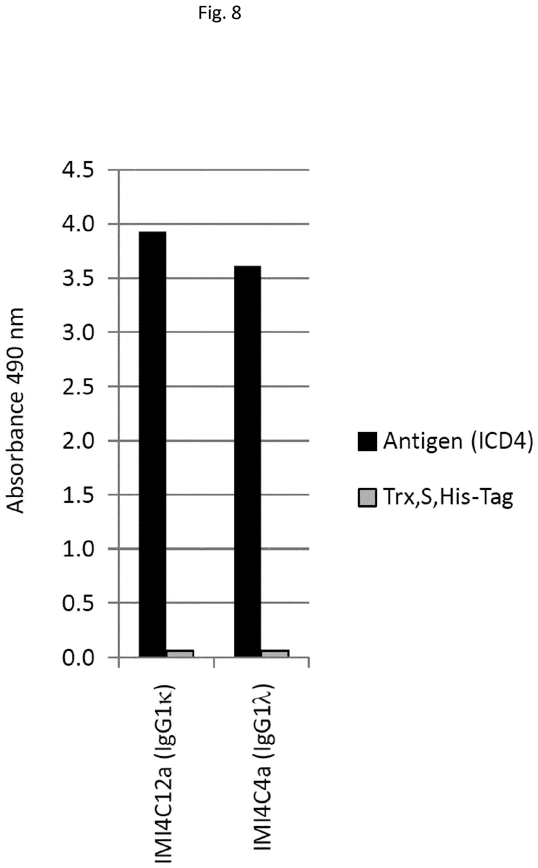

As a result of selection of hybridoma cells producing monoclonal antibodies which were reactive to ICD4 and not reactive to Tag, 14 clones were obtained. From among them, two clones showing particularly high titers were selected and provided for use in the subsequent experiment. Hereinafter, these clones are referred to as "IMI4C4a" and "IMI4C12a," respectively. Hereinafter, antibodies produced from hybridoma cells may also be expressed as their clone numbers. As to the antibodies produced from the hybridoma cells "IMI4C4a" and "IMI4C12a," Figure 8 shows the results of ELISA using their culture supernatants. The results indicated that these two antibodies were both reactive to ICD4 but not reactive to Tag.

After being cloned singly by limiting dilution, these hybridoma cells were each cultured in ten 10 cm dishes to reach 90% confluency and cultured for 10 days in a 1:1 mixed medium of HT medium (Invitrogen) and EX CELL Sp2/0 (Nichirei Bioscience Inc., Japan). The culture supernatants were collected and purified with a Protein G column. The Protein G column (GE Healthcare) was used in a volume of 3.5 mL relative to 100 mL culture supernatant. The cultured solutions were each passed at a flow rate of 1 to 3 ml/min through the Protein G column which had been equilibrated with PBS, followed by washing with 6 mL of washing buffer (25 mM Tris-HCl (pH 7.4), 140 mM NaCl, 10 mM KCl). Then, antibody proteins were eluted with 6 mL of elution buffer (0.1 M glycine (pH 2.5)) and neutralized with 3 M Tris-HCl (pH 7.4) to be within pH 7.0 to 7.4. The antibodies were concentrated with Amicon Ultra 30 (Millipore) and the buffer was replaced with PBS.

In addition, the antibodies produced from the hybridoma cells IMI4C4a and IMI4C12a were analyzed for their isotype. For analysis, an Iso Strip mouse monoclonal antibody isotyping kit (Roche) was used. As a result, the IMI4C4a antibody was found to be subclass IgG1λ, while the IMI4C12a antibody was found to be subclass IgG1κ.

### Example 4 Reactivity between anti-MCT5 monoclonal antibodies and MCT

### (1) ELISA

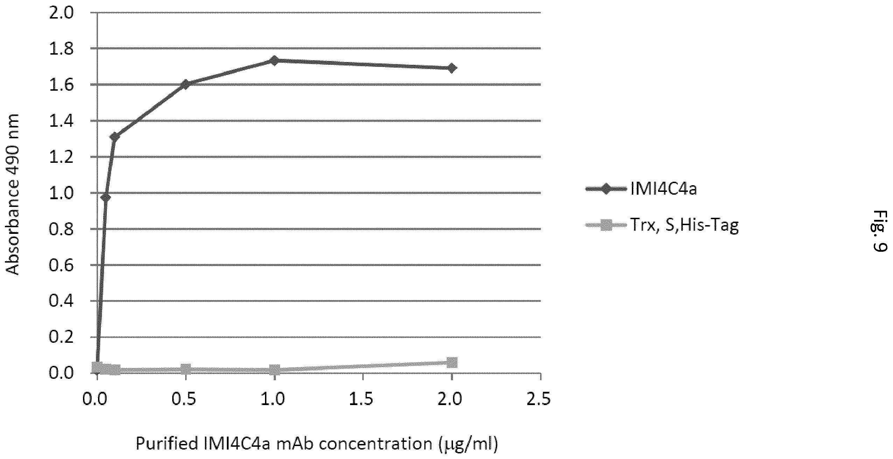

ELISA analysis was performed on the antibody produced from "IMI4C4a" among the two hybridoma cells obtained in Example 3 (6). The same procedure as shown in Example 3 (5) was used and the antibody was diluted to concentrations of 0.125, 0.25, 0.5, 1.0 and 2.0 µg/mL for analysis. The results obtained are shown in Figure 9. The results indicated that IMI4C4a was not reactive to Tag, but reactive to ICD4, and binding signals were elevated in a concentration-dependent manner.

### (2) Immunocytological staining

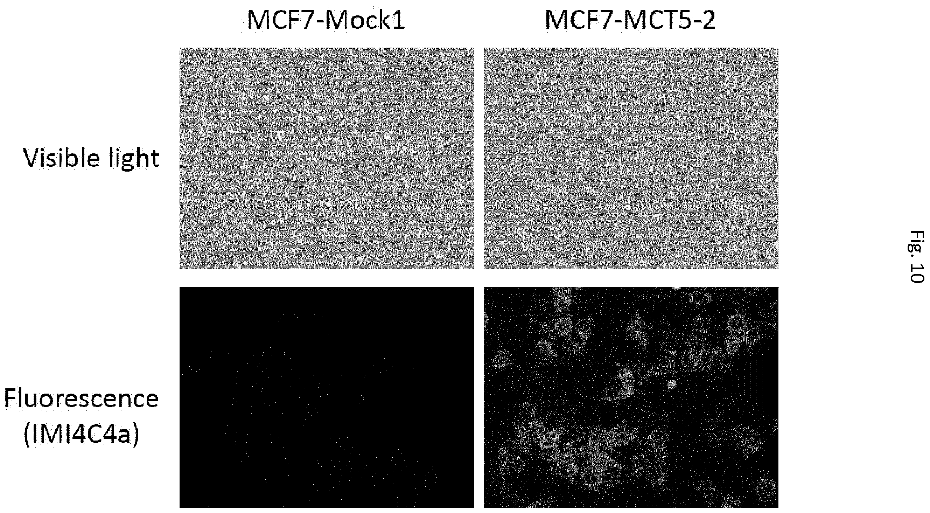

IMI4C4a was analyzed for its reactivity to the breast cancer cells (MCF7-Mock1 and MCF7-MCT5-2) shown in Example 2 (1). MCF7-Mock1 and MCF7-MCT5-2 were cultured to reach 80% confluency and then seeded onto cover slips coated with Cellmatrix Type I-A (Nitta Gelatin Inc., Japan). After culture for 2 days, the cells were fixed with 10% neutral buffered formalin (WAKO). After being treated with 0.3% (v/v) aqueous hydrogen peroxide for 20 minutes, the cover slips were washed three times with TBS-T (25 mM Tris, 150 mM NaCl, 0.05% (v/v) Tween 20) and treated with TBS-T containing 5% (w/v) skimmed milk, followed by addition of the antibody purified in Example 3 (6) above at a concentration of 10 µg/mL and reaction at 4°C for 16 hours. After washing three times with TBS-T, anti-mouse IgG polyclonal antibody-Alexa Fluor 488 label or anti-mouse IgM polyclonal antibody-Alexa Fluor 488 label was reacted as a secondary antibody at room temperature for 30 minutes. After washing three times with TBS-T, the cover slips were sealed with Mounting Medium (Vector Shield). Figure 10 shows the results analyzed for fluorescence intensity under the same conditions.

In the case of using IMI4C4a, strong signals were observed only in MCF7-MCT5-2, thus suggesting that the cell membrane and cytoplasm were stained. Namely, IMI4C4a was confirmed to bind to MCT5 present on the cell surface and in the cytoplasm. MCT5 is a membrane protein with 12 transmembrane domains and is considered to be present on the cell membrane. In view of this fact, signals observed in the cytoplasm are considered to detect MCT5 molecules in the course of transport during which MCT5 translated in ribosomes is transported via the Golgi apparatus and transport vesicles onto the cell membrane.

### (3) Western blotting

The antibody produced from the hybridoma cells "IMIC4a" was analyzed by Western blotting. The MCF7-Mock1 and MCF7-MCT5-2 cells prepared in Example 2 (1) were cultured in 10 cm dishes to reach 90% confluency and washed twice with PBS(-). After addition of 2 × RIPA Buffer (0.1 M Tris, 0.3 M NaCl, 1% (v/v) Triton X-100, 2% (w/v) sodium deoxycholate, 0.2% (w/v) sodium dodecyl sulfate) in a volume of 200 µL, the cells were allowed to stand on ice for 1 minute and then detached with a scraper to collect a cell suspension. The cells were homogenized for 30 seconds in an ultrasonic homogenizer (Branson) to obtain an extract. Extracts were prepared for the respective cell lines and measured for their protein concentration by the Bradford assay, and then adjusted to the same protein amount and provided for SDS-PAGE.

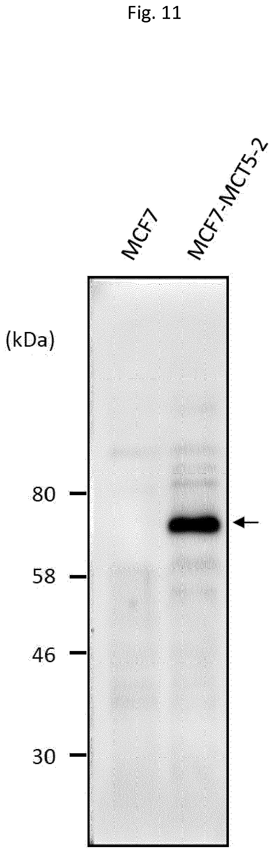

After electrophoresis, proteins were transferred onto a PVDF membrane (PIERCE) with a Trans-Blot SD cell (BioRad Laboratories) in accordance with the manufacturer's recommended protocols. The membrane was blocked at room temperature for 30 minutes with skimmed milk dissolved at a concentration of 5% (w/v) in TBS-T, and then washed three times with TBS-T. The antibody produced from the hybridoma cells IMI4C4a was diluted to 1 µg/mL with TBS-T and reacted with the membrane for 1 hour at room temperature. Likewise, as a negative control, an antibody-free medium was reacted in the same manner. After washing three times with TBS-T, anti-mouse IgG polyclonal antibody-HRP label (BETHYL) was diluted 10,000-fold with TBS-T for use as a secondary antibody. The membrane was reacted with this dilution at room temperature for 30 minutes and then washed three times with TBS-T. The membrane was soaked in Immobilon (Millipore) and then wrapped, followed by signal detection with LAS-3000 (Fuji Photo Film Co., Ltd., Japan). The experimental results obtained are shown in Figure 11. The results indicated that the antibody produced from the hybridoma cells IMI4C4a was also reactive to denatured MCT5. Moreover, it was also indicated that the protein level of MCT5 was increased in MCF7-MCT5-2 cells when compared to MCF7.

### Example 5 Analysis of clinical tissue samples

### (1) Immunohistological staining using breast cancer tissues

The rate of MCT5 expression in breast cancer was analyzed by analysis of the reactivity between IMI4C4a and breast cancer tissue. In this example, human cancer tissue array slides (FDA808b; US Biomax) were used for analysis.

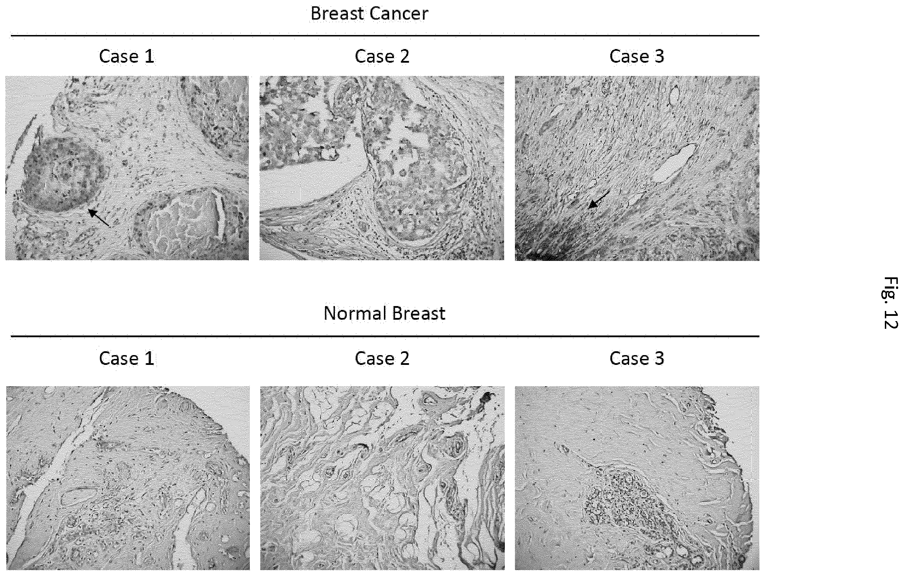

The tissue array slides were deparaffinized by being soaked three times in xylene for 5 minutes. The slides were then soaked twice in 100% ethanol for 5 minutes and further soaked sequentially in 90% ethanol and 80% ethanol for 5 minutes each, and then soaked under running water for 2 minutes to hydrate the samples. The slides were soaked in 10 mM citrate buffer (pH 6.0) and microwaved at 600 W for 5 minutes. This operation was repeated three times in total to activate the antigen. The slides were treated for 10 minutes with 3% aqueous hydrogen peroxide diluted in methanol to thereby deactivate endogenous peroxidase activity. For the subsequent detection, a Histofine (M) kit (Nichirei Corporation, Japan) was used and blocking, secondary antibody reaction, washing and detection reaction were conducted in accordance with the protocols attached to the kit. For primary antibody reaction, IMI4C4a was diluted to 5 µg/mL with TBS-T and reacted at room temperature for 1 hour. For color development, Impact DAB (Vector Laboratories) was used and reacted at room temperature for 5 minutes. The reacted slides were washed under running water and then counter stained with hematoxylin (Merck) and then sealed. The results of tissue staining are shown in Figure 12.

Upon immunohistological staining with the IMI4C4a antibody, Case 1 and Case 3 of breast cancer tissue were found to be stained. Moreover, a region stained with the IMI4C4a antibody was a part of cancer tissue. For example, in Case 1 of breast cancer tissue, among the three cancer sites observed, only one site (indicated with the arrow) was stained. Likewise, in Case 3, there were very strongly stained regions (indicated with the arrow at the lower left) and non-stained regions in the same cancer site. Case 2 showed no staining. In contrast, in the case of normal mammary tissue, Cases 1 to 3 showed no staining.

According to the clinical information attached to the tissues, Case 1 (Position Number D4) is breast cancer at Stage 0 with early invasion, while Case 3 (Position Number D6) is breast cancer with invasion having reached the breast duct. Thus, in the case of using the IMI4C4a antibody, it was shown that there were tissues showing enhanced expression at very early invasion sites of breast cancer at Stage 0.

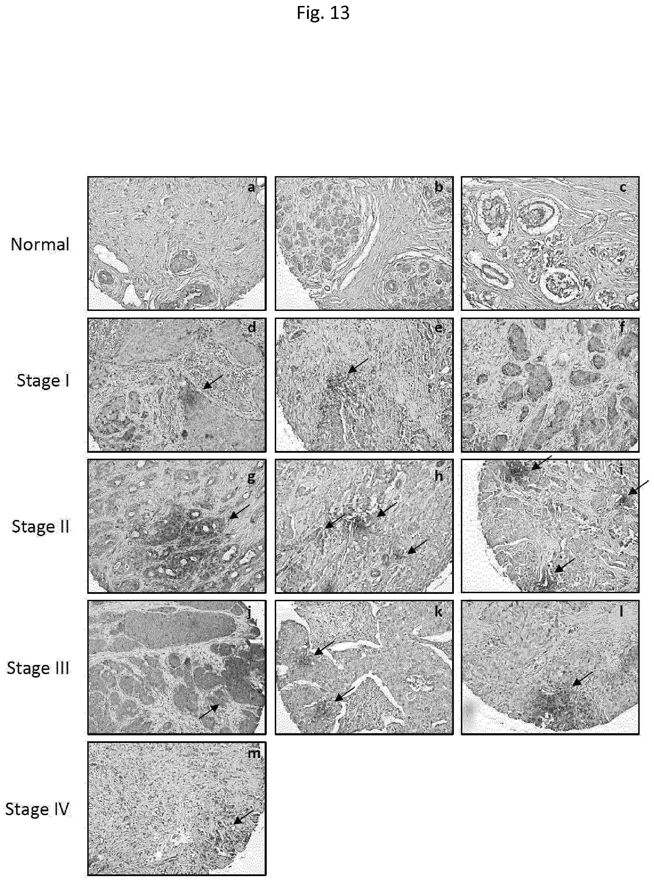

Further, using human breast cancer tissue array slides (BC081120; US Biomax), immunohistological staining was performed on 100 cases of human breast cancer tissue and 10 cases of normal tissue. The results summarized at different stages of cancer progression are shown in Figure 13. The normal tissue cases (a to c) showed no staining and the cancer tissue cases at Stages I to IV (d to e and g to m) were found to be partially stained (indicated with arrows). Figure 13f shows that cancer sites were stained.

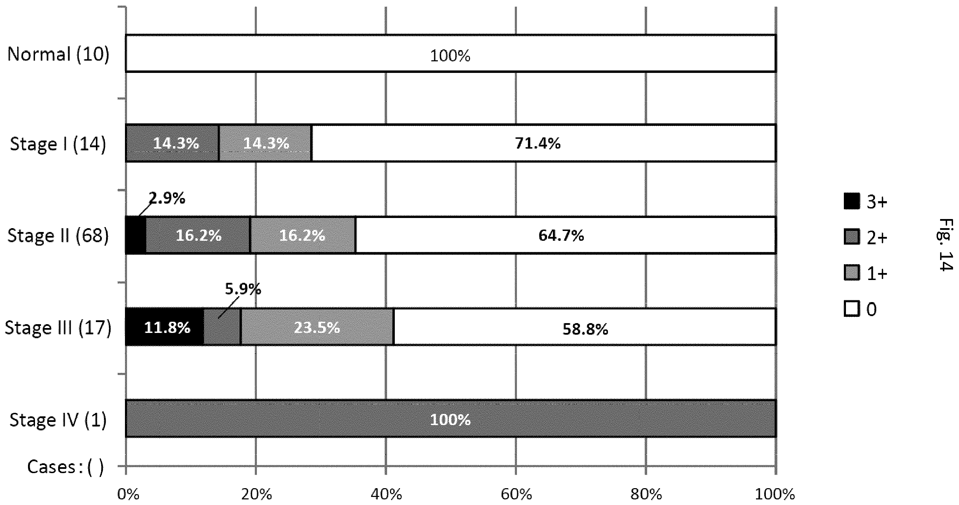

The degree of staining in immunohistological staining was ranked as strongly positive (3+), positive (2+), weakly positive (1+) or negative (0), and the results of immunohistological staining with IMI4C4a were provided for statistical analysis. The results obtained are shown in Figure 14. In the case of normal mammary tissue, none of the 10 cases used for measurement was stained. In contrast, in the case of breast cancer tissue, some tissues were stained strongly positive even at Stage I, and the positive rate tended to increase at more advanced stages of cancer. Among the analyzed 100 cases of breast cancer tissue, 36 cases were found to be positive.

Tissue stained with the IMI4C4a antibody is a part of cancer tissue. In light of the experimental results obtained in Example 1 (6) and Example 2 (3), the enhanced expression of MCT5 is predicted to be involved in invasion of cancer cells; and hence a region stained with the IMI4C4a antibody in breast cancer tissue is highly probable to be a region in the cancer tissue where cells having high invasive capacity are particularly present. The presence of highly invasive cells in cancer tissue has also been expected from previous studies.

Cancer will develop when some malfunction occurs in the proliferation program of normal cells, and it is pointed out that the root cause of difficulties in cancer treatment lies in that cancer causes morphological changes during the course of development or progression (Nature 2013, 501, p. 328-337). A tumor mass at the time of tumor discovery has already become an aggregate of cells with different properties, and such non-uniformity in cancer tissue is called heterogeneity. Namely, it is known that highly invasive cancer regions and less invasive cancer regions are both present in the same cancer tissue.

MCT5 increases the invasive capacity when overexpressed in MCF7 cells which are cultured human breast cancer cells. Namely, a region stained with the IMI4C4a antibody in breast cancer tissue is considered to be a breast cancer tissue having high invasive capacity; and hence cancer tissue having high invasive capacity can be expected to be distinguished when using the antibody of the present invention.

### (2) Triple negative breast cancer tissues

Then, tissue samples derived from the same patients as in the cases used in (1) above were used and analyzed for the expression of estrogen receptors, progesterone receptors and Her2, which are commonly used as breast cancer markers. For detection of estrogen receptors and progesterone receptors, an ER/PgR (MONO) universal kit (Nichirei Corporation, Japan) was used. Likewise, for detection of Her2, Herceptest II (DAKO) was used. Experiments were conducted in accordance with the experimental protocols attached to the respective kits.

In breast cancer, cancer tissue classified as being estrogen receptor-negative, progesterone receptor-negative and Her2-negative does not require these receptors for cancer cell proliferation, and therefore cannot be treated by hormone therapy or by receptor-mediated anticancer therapy, so that there arises a problem in that there is no therapeutic choice but chemotherapy. Moreover, breast cancer of this type is often seen in young patients and is also pointed out to be highly malignant breast cancer.

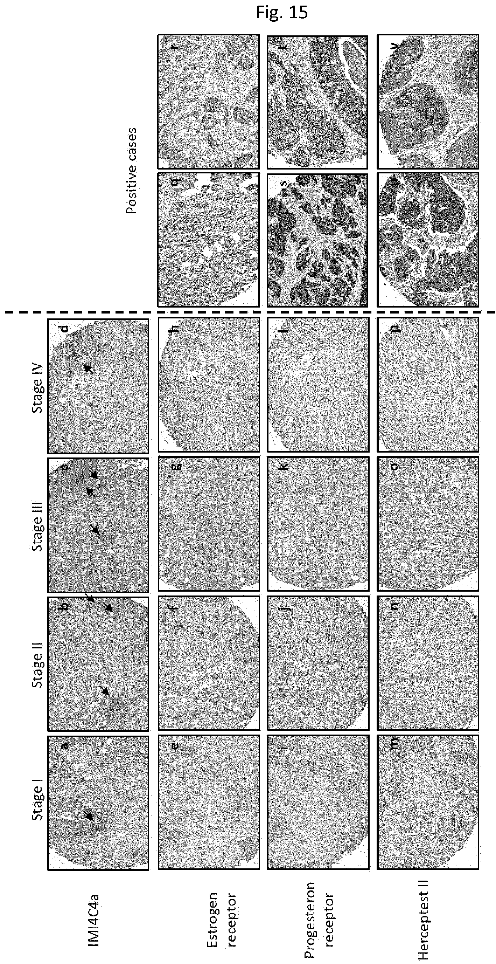

Triple negative breast cancer cases were analyzed with the IMI4C4a antibody, indicating that a part of tissue was stained in 24 cases (57%) of 42 cases. Figure 15 shows some of the results of immunohistological staining. The panels a to d of Figure 15 show the results of staining with IMI4C4a, indicating that clear staining was observed in a part of cancer tissue, as indicated with arrows. On the other hand, the panels e to h of Figure 15 show the results of staining for estrogen receptors, the panels i to 1 of Figure 15 show the results of staining for progesterone receptors, and the panels m to p of Figure 15 show the results of staining for Her2. These results indicate that no signals are observed and these cases can be confirmed to be triple negative. In addition, tissue samples positive to estrogen receptors, progesterone receptors or Her2 are shown in the panels q and r of Figure 15, the panels s and t of Figure 15, or the panels u and v of Figure 15, respectively.

Namely, it is indicated that the IMI4C4a antibody can also be used in highly malignant breast cancer cases which have been undetectable by conventional methods.

### (3) Immunohistological staining using colorectal tissues

The rate of MCT5 expression in cancer was analyzed by analysis of the reactivity between IMI4C4a and colorectal cancer tissue or lung cancer tissue. For analysis, human tissue array slides (FDA808b; USBiomax) were used.

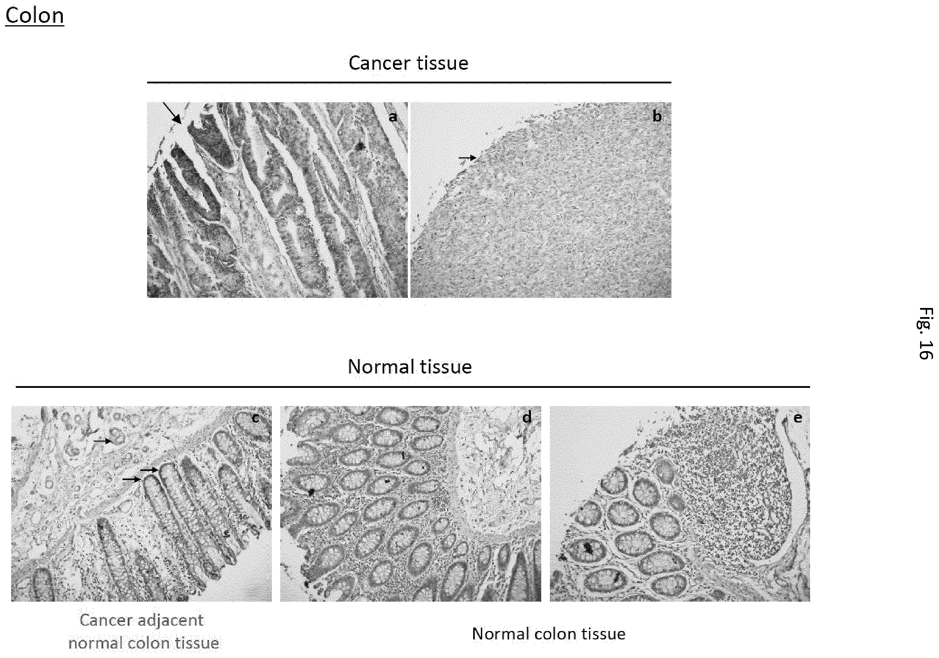

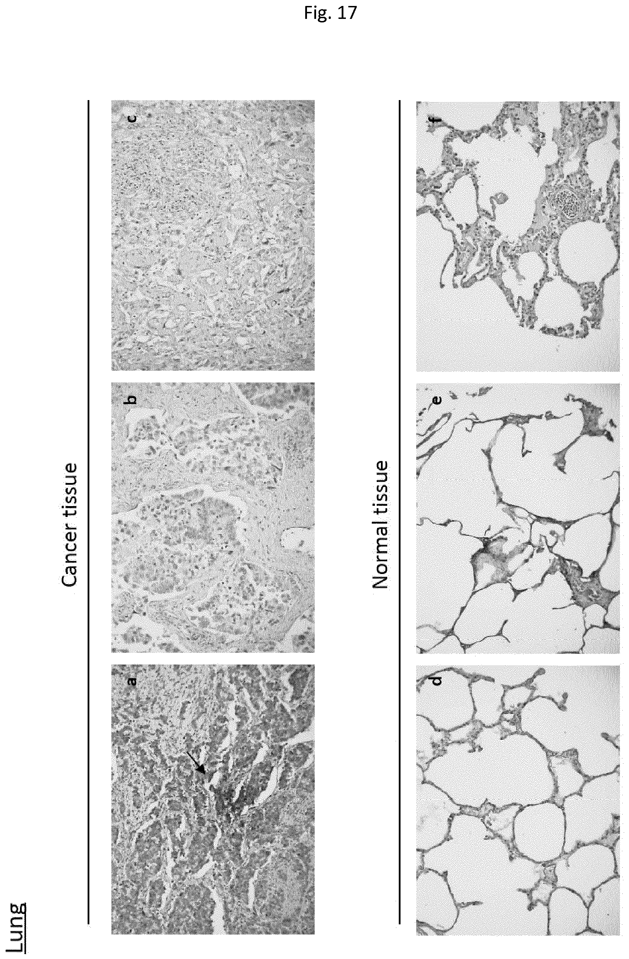

Immunostaining was conducted in the same manner as shown in Example 5 (1) above. The results of staining in colorectal cancer and normal colorectal mucosal tissue are shown in Figure 16, while the results of staining in lung cancer and normal lung tissue are shown in Figure 17.

In the case of colorectal tissue, there were some colorectal cancer cases where a part of cancer was clearly stained (Figure 16, panel a). Likewise, in normal tissue, some cancer patients showed staining around colorectal mucosal crypts in their cancer adjacent tissue (Cancer adjacent normal colon tissue) (Figure 16, panel c). On the other hand, no staining was observed in normal colorectal tissue (Figure 15(16?), panels d and e).

Cancer adjacent tissue in a cancer patient is used as a control in comparison with a cancer site. However, such cancer adjacent tissue already has genetic mutations and is known to show a different expression profile from that of normal tissue. For example, there is a report showing the results obtained when normal colorectal tissue, colorectal cancer adjacent tissue and colorectal cancer tissue were analyzed for their mRNA expression profile with a high-throughput sequencer (PLoS ONE 2012, 7, e41001). This report points out the presence of a factor (tumor suppressor gene *TGFBR2)* which varies in both colorectal cancer tissue and cancer adjacent tissue in comparison with normal colorectal tissue. Moreover, as in the case of breast cancer, colorectal cancer is also pointed out to contain a plurality of cells showing different gene expression patterns in the same cancer tissue (heterogeneity).

### (4) Immunohistological staining using lung tissues

In the case of lung tissue, there were some lung cancer cases where a part of cancer was clearly stained (Figure 17, panel a). On the other hand, no staining was observed in normal tissue (Figure 17, panels d to f).

### (5) Other cancer tissues

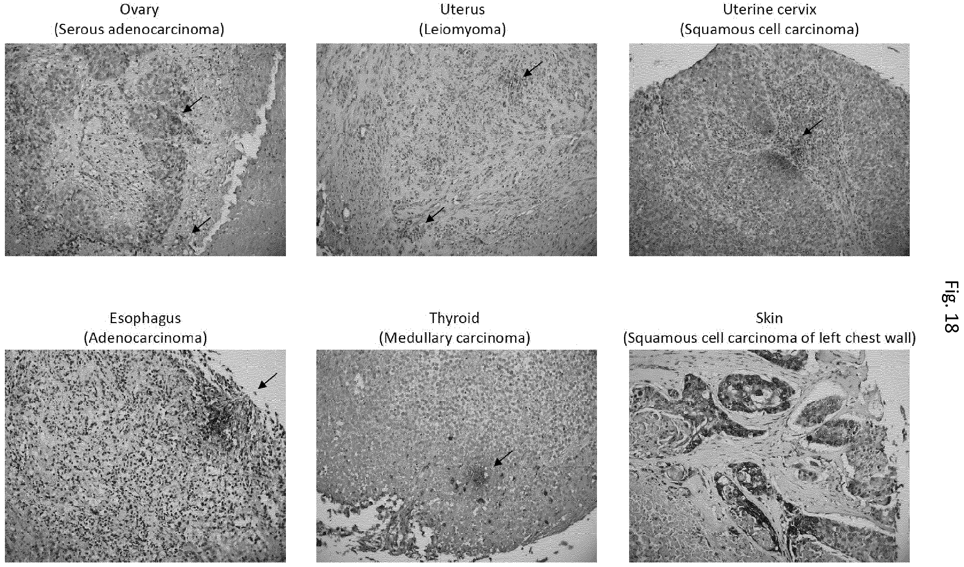

Figure 18 shows the results of immunohistological staining with the IMI4C4a antibody performed on ovarian cancer, uterine cancer, uterine cervical cancer, esophageal cancer, thyroid cancer and skin cancer tissues. In each cancer tissue, staining was observed in a part of cancer site.

The above results indicated that MCT5 had the ability to stain a part of cancer, at least in breast cancer, colorectal cancer, lung cancer, ovarian cancer, uterine cancer, uterine cervical cancer, esophageal cancer, thyroid cancer and skin cancer. In light of the finding that MCT5 enhanced invasion in cultured breast cancer cells, MCT5 was shown to be a marker available for use in the diagnosis of highly malignant cancer tissue. Moreover, the antibody of the present invention was demonstrated to be useful for detection and diagnosis of at least breast cancer, colorectal cancer, lung cancer, ovarian cancer, uterine cancer, uterine cervical cancer, esophageal cancer, thyroid cancer and skin cancer.

### Example 6 Flow cytometer analysis with anti-MCT5 antibody

### (1) Flow cytometer analysis using cultured human colorectal cancer cells

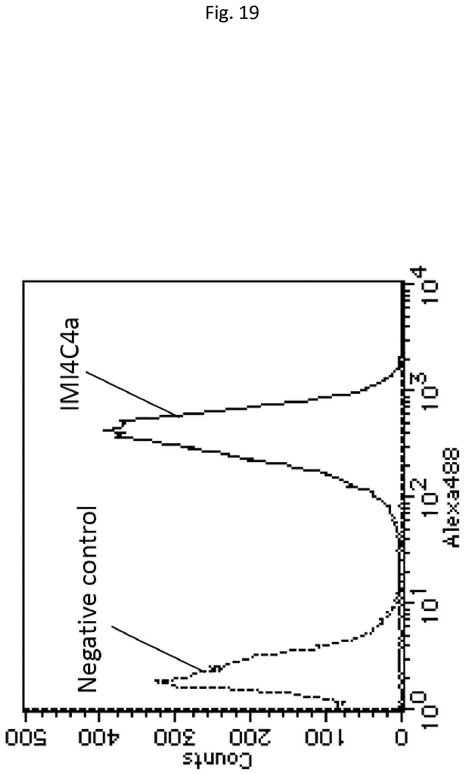

HCT116 cells, which are human colorectal cancer cells, were cultured to reach 90% confluency. After washing twice with PBS, the cells were detached non-enzymatically from the plates with Cell Dissociation Buffer (Invitrogen) and collected into 1.5 mL tubes. The IMI4C4a antibody was diluted with PBS-T to give a final concentration of 10 µg/mL and added in a volume of 50 µL per tube, and then reacted on ice for 60 minutes. As a control for comparison purposes, anti-Trx-tag antibody (clone No.: Mu2C34, subclass: IgG2a, Order-made Medical Research Inc., Japan) was used and reacted in the same manner. After washing twice with PBS + 1% FBS, Alexa Fluor 488-labeled goat anti-mouse IgG (Invitrogen) was added as a 1/200 dilution in PBS + 1% FBS, and then reacted on ice for 30 minutes. After washing twice with PBS + 2% PBS(FBS?), the cells were analyzed by FACS LSR (BD). The results obtained are shown in Figure 19. Clear signals were observed upon reaction with IMI4C4a, when compared to the anti-Tag antibody (negative control). Namely, the IMI4C4a antibody was found to react with living cells (living cancer cells).

In the Uniprot database, a region consisting of amino acids at positions 196 to 299 (SEQ ID NO: 7) in the amino acid sequence shown in SEQ ID NO: 2 of human MCT5 registered as 015374 (MOT5_HUMAN) is predicted to be an intracellular region (Figure 7). Namely, the IMI4C4a monoclonal antibody recognizing this region is predicted to recognize an intracellular region; and hence it was expected that the antibody would not be able to react with living cancer cells because of being unable to permeate the cell membrane. However, as shown in Figure 19, IMI4C4a was found to clearly react with HCT116 cells, which are human colorectal cancer cells. This indicates that at least a part of the region consisting of amino acids at positions 196 to 299 (SEQ ID NO: 7), which is predicted on the database to be an intracellular region of MCT5, is exposed to the extracellular environment. This result is a novel finding which has been unknown.

### (2) Comparison with existing antibodies

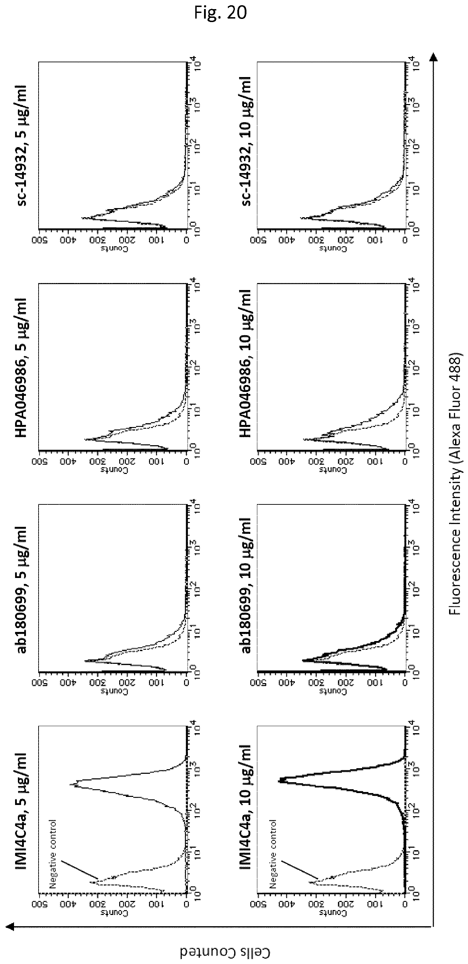

Three types of existing antibodies against MCT5 (ab180699, HPA046986 and sc-14932) were compared with the IMI4C4a antibody in a flow cytometer. The same experimental procedures as shown in Example 6 (1) above were repeated. In this case, as a secondary antibody for ab180699 and HPA04986, which are rabbit polyclonal antibodies, Alexa Fluor 488-labeled goat anti-rabbit IgG (Invitrogen) was used as a 1/200 dilution in PBS + 1% FBS, while as a secondary antibody for the sc-14932 antibody, which is goat polyclonal antibody, Alexa Fluor 488-labeled donkey anti-goat IgG (Invitrogen) was used as a 1/200 dilution in PBS + 1% FBS. The results obtained are shown in Figure 20.

The IMI4C4a antibody was found to clearly bind to HCT116 cells, whereas ab180699 and HPA046986 showed only slight reaction with HCT116 cells. Moreover, the sc-14932 antibody showed no reaction with HCT116 cells. These results indicated that the IMI4C4a antibody was required to detect living cancer cells and any other antibodies showed little reaction with living cancer cells.

### (3) Immunocytological staining

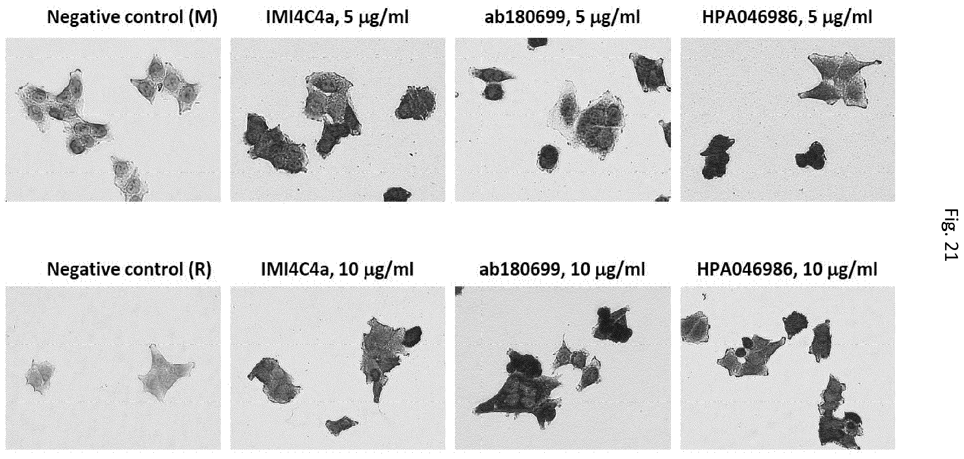

Two types of existing antibodies against MCT5 (ab180699 and HPA046986) were used for immunocytological staining of formalin-fixed HCT116 cells. The same procedure as shown in Example 4 (2) was repeated for formalin fixation of the cells, while the same procedure as shown in Example 5 (1) was repeated for reaction with the antibodies and detection. It should be noted that a Histofine (R) kit (Nichirei Corporation, Japan) was used for ab180699 and HPA046986, and blocking, secondary antibody reaction, washing and detection reaction were conducted in accordance with the protocols attached to the kit. The results obtained are shown in Figure 21. As a negative control, an antibody-free solution (PBS-T) was used for primary antibody reaction.

In the formalin-fixed cells, their cell membrane and cytoplasm were stained equally with all the antibodies used, when compared to the negative control. This indicated that existing antibodies were also available for use in the detection of formalin-fixed samples.

### Example 7 Epitope analysis of IMI4C4a antibody

### (1) Preparation of ICD4 partial proteins

To narrow down candidate regions to which the IMI4C4a antibody binds, ICD4 recombinant partial proteins were prepared.

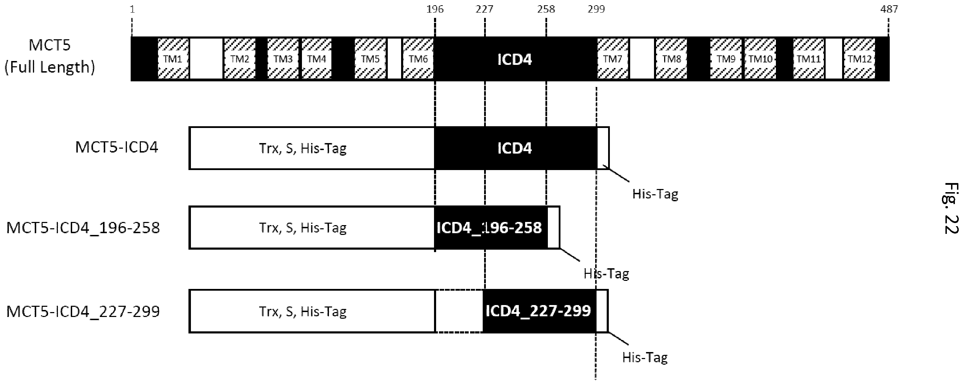

The plasmid prepared in Example 1 (3) was used as a template for PCR reaction with the following primers and with KOD PLUS (TOYOBO) to thereby amplify DNA comprising a nucleotide sequence (SEQ ID NO: 41) encoding amino acids at positions 196 to 258 of MCT5. The same procedure was also repeated to amplify DNA comprising a nucleotide sequence (SEQ ID NO: 42) encoding amino acids at positions 227 to 299 of MCT5. Figure 22 shows a schematic view of positions on the amino acid sequence which are matched between MCT5 and ICD4, ICD4_196-258 or ICD4_227-299.

### Primer sequences

hSLC16A4 ICD4-F1: 5'-GCGCGGATCCGAGACCCATCCATATCAAAAGTG-3' (SEQ ID NO: 38)
hSLC16A4 ICD4_196-258-R1: 5'-AAAGTCGACTTGATTTTGTGAGACTGTTAAATTTTTGC-3' (SEQ ID NO: 43)
ICD4_196-258 DNA sequence (SEQ ID NO: 41) 
AGACCCATCCATATCAAAAGTGAGAACAATTCTGGTATTAAAGATAAAGGCA
GCAGTTTGTCTGCACATGGTCCAGAGGCACATGCAACAGAAACACACTGCCA
TGAGACAGAAGAGTCTACCATCAAGGACAGTACTACGCAGAAGGCTGGACT
ACCTAGCAAAAATTTAACAGTCTCACAAAATCAA
ICD4_196-258 amino acid sequence (SEQ ID NO: 8)

RPIHIKSENNSGIKDKGSSLSAHGPEAHATETHCHETEEESTIKDSTTQKAGLPSKN
LTVSQNQ

### Primer sequences

hSLC16A4 ICD4_227-299F1: 5'-AAAGGATCCACACACTGCCATGAGACAGAAG-3' (SEQ ID NO: 48)
hSLC16A4 ICD4-R1: 5'-GCGCGCGTCGACAGGATTTCTAAAGAGAGAAATGTC-3' (SEQ ID NO: 39)
ICD4_227-299 DNA sequence (SEQ ID NO: 42) 
ACACACTGCCATGAGACAGAAGAGTCTACCATCAAGGACAGTACTACGCAG
AAGGCTGGACTACCTAGCAAAAATTAACAGTCTCACAAAATCAAAGTGAAG
AGTTCTACAATGGGCCTAACAGGAACAGACTGTTATTAAAGAGTGATGAAGA
AAGTGATAAGGTTATTTCGTGGAGCTGCAAACAACTGTTTGACATTTCTCTCT
TTAGAAATCCT
ICD4_227-299 amino acid sequence (SEQ ID NO: 9)

THCHETEEESTIKDSTTQKAGLPSKNLTVSQNQSEEFYNGPNRNRLLLKSDEESDK
VISWSCKQLFDISLFRNP

The resulting amplified fragments were each cleaved with restriction enzymes (*Bam*HI and *Sal*I) at the restriction enzyme sites located on the primers and then integrated into pET32b vector for ICD4_196-258 and into pET32a vector (Invitrogen) for ICD4_227-299 to thereby obtain DNAs each comprising a nucleotide sequence encoding a partial protein of MCT5 having Trx-, S- and His-tags on the N-terminal side and a His-tag on the C-terminal side. These vectors were used to express and purify MCT5 partial proteins in the same manner as shown in Example 3 (2), thereby obtaining the desired partial proteins.

### (2) Competitive inhibition test by ELISA

A competitive inhibition test was performed using the following four types of proteins: the partial protein (ICD4) obtained in Example 3 (2), the two partial proteins (ICD4_196-258 and ICD4_227-299) obtained in (1) above, and a protein having Trx-, S- and His-tags (Tag) serving as a negative control.

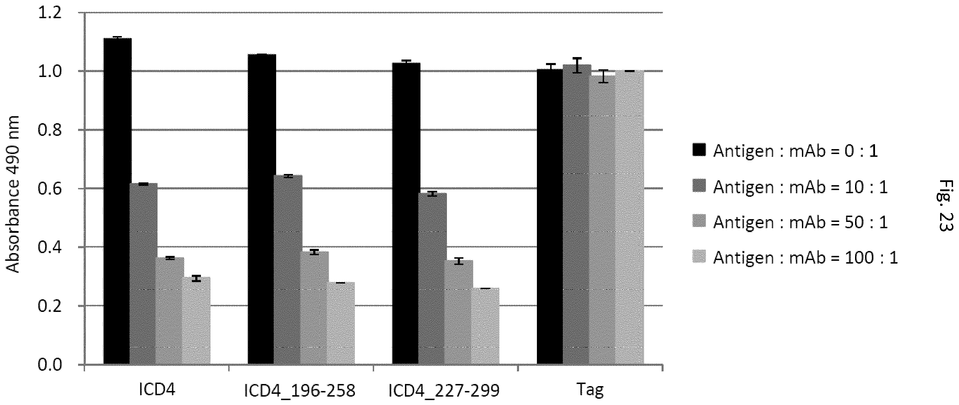

First, the IMI4C4a antibody at 50 ng/mL (333 pM) was reacted at room temperature for 1 hour with these four types of proteins which had been prepared at 3.33 nM (10-fold), 16.7 nM (50-fold) and 33.3 nM (100-fold). Then, a plate in which 50 ng of ICD4 had been added and immobilized per well was provided, and solutions of the above recombinant proteins mixed with the IMI4C4a antibody were added to this plate. After reaction at room temperature for 1 hour, the reaction between antibody and antigen was analyzed in the same manner as shown in Example 3 (5). The results obtained are shown in Figure 23. The binding of IMI4C4a to ICD4 was inhibited by both ICD4_196-258 and ICD4_227-299, indicating that an epitope recognized by IMI4C4a was located within a peptide sequence at positions 227 to 258 (SEQ ID NO: 14) of MCT5.

### (3) Competitive inhibition test in a flow cytometer

Then, analysis was conducted to test whether the binding of the IMI4C4a antibody to cells was also inhibited by the recombinant proteins.

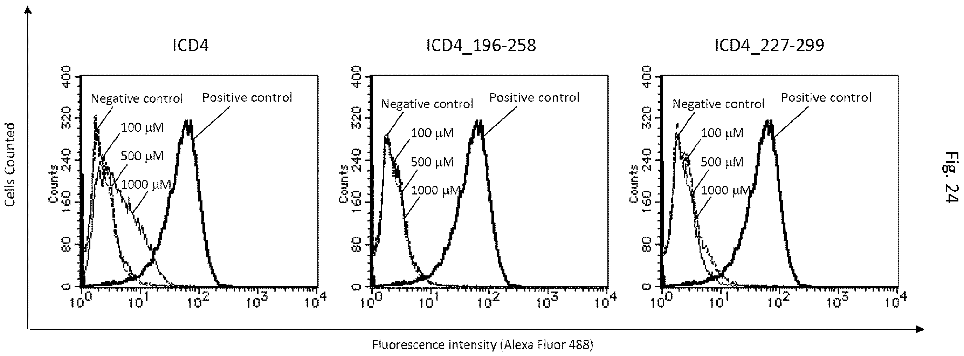

The IMI4C4a antibody at 1.5 µg/mL (20 µM) was mixed respectively with ICD4, ICD4_196-258 and ICD4_227-299 which had been prepared at 100, 500 or 1000 µM, followed by reaction at room temperature for 1 hour. These reaction solutions were reacted on ice for 30 minutes with HCT116 cells (4 × 10⁵ cells). The subsequent washing, secondary antibody reaction and detection were conducted in the same manner as shown in Example 6 (1). The experimental results obtained are shown in Figure 24.

When compared to the sample stained with the antibody alone (Positive control), signals clearly disappeared upon addition of ICD4 (SEQ ID NO: 7), ICD4_196-258 (SEQ ID NO: 8) and ICD4_227-299 (SEQ ID NO: 9), indicating that the binding of the IMI4C4a antibody to the cells was inhibited by the partial proteins of MCT5.

### (4) Determination of epitope

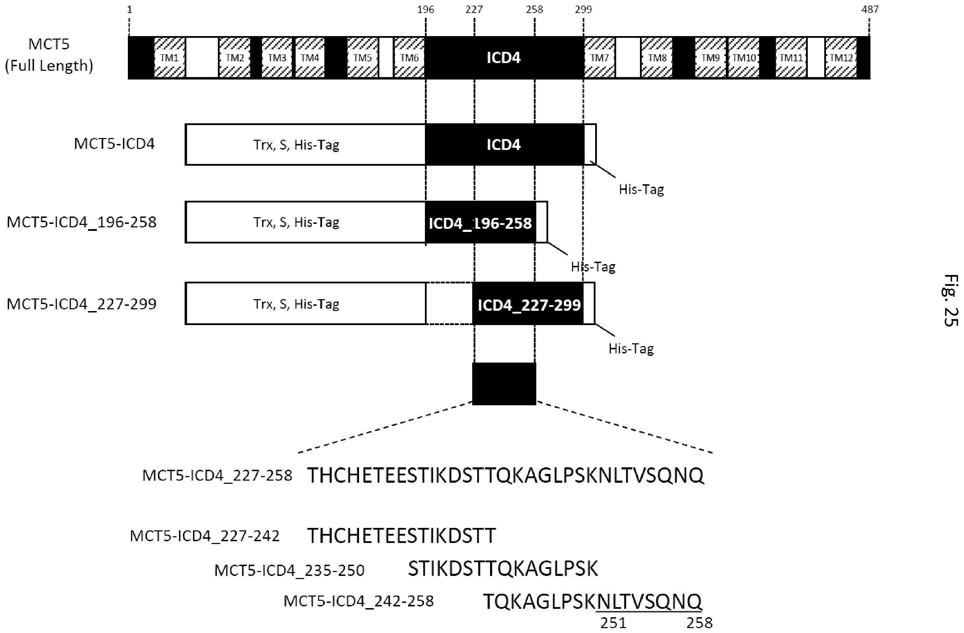

In view of the results obtained in (3) above, the IMI4C4a antibody was predicted to bind to a region within ICD4_227-258 (SEQ ID NO: 14). Then, the following three peptides, i.e., ICD4_227-242 (SEQ ID NO: 10), ICD4_235-250 (SEQ ID NO: 11) and ICD4_242-258 (SEQ ID NO: 12) were chemically synthesized and subjected to the same competitive inhibition test by ELISA as shown in (2) above. Figure 25 shows positions on the amino acid sequence which are matched among ICD4 and the synthesized peptides.

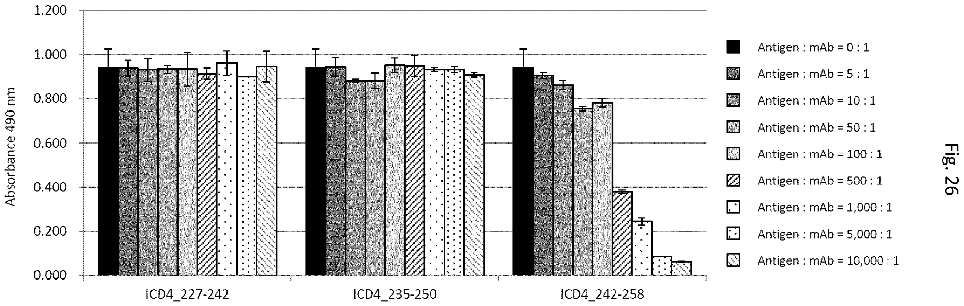

Relative to the antibody at 50 ng/ml (333 pM), ICD44_227-242, ICD4_235-250 and ICD4_242-258 were each prepared at 1.67 nM (5-fold), 3.33 nM (10-fold), 16.7 nM (50-fold), 33.3 nM (100-fold), 167 nM (500-fold), 333 nM (1,000-fold), 1.66 µM (5,000-fold) and 3.33 mM (10,000-fold), followed by analysis in the same manner as shown in (2) above. The experimental results obtained are shown in Figure 26.

ICD4_227-242 and ICD4_235-250 were not able to inhibit the binding of IMI4C4a to ICD4 even when added at a 10,000-fold molar ratio relative to the antibody concentration, whereas ICD4_242-258 started to show an inhibitory effect at a 50-fold molar ratio and showed significant inhibition when added at a 500-fold or higher molar ratio. ICD4_235-250 (SEQ ID NO: 11) showing no inhibitory effect and ICD4_242-258 (SEQ ID NO: 12) showing an inhibitory effect overlap with each other in amino acids at positions 235 to 250 of MCT5, and hence the IMI4C4a antibody was found to recognize an amino acid sequence located at positions 251 to 258 (SEQ ID NO: 13) of MCT5.

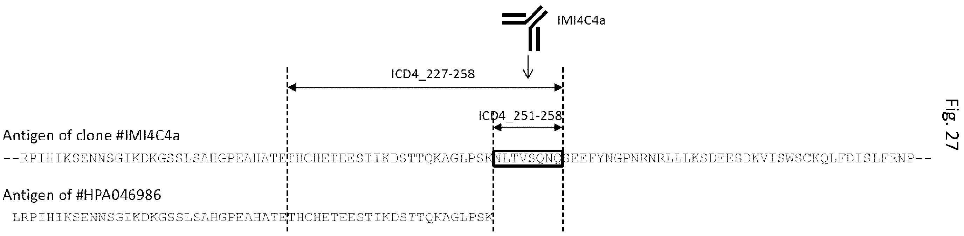

A region comprising an epitope recognized by the IMI4C4a antibody, i.e., ICD4_251-258 (SEQ ID NO: 13) is an amino acid sequence which is not contained in the antigen (ICD4_195-250 (SEQ ID NO: 15)) used to obtain the existing antibody HPA046986. Figure 27 shows a schematic view of positions on the amino acid sequence which are matched among ICD4 (SEQ ID NO: 7), ICD4_251-258 (SEQ ID NO: 13) which is an amino acid sequence comprising an epitope recognized by the IMI4C4a antibody, and ICD4_195-250 (SEQ ID NO: 15) which is an antigen used to obtain the existing antibody HPA046986. Namely, an anti-MCT5 antibody having properties as described above is required to be a monoclonal antibody which recognizes an amino acid sequence comprising ICD4_251-258 (SEQ ID NO: 13).

ICD4_196-258 (SEQ ID NO: 8) 
RPIHIKSENNSGIKDKGSSLSAHGPEAHATETHCHETEEESTIKDSTTQKAGLPSKN
LTVSQNQ
ICD4_227-299 (SEQ ID NO: 9) 
THCHETEESTIKDSTTQKAGLPSKNLTVSQNQSEEFYNGPNRNRLLLKSDEESDK
VISWSCKQLFDISLFRNP
ICD4_227-242 (SEQ ID NO: 10)
   THCHETEESTIKDSTT
ICD4_235-250 (SEQ ID NO: 11)
   STIKDSTTQKAGLPSK
ICD4_242-258 (SEQ ID NO: 12)
   TQKAGLPSKNLTVSQNQ
ICD4_251-258 (SEQ ID NO: 13)
   NLTVSQNQ
ICD4_227-258 (SEQ ID NO: 14)
   THCHETEESTIKDSTTQKAGLPSKNLTVSQNQ
ICD4_195-250 (SEQ ID NO: 15) LRPIHIKSENNSGIKDKGSSLSAHGPEAHATETHCHETEESTIKDSTTQKAGLPSK

### INDUSTRIAL APPLICABILITY

The present invention enables the highly sensitive detection of cancer cells having high invasive capacity which have not been able to be detected by conventional antibodies.

### Sequence Listing Free Text

SEQ ID NO: 16: synthetic DNA
SEQ ID NOs: 17 to 20: synthetic peptides
SEQ ID NOs: 21 to 24: synthetic DNAs
SEQ ID NOs: 25 to 28: synthetic peptides
SEQ ID NOs: 29 to 39: synthetic DNAs
SEQ ID NO: 43: synthetic DNA
SEQ ID NO: 48: synthetic DNA

## Claims

1. A reagent for detection or diagnosis of a cancer having invasive capacity, which comprises a monoclonal antibody against monocarboxylate transporter 5 (MCT5) or a fragment of the antibody.

2. The reagent according to claim 1, wherein the antibody or fragment thereof binds to the extracellular region of monocarboxylate transporter 5 (MCT5).

3. The reagent according to claim 2, wherein the extracellular region comprises the amino acid sequence shown in SEQ ID NO: 15.

4. The reagent according to claim 1, wherein the cancer is at least one selected from the group consisting of breast cancer, colorectal cancer, lung cancer, ovarian cancer, uterine cancer, uterine cervical cancer, esophageal cancer, thyroid cancer, skin cancer, gastric cancer, pancreatic cancer, brain tumor, tongue cancer, small intestinal cancer, duodenal cancer, bladder cancer, kidney cancer, liver cancer, prostate cancer, cervical cancer, gallbladder cancer, pharyngeal cancer, sarcoma, melanoma, leukemia, lymphoma and multiple myeloma.

5. The reagent according to claim 1, wherein the cancer is at least one selected from the group consisting of breast cancer, colorectal cancer, lung cancer, ovarian cancer, uterine cancer, uterine cervical cancer, esophageal cancer, thyroid cancer and skin cancer.

6. The reagent according to claim 1, wherein the cancer is at least one selected from the group consisting of breast cancer, colorectal cancer and lung cancer.

7. The reagent according to claim 1, wherein the cancer is breast cancer or colorectal cancer.

8. A method for detection of cancer, which comprises the step of contacting a monoclonal antibody against monocarboxylate transporter 5 (MCT5) or a fragment of the antibody with a biological sample taken from a subject to thereby detect MCT5 in the sample.

9. A method for diagnosis of cancer, which comprises the step of contacting a monoclonal antibody against monocarboxylate transporter 5 (MCT5) or a fragment of the antibody with a biological sample taken from a subject to thereby detect MCT5 in the sample.

10. The method according to claim 8 or 9, wherein the cancer is a cancer having invasive capacity.

11. A monoclonal antibody against monocarboxylate transporter 5 (MCT5) or a fragment of the antibody.

12. A kit for detection of cancer, which comprises a monoclonal antibody against monocarboxylate transporter 5 (MCT5) or a fragment of the antibody.

13. The kit according to claim 12, wherein the cancer is a cancer having invasive capacity.


SEQUENCE LISTING

<110>  Order-made Medical Redearch Inc.

<120>  An agent for detecting or diagnosing a cancer cell having high
       invasive capacity

<130>  G1187WO

<150>  JP2014-203162
<151>  2014-10-01

<160>  48

<170>  PatentIn version 3.5

<210>  1
<211>  1464
<212>  DNA
<213>  Homo sapiens



<220>
<221>  CDS
<222>  (1)..(1461)

<400>  1
atg ctg aag agg gag ggg aag gtc caa cct tac act aaa acc ctg gat        48
Met Leu Lys Arg Glu Gly Lys Val Gln Pro Tyr Thr Lys Thr Leu Asp
1               5                   10                  15

gga gga tgg gga tgg atg att gtg att cat ttt ttc ctg gtg aat gtg        96
Gly Gly Trp Gly Trp Met Ile Val Ile His Phe Phe Leu Val Asn Val
            20                  25                  30

ttt gtg atg ggg atg acc aag act ttt gca att ttc ttt gtg gtc ttt       144
Phe Val Met Gly Met Thr Lys Thr Phe Ala Ile Phe Phe Val Val Phe
        35                  40                  45

caa gaa gag ttt gaa ggc acc tca gag caa att ggt tgg att gga tcc       192
Gln Glu Glu Phe Glu Gly Thr Ser Glu Gln Ile Gly Trp Ile Gly Ser
    50                  55                  60

atc atg tca tct ctt cgt ttt tgt gca ggt ccc ctg gtt gct att att       240
Ile Met Ser Ser Leu Arg Phe Cys Ala Gly Pro Leu Val Ala Ile Ile
65                  70                  75                  80

tgt gac ata ctt gga gag aaa act acc tcc att ctt ggg gct ttc gtt       288
Cys Asp Ile Leu Gly Glu Lys Thr Thr Ser Ile Leu Gly Ala Phe Val
                85                  90                  95

gtt act ggt gga tat ctg atc agc agc tgg gcc aca agt att cct ttt       336
Val Thr Gly Gly Tyr Leu Ile Ser Ser Trp Ala Thr Ser Ile Pro Phe
            100                 105                 110

ctt tgt gtg act atg gga ctt cta ccc ggt ttg ggt tct gct ttc tta       384
Leu Cys Val Thr Met Gly Leu Leu Pro Gly Leu Gly Ser Ala Phe Leu
        115                 120                 125

tac caa gtg gct gct gtg gta act acc aaa tac ttc aaa aaa cga ttg       432
Tyr Gln Val Ala Ala Val Val Thr Thr Lys Tyr Phe Lys Lys Arg Leu
    130                 135                 140

gct ctt tct aca gct att gcc cgt tct ggg atg gga ctg act ttt ctt     480
Ala Leu Ser Thr Ala Ile Ala Arg Ser Gly Met Gly Leu Thr Phe Leu
145                 150                 155                 160

ttg gca ccc ttt aca aaa ttc ctg ata gat ctg tat gac tgg aca gga     528
Leu Ala Pro Phe Thr Lys Phe Leu Ile Asp Leu Tyr Asp Trp Thr Gly
                165                 170                 175

gcc ctt ata tta ttt gga gct atc gca ttg aat ttg gtg cct tct agt     576
Ala Leu Ile Leu Phe Gly Ala Ile Ala Leu Asn Leu Val Pro Ser Ser
            180                 185                 190

atg ctc tta aga ccc atc cat atc aaa agt gag aac aat tct ggt att     624
Met Leu Leu Arg Pro Ile His Ile Lys Ser Glu Asn Asn Ser Gly Ile
        195                 200                 205

aaa gat aaa ggc agc agt ttg tct gca cat ggt cca gag gca cat gca     672
Lys Asp Lys Gly Ser Ser Leu Ser Ala His Gly Pro Glu Ala His Ala
    210                 215                 220

aca gaa aca cac tgc cat gag aca gaa gag tct acc atc aag gac agt     720
Thr Glu Thr His Cys His Glu Thr Glu Glu Ser Thr Ile Lys Asp Ser
225                 230                 235                 240

act acg cag aag gct gga cta cct agc aaa aat tta aca gtc tca caa     768
Thr Thr Gln Lys Ala Gly Leu Pro Ser Lys Asn Leu Thr Val Ser Gln
                245                 250                 255

aat caa agt gaa gag ttc tac aat ggg cct aac agg aac aga ctg tta     816
Asn Gln Ser Glu Glu Phe Tyr Asn Gly Pro Asn Arg Asn Arg Leu Leu
            260                 265                 270

tta aag agt gat gaa gaa agt gat aag gtt att tcg tgg agc tgc aaa     864
Leu Lys Ser Asp Glu Glu Ser Asp Lys Val Ile Ser Trp Ser Cys Lys
        275                 280                 285

caa ctg ttt gac att tct ctc ttt aga aat cct ttc ttc tac ata ttt     912
Gln Leu Phe Asp Ile Ser Leu Phe Arg Asn Pro Phe Phe Tyr Ile Phe
    290                 295                 300

act tgg tct ttt ctc ctc agt cag tta gca tac ttc atc cct acc ttt     960
Thr Trp Ser Phe Leu Leu Ser Gln Leu Ala Tyr Phe Ile Pro Thr Phe
305                 310                 315                 320

cac ctg gta gcc aga gcc aaa aca ctg ggg att gac atc atg gat gcc    1008
His Leu Val Ala Arg Ala Lys Thr Leu Gly Ile Asp Ile Met Asp Ala
                325                 330                 335

tct tac ctt gtt tct gta gca ggt atc ctt gag acg gtc agt cag att    1056
Ser Tyr Leu Val Ser Val Ala Gly Ile Leu Glu Thr Val Ser Gln Ile
            340                 345                 350

att tct gga tgg gtt gct gat caa aac tgg att aag aag tat cat tac    1104
Ile Ser Gly Trp Val Ala Asp Gln Asn Trp Ile Lys Lys Tyr His Tyr
        355                 360                 365

cac aag tct tac ctc atc ctc tgc ggc atc act aac ctg ctt gct cct    1152
His Lys Ser Tyr Leu Ile Leu Cys Gly Ile Thr Asn Leu Leu Ala Pro
    370                 375                 380

tta gcc acc aca ttt cca cta ctt atg acc tac acc atc tgc ttt gcc    1200
Leu Ala Thr Thr Phe Pro Leu Leu Met Thr Tyr Thr Ile Cys Phe Ala
385                 390                 395                 400

atc ttt gct ggt ggt tac ctg gca ttg ata ctg cct gta ctg gtt gat 1248
Ile Phe Ala Gly Gly Tyr Leu Ala Leu Ile Leu Pro Val Leu Val Asp
                405                 410                 415

ctg tgt agg aat tct aca gta aac agg ttt ttg gga ctt gcc agt ttc 1296
Leu Cys Arg Asn Ser Thr Val Asn Arg Phe Leu Gly Leu Ala Ser Phe
            420                 425                 430

ttt gct ggg atg gct gtc ctt tct gga cca cct ata gca ggc tgg tta 1344
Phe Ala Gly Met Ala Val Leu Ser Gly Pro Pro Ile Ala Gly Trp Leu
        435                 440                 445

tat gat tat acc cag aca tac aat ggc tct ttc tac ttc tct ggc ata 1392
Tyr Asp Tyr Thr Gln Thr Tyr Asn Gly Ser Phe Tyr Phe Ser Gly Ile
    450                 455                 460

tgc tat ctc ctc tct tca gtt tcc ttt ttt ttt gta cca ttg gcc gaa 1440
Cys Tyr Leu Leu Ser Ser Val Ser Phe Phe Phe Val Pro Leu Ala Glu
465                 470                 475                 480

aga tgg aaa aac agt ctg acc tga 1464
Arg Trp Lys Asn Ser Leu Thr
                485

<210> 2
<211> 487
<212> PRT
<213> Homo sapiens

<400> 2

Met Leu Lys Arg Glu Gly Lys Val Gln Pro Tyr Thr Lys Thr Leu Asp
1               5                   10                  15

Gly Gly Trp Gly Trp Met Ile Val Ile His Phe Phe Leu Val Asn Val
            20                  25                  30

Phe Val Met Gly Met Thr Lys Thr Phe Ala Ile Phe Phe Val Val Phe
        35                  40                  45

Gln Glu Glu Phe Glu Gly Thr Ser Glu Gln Ile Gly Trp Ile Gly Ser
    50                  55                  60

Ile Met Ser Ser Leu Arg Phe Cys Ala Gly Pro Leu Val Ala Ile Ile
65                  70                  75                  80

Cys Asp Ile Leu Gly Glu Lys Thr Thr Ser Ile Leu Gly Ala Phe Val
                85                  90                  95

Val Thr Gly Gly Tyr Leu Ile Ser Ser Trp Ala Thr Ser Ile Pro Phe
            100                 105                 110

Leu Cys Val Thr Met Gly Leu Leu Pro Gly Leu Gly Ser Ala Phe Leu
        115                 120                 125

Tyr Gln Val Ala Ala Val Val Thr Thr Lys Tyr Phe Lys Lys Arg Leu
    130                 135                 140

Ala Leu Ser Thr Ala Ile Ala Arg Ser Gly Met Gly Leu Thr Phe Leu
145                 150                 155                 160

Leu Ala Pro Phe Thr Lys Phe Leu Ile Asp Leu Tyr Asp Trp Thr Gly
                165                 170                 175

Ala Leu Ile Leu Phe Gly Ala Ile Ala Leu Asn Leu Val Pro Ser Ser
            180                 185                 190

Met Leu Leu Arg Pro Ile His Ile Lys Ser Glu Asn Asn Ser Gly Ile
        195                 200                 205

Lys Asp Lys Gly Ser Ser Leu Ser Ala His Gly Pro Glu Ala His Ala
    210                 215                 220

Thr Glu Thr His Cys His Glu Thr Glu Glu Ser Thr Ile Lys Asp Ser
225                 230                 235                 240

Thr Thr Gln Lys Ala Gly Leu Pro Ser Lys Asn Leu Thr Val Ser Gln
                245                 250                 255

Asn Gln Ser Glu Glu Phe Tyr Asn Gly Pro Asn Arg Asn Arg Leu Leu
            260                 265                 270

Leu Lys Ser Asp Glu Glu Ser Asp Lys Val Ile Ser Trp Ser Cys Lys
        275                 280                 285

Gln Leu Phe Asp Ile Ser Leu Phe Arg Asn Pro Phe Phe Tyr Ile Phe
    290                 295                 300

Thr Trp Ser Phe Leu Leu Ser Gln Leu Ala Tyr Phe Ile Pro Thr Phe
305                 310                 315                 320

His Leu Val Ala Arg Ala Lys Thr Leu Gly Ile Asp Ile Met Asp Ala
                325                 330                 335

Ser Tyr Leu Val Ser Val Ala Gly Ile Leu Glu Thr Val Ser Gln Ile
            340                 345                 350

Ile Ser Gly Trp Val Ala Asp Gln Asn Trp Ile Lys Lys Tyr His Tyr
        355                 360                 365

His Lys Ser Tyr Leu Ile Leu Cys Gly Ile Thr Asn Leu Leu Ala Pro

370                 375                 380

Leu Ala Thr Thr Phe Pro Leu Leu Met Thr Tyr Thr Ile Cys Phe Ala
385                 390                 395                 400

Ile Phe Ala Gly Gly Tyr Leu Ala Leu Ile Leu Pro Val Leu Val Asp
                405                 410                 415

Leu Cys Arg Asn Ser Thr Val Asn Arg Phe Leu Gly Leu Ala Ser Phe
            420                 425                 430

Phe Ala Gly Met Ala Val Leu Ser Gly Pro Pro Ile Ala Gly Trp Leu
        435                 440                 445

Tyr Asp Tyr Thr Gln Thr Tyr Asn Gly Ser Phe Tyr Phe Ser Gly Ile
    450                 455                 460

Cys Tyr Leu Leu Ser Ser Val Ser Phe Phe Phe Val Pro Leu Ala Glu
465                 470                 475                 480

Arg Trp Lys Asn Ser Leu Thr
                485

<210>  3
<211>  1503
<212>  DNA
<213>  Mus musculs

<220>
<221>  CDS
<222>  (1)..(1500)

<400>  3
atg gtg aag aag gaa aaa acg ccc tcc cct tac acc aaa ccc ctt gat        48
Met Val Lys Lys Glu Lys Thr Pro Ser Pro Tyr Thr Lys Pro Leu Asp
1               5                   10                  15

gga gga tgg ggg tgg atg gtc gtg ctt cat ttt ttc ctg gta aat gtg        96
Gly Gly Trp Gly Trp Met Val Val Leu His Phe Phe Leu Val Asn Val
            20                  25                  30

ttt gtg atg gga atg acc aag aca ttt gca att ttc ttt gtg gtt ttt       144
Phe Val Met Gly Met Thr Lys Thr Phe Ala Ile Phe Phe Val Val Phe
        35                  40                  45

caa gaa gag ttt gaa ggc act tca gag caa att ggt tgg att gga tcc       192
Gln Glu Glu Phe Glu Gly Thr Ser Glu Gln Ile Gly Trp Ile Gly Ser
    50                  55                  60

att atg tca tca ctc cgt ttc tct gca ggt ccc ctg gct gct atc atc       240
Ile Met Ser Ser Leu Arg Phe Ser Ala Gly Pro Leu Ala Ala Ile Ile
65                  70                  75                  80

tgt gac gta ctt gga gag aaa gct acc tcc att ctt ggg acg ttc ctt       288

Cys Asp Val Leu Gly Glu Lys Ala Thr Ser Ile Leu Gly Thr Phe Leu
                85                  90                  95

gtc tct ggt ggc tat gtc atc agc agc tgg gcc aca ggc att ccc ttt      336
Val Ser Gly Gly Tyr Val Ile Ser Ser Trp Ala Thr Gly Ile Pro Phe
            100                 105                 110

ctt tgt gtg acc atg ggg ctg cta cct ggt ttg gga tct gct ttc ttg      384
Leu Cys Val Thr Met Gly Leu Leu Pro Gly Leu Gly Ser Ala Phe Leu
        115                 120                 125

tac caa gta gct gct gtg gta att acc aaa tat ttc aaa aaa cga ttg      432
Tyr Gln Val Ala Ala Val Val Ile Thr Lys Tyr Phe Lys Lys Arg Leu
    130                 135                 140

ggc ctt tct aca gca att gcc cgt tct gga atg ggg ctg aca ttt ttg      480
Gly Leu Ser Thr Ala Ile Ala Arg Ser Gly Met Gly Leu Thr Phe Leu
145                 150                 155                 160

ttg gca ccc ttt aca aaa ttc ctc ata gat ctt tat gac tgg aca ggc      528
Leu Ala Pro Phe Thr Lys Phe Leu Ile Asp Leu Tyr Asp Trp Thr Gly
                165                 170                 175

gct ctt att tta ttt gga gca att ata ttg aac ttg gtg cct tcc agc      576
Ala Leu Ile Leu Phe Gly Ala Ile Ile Leu Asn Leu Val Pro Ser Ser
            180                 185                 190

atg ctc tta aga ccc atc cac agc caa tca aac aac aat tct gac att      624
Met Leu Leu Arg Pro Ile His Ser Gln Ser Asn Asn Asn Ser Asp Ile
        195                 200                 205

gaa aac aaa ggt agc att ttg tct gca act gaa cca gag gca tca tac      672
Glu Asn Lys Gly Ser Ile Leu Ser Ala Thr Glu Pro Glu Ala Ser Tyr
    210                 215                 220

aag aca gag aca tcg aag tgc aag gag aca cag gag cct ttc atc aaa      720
Lys Thr Glu Thr Ser Lys Cys Lys Glu Thr Gln Glu Pro Phe Ile Lys
225                 230                 235                 240

gac agt act atg aag aaa tct gaa caa cct acc aca acc tta act gtc      768
Asp Ser Thr Met Lys Lys Ser Glu Gln Pro Thr Thr Thr Leu Thr Val
                245                 250                 255

tta gga aat caa agc gaa gag ttc agc aat agg cct cac agg aac agg      816
Leu Gly Asn Gln Ser Glu Glu Phe Ser Asn Arg Pro His Arg Asn Arg
            260                 265                 270

ccg tta ctt atg agc aat ggc aaa agt cac aag aaa aag ttt gtt tca      864
Pro Leu Leu Met Ser Asn Gly Lys Ser His Lys Lys Lys Phe Val Ser
        275                 280                 285

tgg aac tgc aaa caa aaa ctt tta gat atc tcg ctc ttt aga aac cct      912
Trp Asn Cys Lys Gln Lys Leu Leu Asp Ile Ser Leu Phe Arg Asn Pro
    290                 295                 300

ttc ttc tac att ttt acc tgg tct ttt ctc ctg agc caa tta gca tat      960
Phe Phe Tyr Ile Phe Thr Trp Ser Phe Leu Leu Ser Gln Leu Ala Tyr
305                 310                 315                 320

ttc att ccc act ttc cac cta gta gcc aga gca aaa aca ctt ggg att     1008
Phe Ile Pro Thr Phe His Leu Val Ala Arg Ala Lys Thr Leu Gly Ile
                325                 330                 335

gac gta atg gat gcc tcc tac ctt gtg tct gta gct ggt atc act gag 1056
Asp Val Met Asp Ala Ser Tyr Leu Val Ser Val Ala Gly Ile Thr Glu
            340                 345                 350

aca gtc agt cag ctc att tct gga tgg att gct gat caa aat tgg atc 1104
Thr Val Ser Gln Leu Ile Ser Gly Trp Ile Ala Asp Gln Asn Trp Ile
        355                 360                 365

aag aag tat caa tac cac aag tct tac ctc att ctg tgt ggt gtg act 1152
Lys Lys Tyr Gln Tyr His Lys Ser Tyr Leu Ile Leu Cys Gly Val Thr
    370                 375                 380

aac ctg ctt gct cct tta gcc acc aca ttc cca ctc ctt atg gcc tat 1200
Asn Leu Leu Ala Pro Leu Ala Thr Thr Phe Pro Leu Leu Met Ala Tyr
385                 390                 395                 400

acc atc ctt ttt gcc att ttt gct ggt ggc tac ctg gca cta ata ctg 1248
Thr Ile Leu Phe Ala Ile Phe Ala Gly Gly Tyr Leu Ala Leu Ile Leu
                405                 410                 415

cca gta ttg gtt gac cta tct aag aat tct aga gta cac aag ttt ttg 1296
Pro Val Leu Val Asp Leu Ser Lys Asn Ser Arg Val His Lys Phe Leu
            420                 425                 430

gga tat gcc agt ttc ttt gcc ggg ata gct gta ctt tct gga cca cca 1344
Gly Tyr Ala Ser Phe Phe Ala Gly Ile Ala Val Leu Ser Gly Pro Pro
        435                 440                 445

ata gca ggc tgg ata tat gat tac aca cag act tac aca ggc tct ttc 1392
Ile Ala Gly Trp Ile Tyr Asp Tyr Thr Gln Thr Tyr Thr Gly Ser Phe
    450                 455                 460

tat ttc tct ggc acc tgc tat atc ctc tct tca gtt tcc ctt ttt ttt 1440
Tyr Phe Ser Gly Thr Cys Tyr Ile Leu Ser Ser Val Ser Leu Phe Phe
465                 470                 475                 480

gta cca ctg gct gaa aga tgg aaa cgc aaa cag tct gac ctt ctg agg 1488
Val Pro Leu Ala Glu Arg Trp Lys Arg Lys Gln Ser Asp Leu Leu Arg
                485                 490                 495

act aca atc aag taa 1503
Thr Thr Ile Lys
            500

<210> 4
<211> 500
<212> PRT
<213> Mus musculs

<400> 4

Met Val Lys Lys Glu Lys Thr Pro Ser Pro Tyr Thr Lys Pro Leu Asp
1               5                   10                  15

Gly Gly Trp Gly Trp Met Val Val Leu His Phe Phe Leu Val Asn Val
            20                  25                  30

Phe Val Met Gly Met Thr Lys Thr Phe Ala Ile Phe Phe Val Val Phe
        35                  40                  45

Gln Glu Glu Phe Glu Gly Thr Ser Glu Gln Ile Gly Trp Ile Gly Ser
    50                  55                  60

Ile Met Ser Ser Leu Arg Phe Ser Ala Gly Pro Leu Ala Ala Ile Ile
65                  70                  75                  80

Cys Asp Val Leu Gly Glu Lys Ala Thr Ser Ile Leu Gly Thr Phe Leu
                85                  90                  95

Val Ser Gly Gly Tyr Val Ile Ser Ser Trp Ala Thr Gly Ile Pro Phe
            100                 105                 110

Leu Cys Val Thr Met Gly Leu Leu Pro Gly Leu Gly Ser Ala Phe Leu
        115                 120                 125

Tyr Gln Val Ala Ala Val Val Ile Thr Lys Tyr Phe Lys Lys Arg Leu
    130                 135                 140

Gly Leu Ser Thr Ala Ile Ala Arg Ser Gly Met Gly Leu Thr Phe Leu
145                 150                 155                 160

Leu Ala Pro Phe Thr Lys Phe Leu Ile Asp Leu Tyr Asp Trp Thr Gly
                165                 170                 175

Ala Leu Ile Leu Phe Gly Ala Ile Ile Leu Asn Leu Val Pro Ser Ser
            180                 185                 190

Met Leu Leu Arg Pro Ile His Ser Gln Ser Asn Asn Asn Ser Asp Ile
        195                 200                 205

Glu Asn Lys Gly Ser Ile Leu Ser Ala Thr Glu Pro Glu Ala Ser Tyr
    210                 215                 220

Lys Thr Glu Thr Ser Lys Cys Lys Glu Thr Gln Glu Pro Phe Ile Lys
225                 230                 235                 240

Asp Ser Thr Met Lys Lys Ser Glu Gln Pro Thr Thr Thr Leu Thr Val
                245                 250                 255

Leu Gly Asn Gln Ser Glu Glu Phe Ser Asn Arg Pro His Arg Asn Arg
            260                 265                 270

Pro Leu Leu Met Ser Asn Gly Lys Ser His Lys Lys Lys Phe Val Ser
        275                 280                 285

Trp Asn Cys Lys Gln Lys Leu Leu Asp Ile Ser Leu Phe Arg Asn Pro
    290                 295                 300

Phe Phe Tyr Ile Phe Thr Trp Ser Phe Leu Leu Ser Gln Leu Ala Tyr
305                 310                 315                 320

Phe Ile Pro Thr Phe His Leu Val Ala Arg Ala Lys Thr Leu Gly Ile
                325                 330                 335

Asp Val Met Asp Ala Ser Tyr Leu Val Ser Val Ala Gly Ile Thr Glu
            340                 345                 350

Thr Val Ser Gln Leu Ile Ser Gly Trp Ile Ala Asp Gln Asn Trp Ile
        355                 360                 365

Lys Lys Tyr Gln Tyr His Lys Ser Tyr Leu Ile Leu Cys Gly Val Thr
    370                 375                 380

Asn Leu Leu Ala Pro Leu Ala Thr Thr Phe Pro Leu Leu Met Ala Tyr
385                 390                 395                 400

Thr Ile Leu Phe Ala Ile Phe Ala Gly Gly Tyr Leu Ala Leu Ile Leu
                405                 410                 415

Pro Val Leu Val Asp Leu Ser Lys Asn Ser Arg Val His Lys Phe Leu
            420                 425                 430

Gly Tyr Ala Ser Phe Phe Ala Gly Ile Ala Val Leu Ser Gly Pro Pro
        435                 440                 445

Ile Ala Gly Trp Ile Tyr Asp Tyr Thr Gln Thr Tyr Thr Gly Ser Phe
    450                 455                 460

Tyr Phe Ser Gly Thr Cys Tyr Ile Leu Ser Ser Val Ser Leu Phe Phe
465                 470                 475                 480

Val Pro Leu Ala Glu Arg Trp Lys Arg Lys Gln Ser Asp Leu Leu Arg
                485                 490                 495

Thr Thr Ile Lys
            500

<210>  5
<211>  1503
<212>  DNA
<213>  Rattus norvegicus

<220>
<221>  CDS
<222>  (1)..(1500)

<400> 5
atg aag aag agg gaa gaa aag ccc tcc cct tac acc aaa ccc ctg gat 48
Met Lys Lys Arg Glu Glu Lys Pro Ser Pro Tyr Thr Lys Pro Leu Asp
1               5                   10                  15

gga gga tgg gga tgg atg gtt gtg ctt cat ttt ttc ctg gtg aat gtg 96
Gly Gly Trp Gly Trp Met Val Val Leu His Phe Phe Leu Val Asn Val
            20                  25                  30

ttt gtg atg gga atg acc aag aca ttt gca att ttc ttt gtg gtt ttt 144
Phe Val Met Gly Met Thr Lys Thr Phe Ala Ile Phe Phe Val Val Phe
        35                  40                  45

caa gaa gag ttt gaa ggc act tca gag caa att ggt tgg att gga tct 192
Gln Glu Glu Phe Glu Gly Thr Ser Glu Gln Ile Gly Trp Ile Gly Ser
    50                  55                  60

att atg tca tca ctc cgt ttt tct gca ggt ccc ctg gct gct att att 240
Ile Met Ser Ser Leu Arg Phe Ser Ala Gly Pro Leu Ala Ala Ile Ile
65                  70                  75                  80

tgt gac ata ttt gga gaa aaa act acc tcc att ctt ggg act ttc ctt 288
Cys Asp Ile Phe Gly Glu Lys Thr Thr Ser Ile Leu Gly Thr Phe Leu
                85                  90                  95

gtt tct ggt ggc tat ctc atc agc agc tgg gcc aca ggc att ccc ttt 336
Val Ser Gly Gly Tyr Leu Ile Ser Ser Trp Ala Thr Gly Ile Pro Phe
            100                 105                 110

ctt tgt gtg acc atg gga ctg cta cct ggt ttg ggg tct gct ttc ttg 384
Leu Cys Val Thr Met Gly Leu Leu Pro Gly Leu Gly Ser Ala Phe Leu
        115                 120                 125

tac caa gta gct gct gtg gta att acc aaa tat ttc aaa aaa cga ttg 432
Tyr Gln Val Ala Ala Val Val Ile Thr Lys Tyr Phe Lys Lys Arg Leu
    130                 135                 140

ggt ctt tct aca gca att gcc cgt tca ggg atg gga ctg aca ttt ttg 480
Gly Leu Ser Thr Ala Ile Ala Arg Ser Gly Met Gly Leu Thr Phe Leu
145                 150                 155                 160

ttg gca ccc ttt aca aaa ctc ctg ata gat ctt tat gac tgg aca ggt 528
Leu Ala Pro Phe Thr Lys Leu Leu Ile Asp Leu Tyr Asp Trp Thr Gly
                165                 170                 175

gct ctt ata tta ttt gga gct att gca ttg aat ctg gtg cct tcc agc 576
Ala Leu Ile Leu Phe Gly Ala Ile Ala Leu Asn Leu Val Pro Ser Ser
            180                 185                 190

atg ctc cta aga cct gtc cac atc caa tca gac agc aat tct gat att 624
Met Leu Leu Arg Pro Val His Ile Gln Ser Asp Ser Asn Ser Asp Ile
        195                 200                 205

aaa aac aaa ggt agc att ttg tct gca act gga cca gag tta tca cac 672
Lys Asn Lys Gly Ser Ile Leu Ser Ala Thr Gly Pro Glu Leu Ser His
    210                 215                 220

aag aca gaa aca cca aac cgc aat aag gca caa gag tct ctc atc aaa 720
Lys Thr Glu Thr Pro Asn Arg Asn Lys Ala Gln Glu Ser Leu Ile Lys
225                 230                 235                 240

gac agt gct gtg aag aaa tct gga caa cct act aca act tta act gtc 768

Asp Ser Ala Val Lys Lys Ser Gly Gln Pro Thr Thr Thr Leu Thr Val
                245                 250                 255

tta gaa aat caa att gaa gag att agc act agg cct cat agg aac agg 816
Leu Glu Asn Gln Ile Glu Glu Ile Ser Thr Arg Pro His Arg Asn Arg
            260                 265                 270

ccg tta ctt atg agc aat gac aaa act tac aag aaa aag ttt gtt tca 864
Pro Leu Leu Met Ser Asn Asp Lys Thr Tyr Lys Lys Lys Phe Val Ser
        275                 280                 285

tgg aac tgc aaa gaa aaa ctt cta gat att tct ctc ttt aga aat cct 912
Trp Asn Cys Lys Glu Lys Leu Leu Asp Ile Ser Leu Phe Arg Asn Pro
    290                 295                 300

ttc ttc tac ata ttt acc tgg tct ttt ctc ctg agt caa cta gca tat 960
Phe Phe Tyr Ile Phe Thr Trp Ser Phe Leu Leu Ser Gln Leu Ala Tyr
305                 310                 315                 320

ttc att cct acc ttt cat ctg gta gcc aga gca aaa aca ctt gga atc 1008
Phe Ile Pro Thr Phe His Leu Val Ala Arg Ala Lys Thr Leu Gly Ile
                325                 330                 335

gac ata atg gat gcc tcc tac ctt gtg tct gta gct ggt atc act gag 1056
Asp Ile Met Asp Ala Ser Tyr Leu Val Ser Val Ala Gly Ile Thr Glu
            340                 345                 350

aca gtc agt cag ctc att tct gga tgg att gca gat cag aat tgg atc 1104
Thr Val Ser Gln Leu Ile Ser Gly Trp Ile Ala Asp Gln Asn Trp Ile
        355                 360                 365

aag aag tat caa tac cac aag gct tac ctc atc ctg tgt ggt gtt act 1152
Lys Lys Tyr Gln Tyr His Lys Ala Tyr Leu Ile Leu Cys Gly Val Thr
    370                 375                 380

aac ctg ctt gct cct tta gcc acc aca ttc cca ctc ctt atg gcc tat 1200
Asn Leu Leu Ala Pro Leu Ala Thr Thr Phe Pro Leu Leu Met Ala Tyr
385                 390                 395                 400

acc atc ttt ttt gcc att ttt gct ggt ggc tac ctg gca cta ata ctg 1248
Thr Ile Phe Phe Ala Ile Phe Ala Gly Gly Tyr Leu Ala Leu Ile Leu
                405                 410                 415

cca gta ttg gtt gac cta tct aag aat tct aga gta cac agg ttt ttg 1296
Pro Val Leu Val Asp Leu Ser Lys Asn Ser Arg Val His Arg Phe Leu
            420                 425                 430

gga tat gcc agt ttc ttt gct ggg atg gct gtt ctt tct gga cca cca 1344
Gly Tyr Ala Ser Phe Phe Ala Gly Met Ala Val Leu Ser Gly Pro Pro
        435                 440                 445

ata gca ggc tgg ata tac gat tat aca cag aca tac gtg ggc tct ttc 1392
Ile Ala Gly Trp Ile Tyr Asp Tyr Thr Gln Thr Tyr Val Gly Ser Phe
    450                 455                 460

tat ttc tct ggc acc tgc tat atc ctc tct tca gtt tcc ctt ttt ttt 1440
Tyr Phe Ser Gly Thr Cys Tyr Ile Leu Ser Ser Val Ser Leu Phe Phe
465                 470                 475                 480

gta cca ctg gct gaa aga tgg aaa ctc aag cag tct gac ctt cta agg 1488
Val Pro Leu Ala Glu Arg Trp Lys Leu Lys Gln Ser Asp Leu Leu Arg
                485                 490                 495

act aca atc aag taa                                                    1503
Thr Thr Ile Lys
        500

<210>  6
<211>  500
<212>  PRT
<213>  Rattus norvegicus

<400>  6

Met Lys Lys Arg Glu Glu Lys Pro Ser Pro Tyr Thr Lys Pro Leu Asp
1               5                   10                  15

Gly Gly Trp Gly Trp Met Val Val Leu His Phe Phe Leu Val Asn Val
            20                  25                  30

Phe Val Met Gly Met Thr Lys Thr Phe Ala Ile Phe Phe Val Val Phe
        35                  40                  45

Gln Glu Glu Phe Glu Gly Thr Ser Glu Gln Ile Gly Trp Ile Gly Ser
    50                  55                  60

Ile Met Ser Ser Leu Arg Phe Ser Ala Gly Pro Leu Ala Ala Ile Ile
65                  70                  75                  80

Cys Asp Ile Phe Gly Glu Lys Thr Thr Ser Ile Leu Gly Thr Phe Leu
                85                  90                  95

Val Ser Gly Gly Tyr Leu Ile Ser Ser Trp Ala Thr Gly Ile Pro Phe
            100                 105                 110

Leu Cys Val Thr Met Gly Leu Leu Pro Gly Leu Gly Ser Ala Phe Leu
        115                 120                 125

Tyr Gln Val Ala Ala Val Val Ile Thr Lys Tyr Phe Lys Lys Arg Leu
    130                 135                 140

Gly Leu Ser Thr Ala Ile Ala Arg Ser Gly Met Gly Leu Thr Phe Leu
145                 150                 155                 160

Leu Ala Pro Phe Thr Lys Leu Leu Ile Asp Leu Tyr Asp Trp Thr Gly
                165                 170                 175

Ala Leu Ile Leu Phe Gly Ala Ile Ala Leu Asn Leu Val Pro Ser Ser
            180                 185                 190

Met Leu Leu Arg Pro Val His Ile Gln Ser Asp Ser Asn Ser Asp Ile
        195                 200                 205

Lys Asn Lys Gly Ser Ile Leu Ser Ala Thr Gly Pro Glu Leu Ser His
    210                 215                 220

Lys Thr Glu Thr Pro Asn Arg Asn Lys Ala Gln Glu Ser Leu Ile Lys
225                 230                 235                 240

Asp Ser Ala Val Lys Lys Ser Gly Gln Pro Thr Thr Thr Leu Thr Val
                245                 250                 255

Leu Glu Asn Gln Ile Glu Glu Ile Ser Thr Arg Pro His Arg Asn Arg
            260                 265                 270

Pro Leu Leu Met Ser Asn Asp Lys Thr Tyr Lys Lys Lys Phe Val Ser
        275                 280                 285

Trp Asn Cys Lys Glu Lys Leu Leu Asp Ile Ser Leu Phe Arg Asn Pro
    290                 295                 300

Phe Phe Tyr Ile Phe Thr Trp Ser Phe Leu Leu Ser Gln Leu Ala Tyr
305                 310                 315                 320

Phe Ile Pro Thr Phe His Leu Val Ala Arg Ala Lys Thr Leu Gly Ile
                325                 330                 335

Asp Ile Met Asp Ala Ser Tyr Leu Val Ser Val Ala Gly Ile Thr Glu
            340                 345                 350

Thr Val Ser Gln Leu Ile Ser Gly Trp Ile Ala Asp Gln Asn Trp Ile
        355                 360                 365

Lys Lys Tyr Gln Tyr His Lys Ala Tyr Leu Ile Leu Cys Gly Val Thr
    370                 375                 380

Asn Leu Leu Ala Pro Leu Ala Thr Thr Phe Pro Leu Leu Met Ala Tyr
385                 390                 395                 400

Thr Ile Phe Phe Ala Ile Phe Ala Gly Gly Tyr Leu Ala Leu Ile Leu
                405                 410                 415

Pro Val Leu Val Asp Leu Ser Lys Asn Ser Arg Val His Arg Phe Leu
            420                 425                 430

Gly Tyr Ala Ser Phe Phe Ala Gly Met Ala Val Leu Ser Gly Pro Pro
        435                 440                 445

Ile Ala Gly Trp Ile Tyr Asp Tyr Thr Gln Thr Tyr Val Gly Ser Phe
    450                 455                 460

Tyr Phe Ser Gly Thr Cys Tyr Ile Leu Ser Ser Val Ser Leu Phe Phe
465                 470                 475                 480

Val Pro Leu Ala Glu Arg Trp Lys Leu Lys Gln Ser Asp Leu Leu Arg
                485                 490                 495

Thr Thr Ile Lys
            500

<210>  7
<211>  104
<212>  PRT
<213>  Homo sapiens

<400>  7

Arg Pro Ile His Ile Lys Ser Glu Asn Asn Ser Gly Ile Lys Asp Lys
1               5                   10                  15

Gly Ser Ser Leu Ser Ala His Gly Pro Glu Ala His Ala Thr Glu Thr
            20                  25                  30

His Cys His Glu Thr Glu Glu Ser Thr Ile Lys Asp Ser Thr Thr Gln
        35                  40                  45

Lys Ala Gly Leu Pro Ser Lys Asn Leu Thr Val Ser Gln Asn Gln Ser
    50                  55                  60

Glu Glu Phe Tyr Asn Gly Pro Asn Arg Asn Arg Leu Leu Leu Lys Ser
65                  70                  75                  80

Asp Glu Glu Ser Asp Lys Val Ile Ser Trp Ser Cys Lys Gln Leu Phe
                85                  90                  95

Asp Ile Ser Leu Phe Arg Asn Pro
            100

<210>  8
<211>  63
<212>  PRT
<213>  Homo sapiens

<400>  8

Arg Pro Ile His Ile Lys Ser Glu Asn Asn Ser Gly Ile Lys Asp Lys
1               5                   10                  15

Gly Ser Ser Leu Ser Ala His Gly Pro Glu Ala His Ala Thr Glu Thr
            20                  25                  30

His Cys His Glu Thr Glu Glu Ser Thr Ile Lys Asp Ser Thr Thr Gln
        35                  40                  45

Lys Ala Gly Leu Pro Ser Lys Asn Leu Thr Val Ser Gln Asn Gln
    50                  55                  60

<210>  9
<211>  73
<212>  PRT
<213>  Homo sapiens

<400>  9

Thr His Cys His Glu Thr Glu Glu Ser Thr Ile Lys Asp Ser Thr Thr
1               5                   10                  15

Gln Lys Ala Gly Leu Pro Ser Lys Asn Leu Thr Val Ser Gln Asn Gln
            20                  25                  30

Ser Glu Glu Phe Tyr Asn Gly Pro Asn Arg Asn Arg Leu Leu Leu Lys
        35                  40                  45

Ser Asp Glu Glu Ser Asp Lys Val Ile Ser Trp Ser Cys Lys Gln Leu
    50                  55                  60

Phe Asp Ile Ser Leu Phe Arg Asn Pro
65                  70

<210>  10
<211>  16
<212>  PRT
<213>  Homo sapiens

<400>  10

Thr His Cys His Glu Thr Glu Glu Ser Thr Ile Lys Asp Ser Thr Thr
1               5                   10                  15

<210>  11
<211>  16
<212>  PRT
<213>  Homo sapiens

<400>  11

Ser Thr Ile Lys Asp Ser Thr Thr Gln Lys Ala Gly Leu Pro Ser Lys
1               5                   10                  15

<210>  12
<211>  17
<212>  PRT
<213>  Homo sapiens

<400>  12

Thr Gln Lys Ala Gly Leu Pro Ser Lys Asn Leu Thr Val Ser Gln Asn
1               5                   10                  15

Gln

<210>  13
<211>  8
<212>  PRT
<213>  Homo sapiens

<400>  13

Asn Leu Thr Val Ser Gln Asn Gln
1               5

<210>  14
<211>  32
<212>  PRT
<213>  Homo sapiens

<400>  14

Thr His Cys His Glu Thr Glu Glu Ser Thr Ile Lys Asp Ser Thr Thr
1               5                   10                  15

Gln Lys Ala Gly Leu Pro Ser Lys Asn Leu Thr Val Ser Gln Asn Gln
            20                  25                  30

<210>  15
<211>  56
<212>  PRT
<213>  Homo sapiens

<400>  15

Leu Arg Pro Ile His Ile Lys Ser Glu Asn Asn Ser Gly Ile Lys Asp
1               5                   10                  15

Lys Gly Ser Ser Leu Ser Ala His Gly Pro Glu Ala His Ala Thr Glu
            20                  25                  30

Thr His Cys His Glu Thr Glu Glu Ser Thr Ile Lys Asp Ser Thr Thr
        35                  40                  45

Gln Lys Ala Gly Leu Pro Ser Lys
    50                  55

<210>  16
<211>  420
<212>  DNA
<213>  Artificial

<220>
<223>  synthetic DNA

<400>  16
atgaaatgca gctgggtcat cttcttcctg atggcagtgg ttacaggggt caattcagag      60

gttcagctgc agcagtctgg ggctgagctt gtgaggccag gggccttagt caagttgtcc     120

tgcaaagctt ctggcttcaa cattaaagac tactatatgt tctgggtgaa gcagaggcct     180

gaacagggcc tggagtggat tggatggatt gatcctgaga atggtaatac tatatttgac     240

ccgaagttcc agggcaaggc cagtataaca gcagacacat cctccaacac agcctacctg     300

cagctcagca gcctgacatc tgaggacact gccgtctatt actgtgctag aatgattacg     360

acctattact atgctatgga cttctggggt caaggaacct cagtcaccgt ctcctcagcc     420


<210>  17
<211>  140
<212>  PRT
<213>  Artificial

<220>
<223>  synthetic peptide

<400>  17

Met Lys Cys Ser Trp Val Ile Phe Phe Leu Met Ala Val Val Thr Gly
1               5                   10                  15


Val Asn Ser Glu Val Gln Leu Gln Gln Ser Gly Ala Glu Leu Val Arg
            20                  25                  30


Pro Gly Ala Leu Val Lys Leu Ser Cys Lys Ala Ser Gly Phe Asn Ile
        35                  40                  45


Lys Asp Tyr Tyr Met Phe Trp Val Lys Gln Arg Pro Glu Gln Gly Leu
    50                  55                  60


Glu Trp Ile Gly Trp Ile Asp Pro Glu Asn Gly Asn Thr Ile Phe Asp
65                  70                  75                  80


Pro Lys Phe Gln Gly Lys Ala Ser Ile Thr Ala Asp Thr Ser Ser Asn
                85                  90                  95


Thr Ala Tyr Leu Gln Leu Ser Ser Leu Thr Ser Glu Asp Thr Ala Val
            100                 105                 110


Tyr Tyr Cys Ala Arg Met Ile Thr Thr Tyr Tyr Tyr Ala Met Asp Phe
        115                 120                 125


Trp Gly Gln Gly Thr Ser Val Thr Val Ser Ser Ala

130                 135                 140

<210>  18
<211>  9
<212>  PRT
<213>  Artificial

<220>
<223>  synthetic peptide

<400>  18

Phe Asn Ile Lys Asp Tyr Tyr Met Phe
1               5

<210>  19
<211>  18
<212>  PRT
<213>  Artificial

<220>
<223>  synthetic peptide

<400>  19

Trp Ile Asp Pro Glu Asn Gly Asn Thr Ile Phe Asp Pro Lys Phe Gln
1               5                   10                  15

Gly Lys

<210>  20
<211>  12
<212>  PRT
<213>  Artificial

<220>
<223>  synthetic peptide

<400>  20

Met Ile Thr Thr Tyr Tyr Tyr Ala Met Asp Phe Trp
1               5                   10

<210>  21
<211>  27
<212>  DNA
<213>  Artificial

<220>
<223>  synthetic DNA

<400>  21
ttcaacatta aagactacta tatgttc                                          27

<210>  22
<211>  54

<212>  DNA
<213>  Artificial

<220>
<223>  synthetic DNA

<400>  22
tggattgatc ctgagaatgg taatactata tttgacccga agttccaggg caag          54


<210>  23
<211>  36
<212>  DNA
<213>  Artificial

<220>
<223>  synthetic DNA

<400>  23
atgattacga cctattacta tgctatggac ttctgg                              36


<210>  24
<211>  390
<212>  DNA
<213>  Artificial

<220>
<223>  synthetic DNA

<400>  24
atggcctgga ctcctctctt cttcttcttt gttcttcatt gctcaggttc tttctcccaa      60

cttgtgctca ctcagtcatc ttcagcctct ttctccctgg gagcctcagc aaaactcacg     120

tgcaccttga gtagtcagca cagtacgtac accattgaat ggtatcagca acagccactc     180

aagcctccta agtatgtgat ggagcttaag aaagatggaa gccacagcac aggtgatggg     240

attcctgatc gcttctctgg atccagctct ggtgctgatc gctaccttag cgtttccagc     300

atccagcctg aagatgaagc aatatacatc tgtggtgtgg gttatacaat taaggaacaa     360

tttgtgtatg ttttcggcgg tggaaccaag                                      390


<210>  25
<211>  130
<212>  PRT
<213>  Artificial

<220>
<223>  synthetic peptide

<400>  25

Met Ala Trp Thr Pro Leu Phe Phe Phe Phe Val Leu His Cys Ser Gly
1               5                   10                  15


Ser Phe Ser Gln Leu Val Leu Thr Gln Ser Ser Ser Ala Ser Phe Ser
            20                  25                  30

Leu Gly Ala Ser Ala Lys Leu Thr Cys Thr Leu Ser Ser Gln His Ser
        35                  40                  45

Thr Tyr Thr Ile Glu Trp Tyr Gln Gln Gln Pro Leu Lys Pro Pro Lys
    50                  55                  60

Tyr Val Met Glu Leu Lys Lys Asp Gly Ser His Ser Thr Gly Asp Gly
65                  70                  75                  80

Ile Pro Asp Arg Phe Ser Gly Ser Ser Ser Gly Ala Asp Arg Tyr Leu
                85                  90                  95

Ser Val Ser Ser Ile Gln Pro Glu Asp Glu Ala Ile Tyr Ile Cys Gly
            100                 105                 110

Val Gly Tyr Thr Ile Lys Glu Gln Phe Val Tyr Val Phe Gly Gly Gly
        115                 120                 125

Thr Lys
    130


<210>  26
<211>  7
<212>  PRT
<213>  Artificial

<220>
<223>  synthetic peptide

<400>  26

Ser Gln His Ser Thr Tyr Thr
1               5


<210>  27
<211>  10
<212>  PRT
<213>  Artificial

<220>
<223>  synthetic peptide

<400>  27

Glu Leu Lys Lys Asp Gly Ser His Ser Thr
1               5                   10


<210>  28
<211>  7
<212>  PRT
<213>  Artificial

<220>

<223>  synthetic peptide

<400>  28

Gly Tyr Thr Ile Lys Glu Gln
1               5


<210>  29
<211>  21
<212>  DNA
<213>  Artificial

<220>
<223>  synthetic DNA

<400>  29
agtcagcaca gtacgtacac c                                                21


<210>  30
<211>  30
<212>  DNA
<213>  Artificial

<220>
<223>  synthetic DNA

<400>  30
gagcttaaga aagatggaag ccacagcaca                                       30


<210>  31
<211>  21
<212>  DNA
<213>  Artificial

<220>
<223>  synthetic DNA

<400>  31
ggttatacaa ttaaggaaca a                                                21


<210>  32
<211>  31
<212>  DNA
<213>  Artificial

<220>
<223>  synthetic DNA

<400>  32
aaaggtacca tgctgaagag ggaggggaag g                                     31


<210>  33
<211>  34
<212>  DNA
<213>  Artificial

<220>
<223>  synthetic DNA

<400>  33
aaatctagag gtcagactgt ttttccatct ttcg                                    34


<210>  34
<211>  23
<212>  DNA
<213>  Artificial

<220>
<223>  synthetic DNA

<400>  34
ctgtagcagg tatccttgag acg                                                23


<210>  35
<211>  25
<212>  DNA
<213>  Artificial

<220>
<223>  synthetic DNA

<400>  35
ggatgaggta agacttgtgg taatg                                              25


<210>  36
<211>  20
<212>  DNA
<213>  Artificial

<220>
<223>  synthetic DNA

<400>  36
tgcaccacca actgcttagc                                                    20


<210>  37
<211>  20
<212>  DNA
<213>  Artificial

<220>
<223>  synthetic DNA

<400>  37
ggcatggact gtggtcatga                                                    20


<210>  38
<211>  33
<212>  DNA
<213>  Artificial

<220>
<223>  synthetic DNA

<400>  38
gcgcggatcc gagacccatc catatcaaaa gtg                                     33

<210>  39
<211>  36
<212>  DNA
<213>  Artificial

<220>
<223>  synthetic DNA

<400>  39
gcgcgcgtcg acaggatttc taaagagaga aatgtc                                36


<210>  40
<211>  312
<212>  DNA
<213>  Homo sapiens

<400>  40
agacccatcc atatcaaaag tgagaacaat tctggtatta aagataaagg cagcagtttg      60

tctgcacatg gtccagaggc acatgcaaca gaaacacact gccatgagac agaagagtct     120

accatcaagg acagtactac gcagaaggct ggactaccta gcaaaaattt aacagtctca     180

caaaatcaaa gtgaagagtt ctacaatggg cctaacagga acagactgtt attaaagagt     240

gatgaagaaa gtgataaggt tatttcgtgg agctgcaaac aactgtttga catttctctc     300

tttagaaatc ct                                                         312


<210>  41
<211>  189
<212>  DNA
<213>  Homo sapiens

<400>  41
agacccatcc atatcaaaag tgagaacaat tctggtatta aagataaagg cagcagtttg      60

tctgcacatg gtccagaggc acatgcaaca gaaacacact gccatgagac agaagagtct     120

accatcaagg acagtactac gcagaaggct ggactaccta gcaaaaattt aacagtctca     180

caaaatcaa                                                             189


<210>  42
<211>  219
<212>  DNA
<213>  Homo sapiens

<400>  42
acacactgcc atgagacaga agagtctacc atcaaggaca gtactacgca gaaggctgga      60

ctacctagca aaaatttaac agtctcacaa aatcaaagtg aagagttcta caatgggcct     120

aacaggaaca gactgttatt aaagagtgat gaagaaagtg ataaggttat ttcgtggagc     180

tgcaaacaac tgtttgacat ttctctcttt agaaatcct                            219

<210>  43
<211>  38
<212>  DNA
<213>  Artificial

<220>
<223>  synthetic DNA

<400>  43
aaagtcgact tgattttgtg agactgttaa atttttgc                              38


<210>  44
<211>  439
<212>  PRT
<213>  Homo sapiens

<400>  44

Met Leu Lys Arg Glu Gly Lys Val Gln Pro Tyr Thr Lys Thr Leu Asp
1               5                   10                  15

Gly Gly Trp Gly Trp Met Ile Val Ile His Phe Phe Leu Val Asn Val
            20                  25                  30

Phe Val Met Gly Met Thr Lys Thr Phe Ala Ile Phe Phe Val Val Phe
        35                  40                  45

Gln Glu Glu Phe Glu Gly Thr Ser Glu Gln Ile Gly Trp Ile Gly Ser
    50                  55                  60

Ile Met Ser Ser Leu Arg Phe Cys Ala Gly Leu Gly Ser Ala Phe Leu
65                  70                  75                  80

Tyr Gln Val Ala Ala Val Val Thr Thr Lys Tyr Phe Lys Lys Arg Leu
                85                  90                  95

Ala Leu Ser Thr Ala Ile Ala Arg Ser Gly Met Gly Leu Thr Phe Leu
            100                 105                 110

Leu Ala Pro Phe Thr Lys Phe Leu Ile Asp Leu Tyr Asp Trp Thr Gly
        115                 120                 125

Ala Leu Ile Leu Phe Gly Ala Ile Ala Leu Asn Leu Val Pro Ser Ser
    130                 135                 140

Met Leu Leu Arg Pro Ile His Ile Lys Ser Glu Asn Asn Ser Gly Ile
145                 150                 155                 160

Lys Asp Lys Gly Ser Ser Leu Ser Ala His Gly Pro Glu Ala His Ala
                165                 170                 175

Thr Glu Thr His Cys His Glu Thr Glu Glu Ser Thr Ile Lys Asp Ser
            180                 185                 190

Thr Thr Gln Lys Ala Gly Leu Pro Ser Lys Asn Leu Thr Val Ser Gln
        195                 200                 205

Asn Gln Ser Glu Glu Phe Tyr Asn Gly Pro Asn Arg Asn Arg Leu Leu
    210                 215                 220

Leu Lys Ser Asp Glu Glu Ser Asp Lys Val Ile Ser Trp Ser Cys Lys
225                 230                 235                 240

Gln Leu Phe Asp Ile Ser Leu Phe Arg Asn Pro Phe Phe Tyr Ile Phe
                245                 250                 255

Thr Trp Ser Phe Leu Leu Ser Gln Leu Ala Tyr Phe Ile Pro Thr Phe
            260                 265                 270

His Leu Val Ala Arg Ala Lys Thr Leu Gly Ile Asp Ile Met Asp Ala
        275                 280                 285

Ser Tyr Leu Val Ser Val Ala Gly Ile Leu Glu Thr Val Ser Gln Ile
    290                 295                 300

Ile Ser Gly Trp Val Ala Asp Gln Asn Trp Ile Lys Lys Tyr His Tyr
305                 310                 315                 320

His Lys Ser Tyr Leu Ile Leu Cys Gly Ile Thr Asn Leu Leu Ala Pro
                325                 330                 335

Leu Ala Thr Thr Phe Pro Leu Leu Met Thr Tyr Thr Ile Cys Phe Ala
            340                 345                 350

Ile Phe Ala Gly Gly Tyr Leu Ala Leu Ile Leu Pro Val Leu Val Asp
        355                 360                 365

Leu Cys Arg Asn Ser Thr Val Asn Arg Phe Leu Gly Leu Ala Ser Phe
    370                 375                 380

Phe Ala Gly Met Ala Val Leu Ser Gly Pro Pro Ile Ala Gly Trp Leu
385                 390                 395                 400

Tyr Asp Tyr Thr Gln Thr Tyr Asn Gly Ser Phe Tyr Phe Ser Gly Ile
                405                 410                 415

Cys Tyr Leu Leu Ser Ser Val Ser Phe Phe Phe Val Pro Leu Ala Glu
            420                 425                 430

Arg Trp Lys Asn Ser Leu Thr
        435

<210>  45
<211>  11
<212>  PRT
<213>  Homo sapiens

<400>  45

Gly Ser Ile Met Ser Ser Leu Arg Phe Cys Ala
1               5                   10

<210>  46
<211>  10
<212>  PRT
<213>  Homo sapiens

<400>  46

Met Gly Met Asp Asp Cys Asp Ser Phe Phe
1               5                   10

<210>  47
<211>  47
<212>  PRT
<213>  Homo sapiens

<400>  47

Glu Ile Ile Pro Ser Phe Gln Ala Gly Tyr Met Ile Ile Pro Arg His
1               5                   10                  15

Thr Met Ala Leu Ser Thr Ser Leu Ala Tyr Ala Ile Ser Ser Leu Gln
            20                  25                  30

Phe Pro Phe Phe Leu Tyr His Trp Pro Lys Asp Gly Lys Thr Val
        35                  40                  45

<210>  48
<211>  31
<212>  DNA
<213>  Artificial

<220>
<223>  synthetic DNA

<400>  48
aaaggatcca cacactgcca tgagacagaa g                                    31